(19) Europäisches Patentamt
European Patent Office
Office européen des brevets

(11) Publication number: **0 225 175**
**A2**

(12) # EUROPEAN PATENT APPLICATION

(21) Application number: 86309244.1

(22) Date of filing: 27.11.86

(51) Int. Cl.[4]: **C 07 D 211/90**
C 07 D 401/06,
C 07 D 401/12,
C 07 D 409/12,
C 07 D 409/04,
A 61 K 31/445, A 61 K 31/505

(30) Priority: 28.11.85 GB 8529301
03.12.85 GB 8529786
03.12.85 GB 8529787
21.02.86 GB 8604421
21.02.86 GB 8604422
21.02.86 GB 8604423
21.02.86 GB 8604424
28.02.86 GB 8605000
06.09.86 GB 8621514

(43) Date of publication of application:
**10.06.87 Bulletin 87/24**

(84) Designated Contracting States:
**AT BE CH DE ES FR GB GR IT LI LU NL SE**

(71) Applicant: **FISONS plc**
**Fison House Princes Street**
**Ipswich Suffolk IP1 1QH (GB)**

(72) Inventor: **Baxter, Andrew John Gilby**
**Flat 4, 6 Western Terrace**
**The Park Nottingham (GB)**

**Dixon, John**
**Church Farmhouse Main Street**
**Gr. Dalby Melton Mowbray Leicestershire (GB)**

**McInally, Thomas**
**24 Borrowell**
**Kegworth Derbyshire (GB)**

**Tinker, Alan Charles**
**97 Knighttthorpe Road**
**Loughborough Leicestershire (GB)**

(74) Representative: **Craig, Christopher Bradberry et al**
**Fisons plc 12 Derby Road**
**Loughborough Leicestershire LE11 0BB (GB)**

Claims for the following Contracting States: AT + ES + GR.

(54) **Dihydropyridine derivatives, processes for their preparation and pharmaceutical compositions thereof.**

(57) There are described compounds of Formula I,

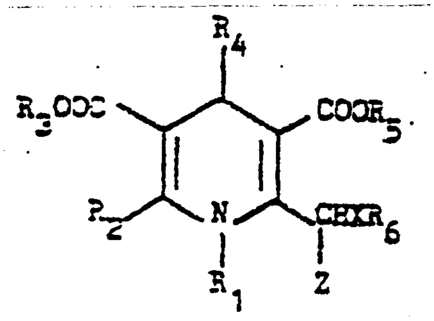

I

in which $R_1$, $R_2$, $R_3$ and $R_4$ are as defined in the specification,
$R_5$ is alkyl optionally interrupted by an amide group and optionally substituted by halogen or by a carboxy or a heterocyclic group,
X is -O-, -NR-, -S(O)$_m$,, or a bond,
Z is hydrogen or together with R forms a bond,
R is hydrogen, alkyl or together with Z forms a bond,
$R_6$ is a 5 or 6 membered unsaturated nitrogen containing heterocyclic ring, optionally fused to a phenyl ring and optionally substituted by one or more of oxo, hydroxy, alkyl, $-(CH_2)_{n_1}NH_2$, alkoxy, phenyl or -COOH,
and when X is -NR- or -S(O)$_{m_1}$-, $R_6$ may in addition be hydrogen or a group $-CH_2CH_2NH_2$, $-CH_2CH_2$-het. or $NHC(=NH)NH_2$,
when X is -O-, $R_6$ may also be a group $-NH_2$, $-C(=O)(CH_2)_nNH_2$, $-CH_2CH_2NR_{14}R_{15}$ or $CH_2CH_2OCH_2CH_2NR_{14}R_{15}$,
when X is -NR-, -S(O)$_{m_1}$-, or a bond, $R_6$ may also be hydrogen or a group $-C(=NR_{12})NHR_{13}$,
$R_{13}$ and $R_{14}$ may be hydrogen or alkyl,
$R_{12}$ is hydrogen, alkylcarbonyl, nitrile or hydroxy,
$R_{15}$ is hydrogen or a group $R_{11}$,
m and $m_1$ are each 0, 1 or 2,
n is 0 or 1,
$n_1$ is an integer from 0 to 6
and pharmaceutically acceptable salts, esters, amides, analogues and protected derivatives thereof.

EP 0 225 175 A2

**Description**

This invention relates to new compounds, methods and intermediates for their preparation and compositions containing them.

A wide variety of dihydropyridines have been described as being useful as pharmaceuticals and some, notably nifedipine, have been sold for this use. Useful dihydropyridines are disclosed in European Patent Applications 0172029, 0169009, 0174131 and 0125803.

We have now found a new group of dihydropyridines having advantageous properties.

According to the invention we provide compounds of formula I,

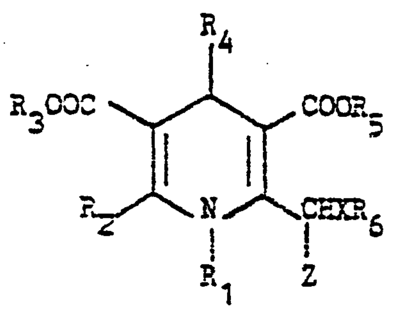

I

in which $R_2$ is alkyl C 1 to 6 optionally substituted by one or more fluorine atoms,
$R_3$ is alkyl C 1 to 6,
$R_4$ is phenyl, napthyl or an unsaturated sulphur containing heterocyclic ring, each of which is optionally substituted by one or more groups selected from $-CF_3$, -CN, $-NO_2$, $NH_2$, halogen, alkyl C 1 to 6, alkoxy C 1 to 6 or $C_2F_5$,
$R_5$ is alkyl C 1 to 10 optionally interrupted by $-CONR_8-$ and optionally substituted by one or more halogen atoms or by a group $-YR_7$
Y is -O-, $-S(O)_m$, $-NR_9$ or a bond,
$R_7$ is -COOH or a group $R_{10}$,
X is -O-, -NR-, $-S(O)_{m_1}$, or a bond,
Z is hydrogen or together with R forms a bond,
R is hydrogen, alkyl C 1 to 6 or together with Z forms a bond,
$R_6$ is a group $R_{11}$,
$R_{10}$ and $R_{11}$, which may be the same or different, represent a 5 or 6 membered unsaturated nitrogen containing heterocyclic ring which is optionally fused to a phenyl ring, $R_{10}$ and $R_{11}$ optionally being substituted by one or more groups selected from oxo, hydroxy, alkyl C 1 to 6, $-(CH_2)_{n_1}NH_2$, alkoxy C 1 to 6, phenyl or -COOH,
and when X is -NR- or $-S(O)_{m_1}-$, $R_6$ may in addition by hydrogen or a group $-CH_2CH_2NH_2$, $-CH_2CH_2R_{11}$ or $NHC(=NH)NH_2$.
when X is -O-. $R_6$ may also be a group $-NH_2$, $-C(=O)\ (CH_2)_nNH_2$, $-CH_2CH_2NR_{14}R_{15}$ or $CH_2CH_2OCH_2CH_2NR_{14}R_{15}$,
when X is -NR-, $-S(O)_{m_1}-$, or a bond, $R_6$ may also be hydrogen or a group $-C(=NR_{12})NHR_{13}$,
when Y is -O-, $R_{10}$ may in addition be phenyl,
and when $-CHZXR_6$ is $-CH_2OCH_2CH_2NH_2$ then $R_5$ may be thietanyl,
$R_1$, $R_8$, $R_9$, $R_{13}$ and $R_{14}$, which may be the same or different, are each hydrogen or alkyl C 1 to 6,
$R_{12}$ is hydrogen, alkylcarbonyl C2 to 7, nitrile or hydroxy,
$R_{15}$ is hydrogen or a group $R_{11}$,
m and $m_1$, which may be the same or different, are each 0, 1 or 2,
n is 0 or 1,
$n_1$ is an integer from 0 to 6
and pharmaceutically acceptable salts, esters, amides and protected derivatives thereof,
with the provisos that:-

i) when $R_2$ is unsubstituted alkyl then

a) the group $-CHZXR_6$ is not methyl, $-CH_2OCONH_2$, $-CH_2OCH_2CH_2NH_2$, $-CH=NCH_2CH_2NH_2$ or the groups of formulae XXV and XXVI,

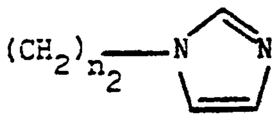

XXV

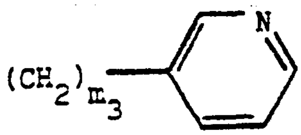

XXVI

in which $n_2$ is 1 or 2, and
$m_3$ is 0, 1 or 2, and
b) $R_4$ is not a group of formula XXVII,

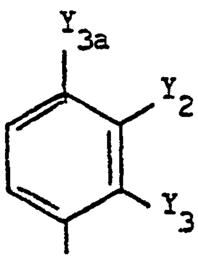

XXVII

in which $Y_2$ is hydrogen, -Cl or $-CF_3$, and $Y_3$ and $Y_{3a}$, which may be same or different, are each hydrogen or -Cl,

ii) when $-CHZXR_6$ is methyl and

A. $R_5$ is unsubstituted alkyl, then $R_4$ is not a monosubstituted phenyl group, 2-fluoro-6-(trifluoromethyl) phenyl, a group of formula XXVIII,

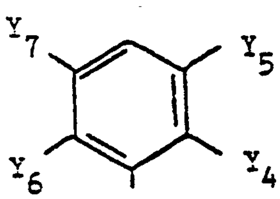

XXVIII

in which, either
a) $Y_4$ is -Cl, methyl or methoxy, $Y_5$ is -Cl, $-NO_2$, $-CF_3$, methyl or methoxy, and $Y_6$ and $Y_7$ are both hydrogen or
b) $Y_5$ is $-NO_2$, $Y_6$ is -Cl or methyl and $Y_4$ and $Y_7$ are both hydrogen:
or a 2,3,6-trisubstituted phenyl group other than 6-chloro-2-fluoro-3-methoxyphenyl; and

B. $R_5$ is methyl then $R_4$ is not a pentafluorophenyl group,

iii) when $R_2$ is $-CH_2F$ and

A. $R_5$ is unsubstituted alkyl, other than cycloalkyl, and the group $-CHZXR_6$ is $-CH_2OCH_2CH_2NR_{14}R_{15}$ then $R_4$ is not a group of formula XXIX,

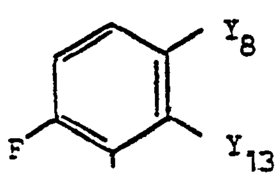

XXIX

in which,
a) $Y_{13}$ is $-CF_3$ and $Y_8$ is -Cl, or
b) $Y_{13}$ is -F and $Y_8$ is either -F or $-NO_2$, and

B. $R_5$ is alkyl substituted by halogen then $R_4$ is not 2,3,6-trisubstituted phenyl group, and

C. $R_5$ is 1-methylethyl then

a) $R_4$ is not a group of formula XXX,

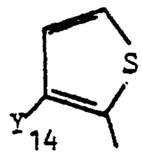

XXX

in which $Y_{14}$ is nitro, nitrile or methyl, and

b) $R_4$ is not a group of formula XXXI.

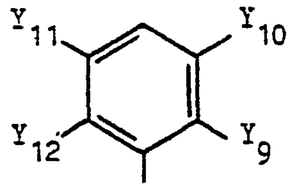

XXXI

in which $Y_9$ and $Y_{11}$, which may be the same or different, are each -Cl or -F,

$Y_{10}$ is -Cl or $-CF_3$, and

$Y_{12}$ is $-CF_3$ or hydrogen;

iv) when $R_5$ is an uninterrupted alkyl group and Y is -O-, then $R_7$ is not a monosubstituted pyrazol-3-yl group, a 2,5-disubstituted pyrimidin-4-yl group, or protected derivatives thereof.

According to the invention we further provide a process for the production of a compound of formula I, or pharmaceutically acceptable salts, esters, amides and protected derivatives thereof, which comprises

a) production of a compound of formula I in which X is -O-, -NR-, -S- or a bond other than a carbon carbon bond, by reaction of a compound of formula II,

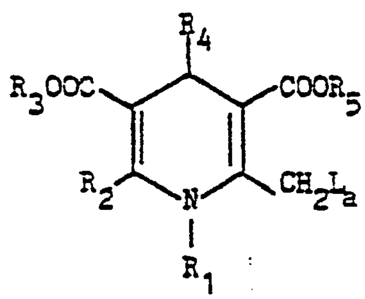

II

in which $R_1$, $R_2$, $R_3$, $R_4$ and $R_5$ are as defined above, and

$L_a$ is a good leaving group,

with a compound of formula III,

$R_{11}X_aH$ III

in which $R_{11}$ is as defined above, and

$X_a$ represents -O-, -NR-, -S- or a bond

b) production of a compound of formula I in which at least one of m or $m_1$ is 1 or 2 by selective oxidation of a corresponding compound of formula I in which at least one of m or $m_1$ is 0 or 1 respectively,

c) production of a compound of formula I in which at least one of $R_5$, $R_6$, $R_{10}$ and $R_{11}$ carries an $NH_2$ group by deprotection of a corresponding compound of formula I in which at least one $R_5$, $R_6$, $R_{10}$ and $R_{11}$ carries a protected -NH- group,

d) production of a compound of formula I in which X is a bond and $R_{11}$ is a group of formula IV,

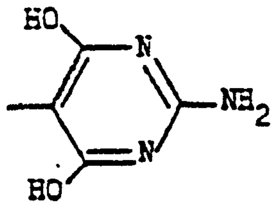

IV

by reacting guanidine with a compound of formula V,

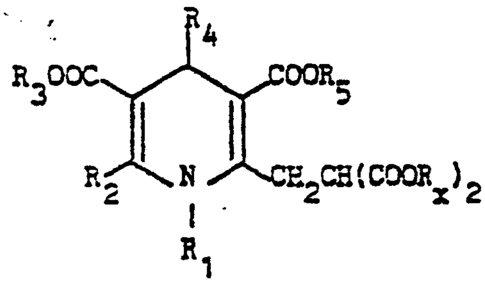

V

in which $R_1$, $R_2$, $R_3$, $R_4$ and $R_5$ are as defined above, and
$R_x$ is alkyl C 1 to 6,

e) production of a compound of formula I in which X is -O-, -S- or -NR- and $R_{11}$ is a pyrimidin-2-yl group by reaction of a corresponding compound of formula I in which X is -O-, -S- or -NR- and $R_{11}$ is hydrogen with a compound of formula VI,

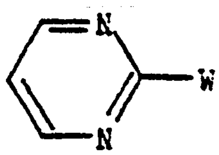

VI

in which W is a good leaving group,

f) production of a compound of formula I in which X is a bond and $R_{11}$ is a 1H-tetrazol-5-yl group by reacting a corresponding compound of formula I in which $R_{11}$ is nitrile with an azide,

g) production of a compound of formula I in which X is a bond and $R_{11}$ is 1H-pyrrol-1-yl group by reaction of a corresponding compound of formula I in which the group $XR_{11}$ is $-NH_2$ with 2,5-dimethoxytetrahydrofuran,

h) production of a compound of formula I in which X is -O- and $R_6$ is $-CONH_2$ by hydrolysis of the product of the reaction of a corresponding compound of formula I in which X is -O- and $R_6$ is hydrogen with a compound of formula VII

$L_h-N=C=O$ VII

in which $L_h$ is a good leaving group,

i) production of a compound of formula I in which R and Z together form a bond and $R_6$ is a group $-NHC(=NH)NH_2$ by reacting a compound of formula VIII,

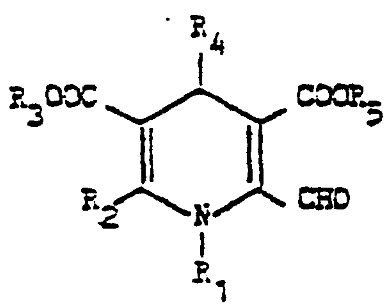

VIII

in which $R_1$, $R_2$, $R_3$, $R_4$ and $R_5$ are as defined above,
with aminoguanidine or a salt thereof,

j) production of a compound of formula I in which $R_{11}$ is a 1,2,3-triazol-1-yl group by reaction of a compound of formula I in which $R_{11}$ is an azide group. with a compound of formula IX,

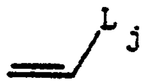

IX

in which $L_j$ is a good leaving group.

k) production of a compound of formula I in which $R_{11}$ is a 1,2,4-triazol-4-yl group by reaction of a compound of formula I in which $R_{11}$ is an amino group, with a compound of formula XI.

$L_kHC=N-N=CHL_{k'}$, XI

in which $L_k$ and $L_{k'}$, which may be the same or different, are good leaving groups,

l) production of a compound of formula I in which $R_6$ is a group $-C(=NOH)NH_2$ by reaction of a compound of formula I in which $R_6$ is nitrile, with hydroxylamine, or a salt thereof,

m) production of a compound of formula I in which at least one of $R_5$, $R_{10}$ and $R_{11}$ carries a -COOH group by reductive cleavage or hydrolysis of a corresponding compound of formula I in which at least one of $R_5$, $R_{10}$ and $R_{11}$ carries a carboxylic acid ester group,

n) production of a compound of formula I in which at least one of $R_5$, $R_{10}$ and $R_{11}$ carries a carboxylic acid ester group by esterification or transesterification of a corresponding compound of formula I in which at least one of $R_5$, $R_{10}$ and $R_{11}$ carries a -COOH group,

o) production of a compound of formula I in which $R_1$ is alkyl C 1 to 6, by C 1 to 6 alkylation of a corresponding compound of formula I in which $R_1$ is hydrogen,

p) production of a compound of formula I in which $R_6$ contains a 3-amino-4H-1,2,4-triazol-5-yl group by reaction of a corresponding compound of formula I in which $R_6$ contains an amine group, with a compound of formula XII,

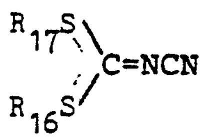

XII

in which $R_{17}$ and $R_{16}$, which may be the same or different are each alkyl C 1 to 6,
and
hydrazine or a derivative thereof,

q) production of a compound of formula I in which Z is hydrogen, X is -O- and $R_6$ is $-CH_2CH_2NH_2$ by reduction of a compound of formula XIII,

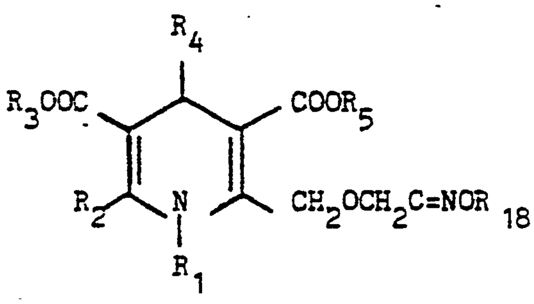

XIII

in which $R_1$, $R_2$, $R_3$, $R_4$ and $R_5$ are as defined above,
$R_{18}$ is hydrogen or alkyl C 1 to 10,

r) production of a compound of formula I in which $R_5$ contains an amide group by reaction of a corresponding compound of formula I in which $R_5$ is a group $-(CH_2)_{nr}$ $CO_2H$ with the corresponding amine,
in which $n_r$ is an integer from 1 to 8.

s) production of an optical isomer of a compound of formula I by resolution of a mixture of optical isomers of the compound,

t) production of a pharmaceutically acceptable salt of a compound of formula I, by treating a compound of formula I, or another salt, with a compound containing an available pharmaceutically acceptable ion and capable of converting the compound of formula I or the other salt to a pharmaceutically acceptable salt of the compound of formula I.

u) reaction of the compounds of formulae XIV, XV, XVI and XVII,

$R_4CHO$ XIV

$R_5OOCCH_2COCH_2XR_6$ XV

$R_3OOCCH_2COR_2$ XVI

$R_1NH_2$ XVII

or their partial condensates, or protected derivatives thereof,

in which $R_1$, $R_2$, $R_3$, $R_4$, $R_5$, X and $R_6$ are as defined above,

and where desired or necessary converting the resulting compound of formula I to a pharmaceutically acceptable salt, ester, amide or protected derivative thereof or vice versa.

The reaction of process a) may be carried out in a solvent which is inert under the reaction conditions, eg acetonitrile. The reaction many be carried out in the presence of a base for example, an alkali metal alkoxide, eg sodium methoxide, and at a temperature of from about 10° to 40°C. We prefer $L_a$ to be a halogen, eg bromine.

The selective oxidation of process b) may be carried out in a solvent with is inert under the reaction conditions, eg ethyl acetate, dichloromethane, chloroform or a mixture thereof. The reaction is preferably carried out at less than room temperature, eg -20°C to +10°C. Suitable oxidising agents for use in the reaction are peracids, eg m-chloroperbenzoic acid or potassium peroxymonosulphate.

In process c) the protecting group may be, for example, phenylmethyl, haloalkanoyl C2 to 6, especially trifluoroacetyl, t-butyloxycarbonyl or phthalimide. Other protecting groups are well known and include those described in Protective Groups in Organic Chemistry, ed: J W F McOmie, Plenum Press (1973), and Protective Groups in Organic Synthesis, T W Greene, Wiley-Interscience (1981). Removal of the protecting groups depends on the nature of the protecting group; however conventional techniques may be generally employed, including acidic or basic cleavage or hydrogenolysis.

The reaction of process d) may be carried out in a solvent which is inert under the reaction conditions, eg methanol. The reaction is also preferably carried out in the presence of a base, for example an alkali metal alkoxide, eg sodium methoxide. The reaction may be carried out at an elevated temperature, eg under reflux.

The reaction of process e) may be carried out in a solvent which is inert under the reaction conditions, eg tetrahydrofuran, and in the presence of a base, eg sodium hydride. The reaction is preferably carried out at low temperatures, eg from -20 to 20°C, preferably from -5 to 5°C and the leaving group is preferably a halogen, eg chlorine.

The reaction of process f) may be carried out in a solvent which is inert under the reaction conditions, eg dimethylformamide. The reaction is also preferably carried out in the presence of an acid, preferably a weak acid, eg ammonium chloride. The reaction may be carried out at elevated temperature, eg from 60 to 100°C, preferably from 60 to 80°C. The azide is preferably an alkali metal azide, eg sodium azide.

The reaction of process g) may be carried out in an acidic solvent, eg glacial acetic acid. The reaction is preferably carried out at elevated temperature, eg from 50 to 100°C, preferably from 70 to 90°C.

The hydrolysis of process h) may be carried out at low temperature, eg from -40 to 20°C, preferably from -10 to 0°C. We prefer the leaving group $L_h$ to be a sulphur containing group, eg chlorosulphonyl.

The reaction of process i) may be carried out in the presence of a base, for example an alkali metal alkoxide, eg sodium methoxide. The reaction may be carried out at elevated temperature, preferably from 30°C to the boiling point of the solvent, eg from 50 to 100°C reflux.

The reaction of process j) may be carried out in a solvent which is inert under the reaction conditions, eg toluene, at a temperature of from 20°C to the boiling point of the solvent, eg 20° to 100°C. $L_j$ may be alkoxy C 1 to 6, dialkylamino C 1 to 6 or alkylcarbonyloxy C2 to 7, eg acetate.

In the reaction of process k) we prefer $L_k$ and $L_{k'}$ to be dialkylamino, eg dimethylamino. The reaction may be carried out in a solvent which is inert under the reaction conditions, eg toluene, at a temperature of from 50° to 150°C, optionally in the presence of an acid catalyst, eg toluenesulphonic acid.

The reaction of process l) may be carried out in a polar solvent for example a C 1 to 6 alkanol, eg methanol or ethanol, at a temperature of from 30°C to the boiling point of the solvent, preferably from 50 to 100°C. The reaction may be carried out in the presence of a base for example a C 1 to 6 alkoxide, eg sodium ethoxide.

The reductive cleavage of process m) may be carried out chemically, eg using zinc and formic acid. The reaction may conveniently be carried out in a solvent which is inert under the reaction conditions, eg acetonitrile. When process m) involves a hydrolysis the hydrolysis may be carried out using conventional techniques known per se.

Process n) may, when it involves an esterification, be carried out using the appropriate alcohol, preferably in excess and in the presence of a dehydrating agent, eg dicyclohexylcarbodiimide, or by using the acid halide, imidazole or the mixed anhydride (which compounds may be prepared by conventional processes known per se). The reaction may conveniently be carried out in a solvent which is inert under the reaction conditions, eg ethyl acetate. When a transesterification is involved the process may be carried out by treating the starting ester with the sodium alcoholate corresponding to the desired ester moiety.

The reaction of process o) may be carried out using a suitable alkylating agent, eg an alkyl halide, preferably an alkyl iodide, at a temperature of from about 20° to 100°C in the presence of a suitable solvent, eg dimethylformamide or dimethyl sulphoxide. The reaction is preferably carried out in the presence of a base, eg potassium hydroxide or sodium hydride.

The reaction of process p) may be carried out at an elevated temperature, eg at reflux, in a solvent which is

inert under the reaction conditions, eg an alkanol such as ethanol.

The reduction of process q) may be carried out using any conventional reducing agent.

The reduction may be carried out using a hydride such as an alkali metal salt of a Group III metal hydride, eg $NaBH_4$ or $LiAlH_4$. The reduction is preferably carried out in the presence of a catalyst, preferably a metal halide for example a transition metal chloride such as titanium tetrachloride. The reduction may be carried out in a solvent which is inert under the reaction conditions, eg 1,2-dimethoxyethane. The solvent is preferably dry. The reduction may be carried out at a temperature of from -20 to 60°C, preferably of from 0 to 40°C and most preferably of from 0 to 20°C.

The reduction may also be carried out by hydrogenation. Hydrogenation is preferably carried out in the presence of a catalyst, for example a metal catalyst, and preferably a transition metal catalyst such as palladium or palladium on charcoal. The reduction may be carried out in a solvent which is inert under the reaction conditions such as an alcohol or a mixture of alcohols, for example an alkyl C 1 to 10 alcohol, or a mixture thereof, eg ethanol or a methanol/ethanol mixture.

The reduction of a compound of formula I, in which X is a group $-CH=NOR_{18}$, and $R_{18}$ is alkyl C 1 to 10, may be carried out using a borane, eg borane-tetrahydrofuran complex or a borane prepared in situ. The reaction may be carried out in a polar solvent, eg an ethereal solvent, for example tetrahydrofuran. The reaction may be carried out at a temperature of from 0°C to the boiling point of the solvent, preferably from 0°C to 100°C, more preferably from 20°C to 100°C and most preferabaly from 25°C to 80°C.

The reaction of process r) may be carried out using any conventional methods for amid formation. We prefer the reaction to be carried out in a solvent which is inert under the reaction conditions, eg dichloromethane, at a temperature of from 0 to 60°C, preferably of from 10 to 40°C and most preferably of from 20 to 30°C. We prefer to react the presence of a coupling agent, eg carbonyldiimidazole.

The resolution of process s) may be carried out by means of conversion of the mixture to, when one of $R_5$, $R_{10}$ and $R_{11}$ carries a -COOH group, a salt with an optically active base or an ester with an optically active alcohol (eg $CCl_3$ $(C_6H_5)CHOH$ or $C_6H_5(OCH_3)CHCH_2OH$), and separation of the product by selective crystallisation, or, preferably, by means of high performance liquid chromatography (HPLC). The separated product may then be converted to the desired optically active acid or ester by conventional means known per se.

Optically active compounds of formula I which do not carry a -COOH group may be resolved by conventional processes known per se.

In process t) the salts may be formed by reacting the free acid, or a salt, ester, amide or derivative thereof, or the free base, or a salt or derivative thereof, with one or more equivalents of the appropriate base or acid. The reaction may be carried out in a solvent or medium in which the salt is insoluble or in a solvent in which the salt is soluble, eg ethanol, tetrahydrofuran or diethyl ether, which may be removed in vacuo, or by freeze drying. The reaction may also be a metathetical process or it may be carried out on an ion exchange resin.

Pharmaceutically acceptable salts of the compounds of formula I include salts with organic or inorganic acids, eg with HCl, HBr, $H_2SO_4$, $H_3PO_4$, methanesulfonic, toluensulfonic, maleic, fumaric, oxalic or camphorsulfonic acids. Also when the molecule contains an acidic group, salts include ammonium salts, alkali metal salts, eg sodium and potassium salts; alkaline earth metal salts, eg the calcium and magnesium salts; salts with organic bases, eg salts with dicyclohexylamine or N-methyl-D-glucamine; and salts with amino acids, eg with arginine, lysine etc. The non-toxic physiologically acceptable salts are preferred, although other salts are also useful, eg in isolating or purifying the product.

The reactions of process u) (Hantzch Synthesis) may be carried out by subjecting the reagents to an elevated temperature, eg of from about 20° to 140°C in the presence or absence of a suitable solvent, eg a lower alkanol. The reaction of process u) may comprise

i) reaction of a compound of formula XIV,

$R_4CHO$ XIV

with compounds of formulae XVIII and XV,

$R_3OOCCH=C(R_2)NH_2$ XVIII

$R_5OOCCH_2COCH_2COCH_2XR_6$ XV

in which formulae $R_2$, $R_3$, $R_4$, $R_5$, X and $R_6$ are as defined above, or

ii) reaction of a compound of formula XIX,

$R_4CH=C(COOR_5)COCH_2XR_6$ XIX

in which $R_4$, $R_5$, X and $R_6$ are as defined above,

with a compound of formula XVIII, or

iii) reaction of compounds of formulae XV, XX and XVII,

$R_4CH=C(COOR_3)COR_2$ XX

iv) reaction of compounds of formulae XIV, XVI and XXI,

$R_5OOCCH=C(R_6)NH_2$ XXI

v) or reaction of compounds of formulae XIX, XVI and XVII, or

vi) reaction of compounds of formulae XIV, XV, XVI and XVII.

In process u)iii) the compound (XVII) may be present as a salt, eg ammonium acetate. In process u) a proportion of an intermediate hydroxy-tetrahydropyridine may be formed and the yield of the desired dihydropyridine may be increased by dehydration of this intermediate, eg using a dehydrating agent such as trifluoroacetic anhydride in a solvent which is inert under the reaction conditions. eg methylene chloride.

The compounds of formula I may also be made from known starting materials using one or more known steps and other processes which are known per se.

The compounds of formula II in which $L_a$ is a halogen, eg bromine may be made by reaction of a corresponding compound of formula II in which $L_a$ is hydrogen, with an appropriate halogenating agent, eg pyridinium bromide perbromide. The reaction may be carried out as described in Example A. We have surprisingly found that the compounds of formula II in which the leaving group is Br are sufficiently stable to be isolated and used as intermediates.

The compounds of formula I in which $R_1$ is hydrogen are useful as intermediates to the compounds of formula I in which $R_1$ is alkyl C 1 to 6.

The compounds of formula I in which $R_6$ is $-CH_2CH_2NH_2$ are useful as intermediates to the compounds of formula I in which $R_6$ is a 2-(1,2,4-triazol-4-yl)ethyl group.

The compounds of formula XIII may be made by reacting a compound of formula XXII,

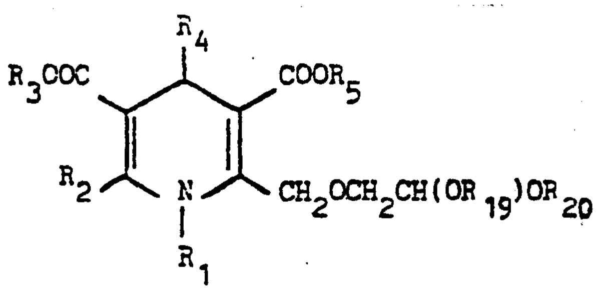

XXII

in which $R_1$, $R_2$, $R_3$, $R_4$ and $R_5$ are as defined above, and

$R_{19}$ and $R_{20}$, which may be the same or different, are each alkyl C 1 to 10 or $R_{19}$ and $R_{20}$ may together form an alkyl C 1 to 10 chain,

with a compound of formula XXIII,

$X_2NOR_{18}$ XXIII

in which $R_{18}$ is as defined above, or a salt thereof.

The compounds of formula XXII may be made by reacting a compound of formula II, with a compound of formula XXIV,

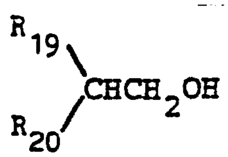

XXIV

in which $R_{19}$ and $R_{20}$ are as defined above.

The compounds of formula I and the intermediates therefore may be isolated from their reaction mixtures using conventional processes, eg crystallisation or chromatography.

Acceptable derivatives of the compounds of formula I include those compounds containing an -NH- group which is protected by a conventional protecting group known per se. Specific protecting groups which may be mentioned are alkyl C 1 to 6, alkylcarbonyl C2 to 7, phenylmethyl, phenylcarbonyl and phthalimide.

The starting materials for the preparation of compounds of formula I are known per se.

Pharmaceutically acceptable esters include esters with C 1 to 10 alcohols, eg alkyl C 1 to 6 esters, preferably ethyl, methyl or 1-methylethyl.

Pharmaceutically acceptable amides include unsubstituted or mono- or di- C 1 to 6 alkyl amides and may be made by conventional techniques, eg reaction of an ester of the corresponding acid with ammonia or an appropriate amine.

By the term alkyl we means a straight or branched alkyl group or a mono-, bi- or tri-cyclic alkyl group.

We prefer $R_2$ to be methyl or substituted alkyl C 1 to 6, more preferably substituted methyl, most preferably trifluoromethyl and particularly fluoromethyl.

We prefer $R_1$ to be methyl or more preferably hydrogen.

$R_3$ is preferably alkyl C to 1 to 4, more preferably alkyl C 1 to 2 and most preferably methyl.

When $R_4$ is a sulphur containing heterocyclic ring we prefer it to be a 5- or 6-membered ring and most preferably a thienyl group. We prefer the thienyl group to be a thien-2-yl group. Preferred substituents on the thien-2-yl group are nitrile, nitro or alkyl C 1 to 6. The alkyl C 1 to 6 group is most preferably methyl. We prefer the thien-2-yl group to be monosubstituted and we prefer the substituent to be in the 3- or 5- position.

We particularly prefer $R_4$ to be a phenyl group and preferably a substituted phenyl group. We prefer $R_4$ to be a phenyl group substituted in one or more of the 2-, 3-and 6- positions. We prefer the substituents to be selected from trifluoromethyl, chloro or fluoro, we particularly prefer $R_4$ to be 2-chlorophenyl or 2,3-dichlorophenyl or a polyfluorinated phenyl group, for example 2,3,5,6-tetrafluorophenyl or pentafluorophe-

nyl. We especially prefer $R_4$ to be 3-chloro-6-fluoro-2-(trifluoromethyl)phenyl.

When $R_4$ is naphthyl we prefer it to be a naphthalen-1-yl group, preferably substituted in the 8-position by halogen. We prefer the halogen to be fluorine.

When $R_5$ is unsubstituted alkyl we prefer it to be alkyl C 1 to 6, more preferably alkyl C 1 to 4 and most preferably 1-methylethyl or ethyl.

When $R_5$ represents substituted alkyl we prefer the alkyl chain to contain from 4 to 10 carbon atoms more preferably from 4 to 8 carbon atoms, most preferably from 5 to 8 carbon atoms and particularly 6 or 7 carbon atoms. We prefer the alkyl chain to be straight and unbranched and uninterrupted.

We prefer the heterocyclic groups $R_{10}$ and $R_{11}$ to be completely unsaturated.

$R_{10}$ is preferably phenyl or a heterocyclic ring containing 3 nitrogen atoms, more preferably two nitrogen atoms. We particularly prefer $R_{10}$ to be a 5-membered ring and most preferably a pyrazole or an imidazole group.

A group of compounds which may be mentioned are those in which X is -NR- or -S(O)$_m$,- and $R_6$ is $-CH_2CH_2NH_2$ or $-CH_2CH_2R_{11}$.

The group of compounds in which X is -O- and $R_6$ is $-CH_2CH_2NR_{14}R_{15}$ or $-CH_2CH_2OCH_2CH_2NR_{14}R_{15}$ may also be mentioned.

A further group of compounds which may be mentioned are those in which X is -NR-, -S(O)$_m$,- or a bond and $R_6$ is $-C(=NR_{12})NHR_{13}$.

The group of compounds in which X is -O- and $R_6$ is $-NH_2$ or $-C(=O)(CH_2)_nNH_2$ may be mentioned.

Compounds in which $-CHZXR_6$ is $-CH_2OCH_2CH_2NH_2$ and $R_5$ is thietanyl may also be mentioned.

A preferred group of compounds are those in which $-CHZXR_6$ is methyl. In particular those in which $-CHZXR_6$ is methyl and $R_5$ is a group $YR_{10}$.

Another preferred group of compounds are those in which $R_5$ is alkyl C 1 to 6 and $R_6$ is $CH_2CH_2NH_2$ or a group $R_{11}$.

A further preferred group of compounds are those in which $R_4$ is a polyfluorinated phenyl group and $R_6$ is 2-((5-amino-1H-1,2,4-triazol-3-yl)amino) ethyl.

$R_5$ may represent the following groups:

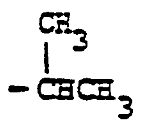

$-CH_2CH_3$

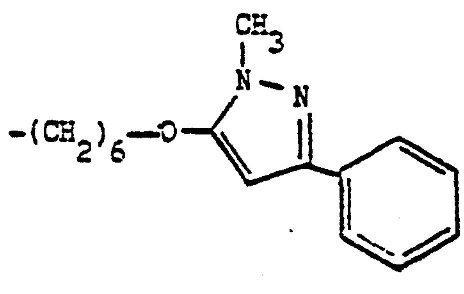

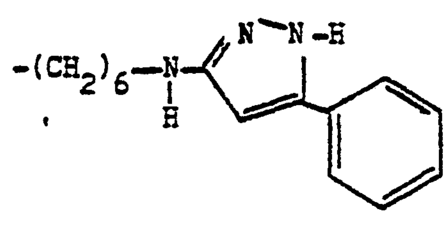

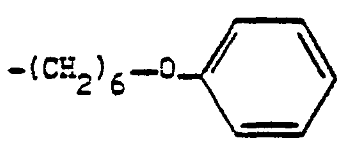

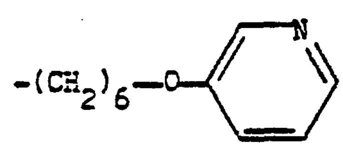

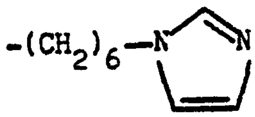

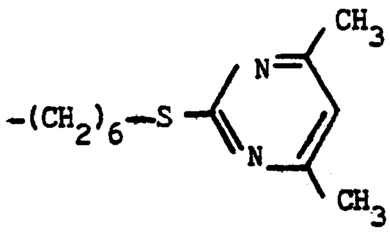

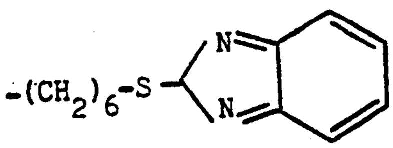

$-(CH_2)_7-COOCH_2CH_3$

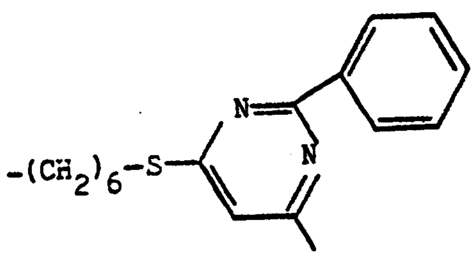

$-(CH_2)_7-COOH$

$-(CH_2)_7-COOCH_3$

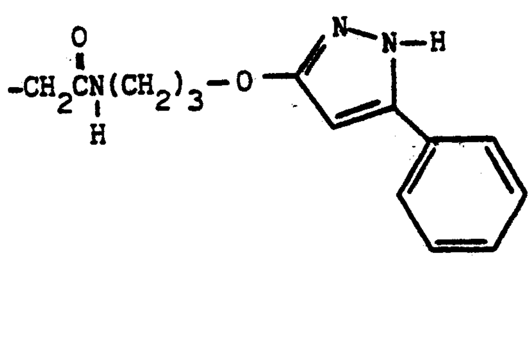

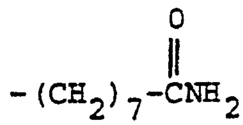

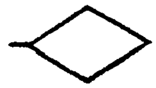

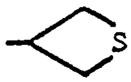

$-CH_2CF_3$

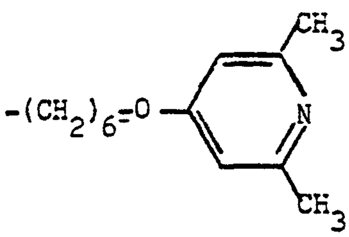

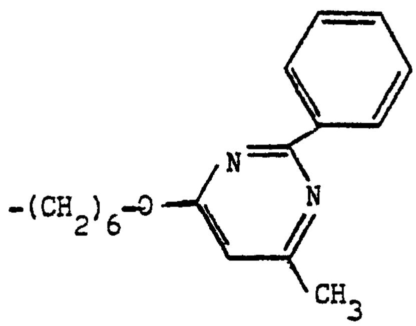

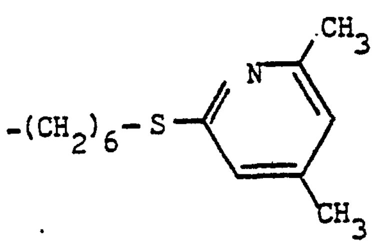

$-CH_2CCl_3$

We prefer compounds in which $-CHZXR_6$ is a group $-CH_2XR_{11}$ or $-CH_2XCH_2CH_2NH_2$. The group $-CHZXR_6$ may represent the following:

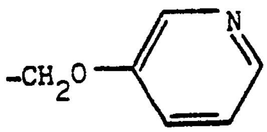

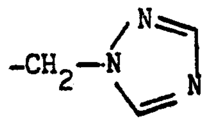

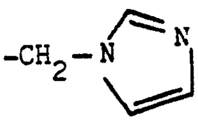

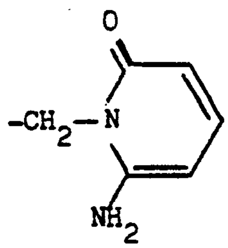

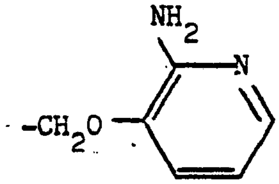

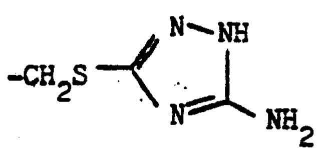

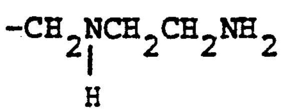

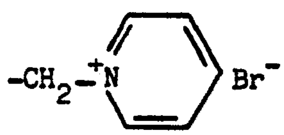

$-CH_2SCH_2CH_2NH_2$

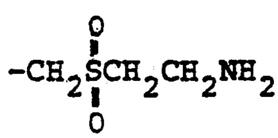

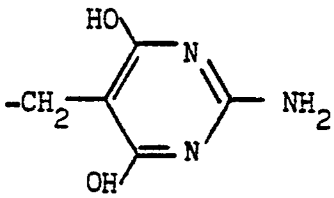

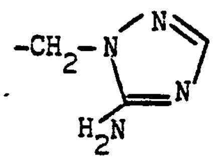

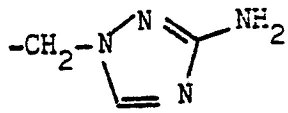

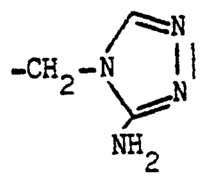

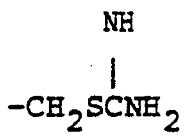

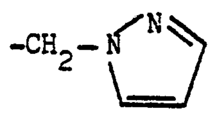

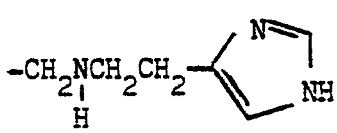

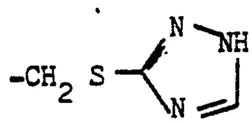

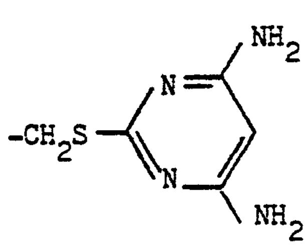

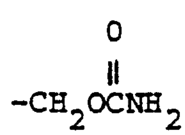

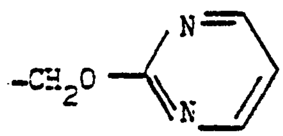

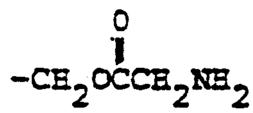

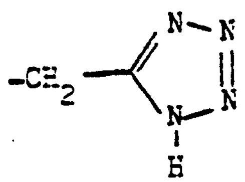

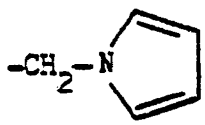

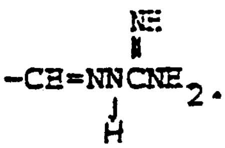

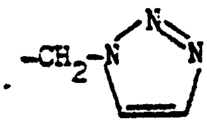

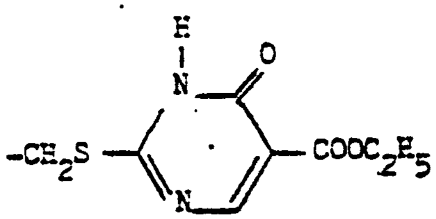

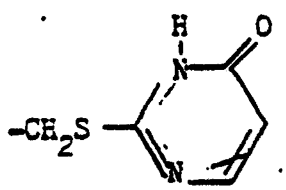

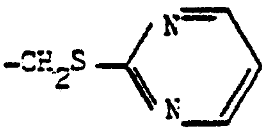

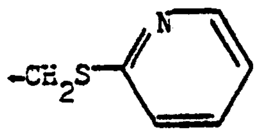

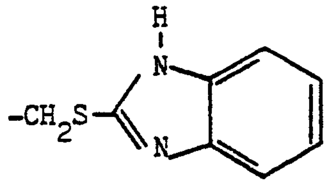

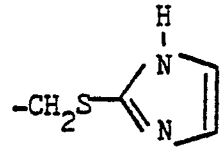

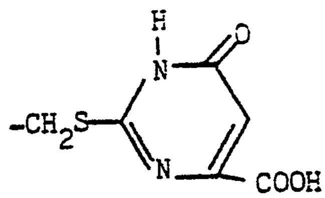

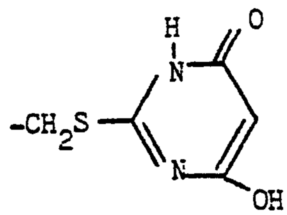

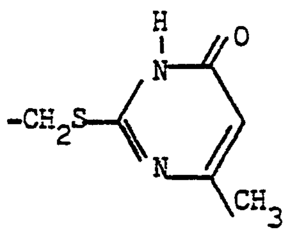

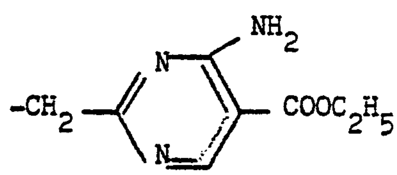

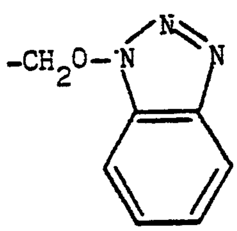

$-CH_2O-NH_2$

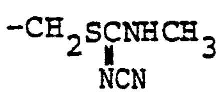

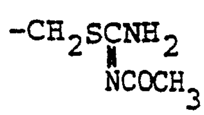

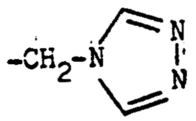

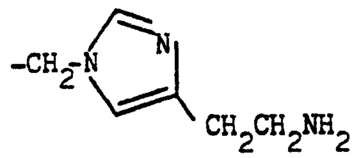

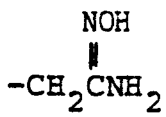

$-CH_2OCH_2CH_2OCH_2CH_2NH_2$

$-CH_2OCH_2CH_2NH_2$

$-CH_3$

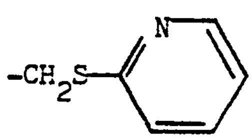

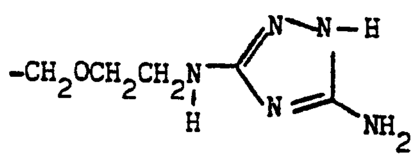

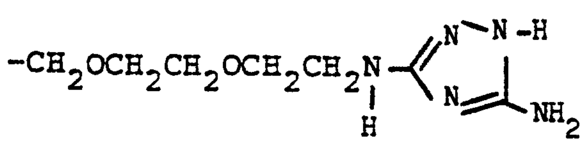

When $R_{11}$ is a 6-membered ring we prefer it to comprise less than 4 N atoms and preferably less than 3. We particularly prefer the 6-membered ring to be a pyrimidine or a pyridine group. When $R_{11}$ is a pyrimidine

group we prefer it to be a pyrimidin-2-yl group and preferably a 4-oxo-3,4-dihydro-pyrimidin-2-yl group. When $R_{11}$ is a pyrimidine group we further prefer X to be S. When $R_{11}$ is a pyridine group we prefer it to be unsubstituted. We particularly prefer the pyridine group to be a pyridin-2-yl group. When $R_{11}$ is pyridinyl we prefer X to be S or O.

When $R_{11}$ is a 5-membered ring we prefer it to be pyrrole or a triazole. When $R_{11}$ is pyrrole we prefer it to be a pyrrol-1-yl group, we prefer the pyrrole group to be unsubstituted. When $R_{11}$ is a triazole we prefer the triazole to be 1,2,4-triazole and more preferably a 1,2,4-triazol-4-yl group.

We particularly prefer $CHZXR_6$ to be one of the following groups:

$CH_2S(CH_2)_2NH_2$ $CH_2SO_2(CH_2)_2NH_2$

$CH_2SC(=NH)NH_2$

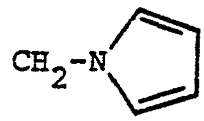

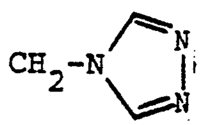

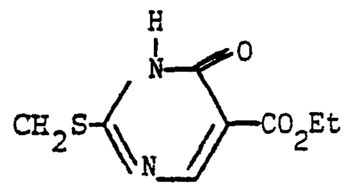

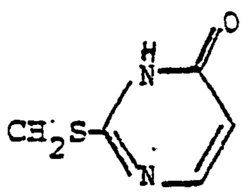

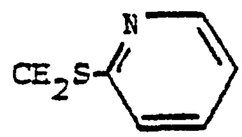

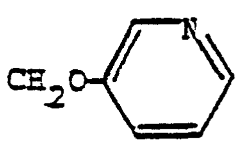

$-CH_3$

$CH_2OCH_2CH_2NH_2$

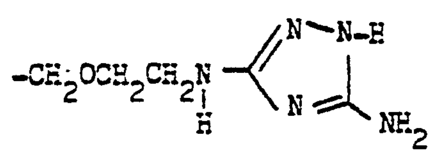

The compounds of formula I, and the pharmaceutically acceptable salts thereof, are useful because they exhibit pharmacological properties in animals. More particularly they block the entry of calcium into vascular and cardiac muscle leading to falls in blood pressure, inotropy and heart rate. They are active in the following systems:-

(a) Relaxation of contracted vascular smooth muscle. Van Breemen, Aaronson, Loutzenhiser and Meisheri, Chest, 78, Supplement, 157-165, 1980.

(b) Reduction of inotropy and chronotropy of isolated atria. Henry, Excerpta Med. Int. Congr. Ser., 474, 14-23, 1979.

(c) Reduction of blood pressure and increased cardiac output in anaesthetised dogs. Hirakawa, Ito, Kondo, Watanbe, Hiei, Banno & Hyase, Arzneim-Forsch, 22, 344-349, 1972.

(d) Reduction of blood pressure in conscious dogs when given by the intravenous and oral routes. Newman, Bishop, Peterson, Leroux & Horowitz, J Pharm. Exp. Ther. 201, 728-730, 1977.

The compounds are thus indicated for use in the treatment of renovascular. malignant or essential hypertension (including hypertensive emergencies), pulmonary hypertension, vasospastic angina, chronic stable angina and congestive heart failure. Other indications are the treatment of renal failure, cardiac arrhythmias, hypertrophic cardiomyopathy, cerebrovascular diseases (including cerebral haemorrhage, ischaemia and vasospasm, migraine, altitude sickness and hearing loss), peripheral vascular diseases (including Raynauds syndrome, intermittent claudication and digital ulceration); use as a cardioplegic agent during surgery eg in cardiopulmonary bypass, and for the treatment of, and protection against, myocardial infarction and ischaemia.

By virtue of their ability to inhibit calcium entry into other cells and tissues the compounds are also indicated in the treatment of thrombosis, atherosclerosis, respiratory diseases (including asthma and bronchitis) glaucoma, aldosteronism, uterine hypermotility and for the relief of oesophageal and skeletal muscle spasm.

For the above uses the dosage will depend upon the compound used, the route of administration and the effect desired, but in general will be in the range of 0.1-10mg per kilogram body weight per day. For man the indicated total daily dose will be from about 5-500mg, preferably from 5- 200mg and more preferably from 5 to 100mg, which may be administered preferably once daily, or in divided doses of about 1-200mg, preferably 2 to 25 mg, eg 2 to 4 times per day.

The compounds of formula I are also useful as intermediates for the production of other pharmacologically active compounds.

The compounds of formula I are advantageous in that they possess greater potency (eg with respect to hypotensive and direct negative chronotropic effects), produce a lower level of reflex tachycardia, are more selective (eg for vascular smooth muscle vs cardiac muscle), produce less depression of cardiac contractility, are longer acting, have a slower onset of action, are more readily absorbed or less readily metabolised, are more easily formulated, possess less, or less undesirable, side effects, are more stable or have other more beneficial properties than known compounds of similar structure.

The compounds of the invention may be administered by a wide variety of routes and may act systemically or locally. Thus the compounds may be administered by oral or nasal inhalation to the lung, to the buccal cavity, osesophageally, rectally, topically to the skin, the eye or to other available surfaces of the body; by injection, eg intravenously, intramuscularly, intraperitoneally, or by surgical implant.

According to our invention we also provide a pharmaceutical composition comprising preferably less than 80%, more preferably less than 50%, eg 1 to 20%, by weight of a compound of formula I, or a pharmaceutically acceptable salt thereof, in admixture with a pharmaceutically acceptable adjuvant, diluent or carrier.

Thus the compound may be put up as a tablet, capsule, dragee, suppository, suspension, solution, injection, implant, a topical, eg transdermal, preparation such as a gel, cream, ointment, aerosol, or a polymer system, or an inhalation form, eg an aerosol or a powder formulation.

We prefer compositions which are designed to be taken oesophageally and to release their contents in the gastrointestinal tract. Thus we prefer tablets which may, for example, be made by direct compression. In such a process the active ingredient is mixed with one or more of modified forms of starch, calcium phosphate, a sugar eg lactose, microcrystalline cellulose and/or other directly compressible excipients, together with lubricant(s), eg stearic acid or magnesium stearate, flow aid(s), eg talc or colloidal silicon dioxide, and disintegrant(s), eg starch, substituted sodium carboxymethyl cellulose, cross linked sodium carboxy methyl cellulose, carboxy methyl starch or cross linked polyvinylpyrrolidone. Tablets are then formed by direct compression, and may be sugar or film coated eg with hydroxypropylmethylcellulose.

Alternatively the active ingredient may be granulated before tabletting. In such cases the active ingredient is mixed with one or more of starch, calcium phosphate, a sugar eg lactose, microcrystalline cellulose or other suitable excipients and granulated with a binder such as starch, pregelled starch, polyvinylpyrrolidone, gelatine, a modified gelatine, or a cellulose derivative, eg hydroxypropylmethylcellulose. The mass is then dried, sieved and mixed with lubricant(s), flow aid(s) and disintegrant(s), such as described in the previous paragraph. Tablets are then formed by compression of the granules, and may be sugar or film coated, eg with hydroxypropylmethylcellulose.

As a further alternative a powder, blend or granules, such as are described above as intermediates in tabletting, may be filled into a suitable, eg gelatine, capsule.

In order to improve the bioavailability, or decrease variability of availability, of the active ingredient the compound may be:-

a) dissolved in a suitable solvent, eg polyethylene glycol, Gelucaire, arachis oil, a (hydrogenated) vegetable oil or beeswax and the solution is then filled into a gelatine capsule,

b) produced as a spray-dried or freeze-dried form prior to mixing with other excipients,

c) milled and/or micronised to produce a powder with a large surface area prior to mixing with other excipients,

d) made into a solution and distributed over an inert excipient having a large surface area, eg colloidal silicon dioxide. The solvent is evaporated and further excipients added,

e) formed into a complex with cyclodextrin prior to mixing with other excipients. This complex also assists increasing light stability, or

f) made into a solid solution or co-precipitated, eg with polyvinylpyrrolidone, polyethyleneglycol, modified cellulose, hydroxypropylmethylcellulose, urea or a sugar prior to mixing with further excipients.

The compounds, either in their normal form or in a modified form, eg as described immediately above, may

be formulated in a controlled release form. Thus the compound may be dispersed, or contained in, a polymer matrix formed from, for example, ethylcellulose, hydroxypropylmethylcellulose or an acrylate/methacrylate polymer. Alternatively the compound may be formulated as a tablet or beads which are surrounded by a semi-permeable membrane. eg shellac, ethylcellulose or an acrylate/methacrylate polymer.

The compounds of this invention may be given in combination with other pharmaceutically active compounds, eg diuretics, beta-blockers, antihypertensives or inotropic agents. The dosage of the other pharmaceutically active compound can be that conventionally used when the compound is administered on its own, but is preferably somewhat lower. To illustrate these combinations, a compound of this invention effective in the range, eg 5-100 milligrams per day, can be combined at levels ranging, eg for 1-200 milligrams per day with the following beta-blockers, antihypertensives and diuretics in dose ranges per day as indicated: hydrochlorothiazide (15-200mg), chlorothiazide (125-2000mg), ethacrynic acid (15-100mg), amiloride (5-20mg), furosemide (5-80mg), propanolol (20-480mg), timolol (5-50mg), captopril (10-500mg), methyldopa (65-2000mg) or digoxin (0.1-0.5mg). In addition, the triple drug combinations of hydrochlorothiazide (15-200mg) plus amiloride (5-20mg) plus a compound of this invention (3-200mg) and hydrochlorothiazide (15-200mg) plus timolol (5-50mg) plus a compound of this invention (3-200mg), are provided. The above dose ranges may be adjusted on a unit basis as necessary to permit divided daily dosage. Also, the dose may vary depending on the severity of the disease, weight of patient and other factors which a person skilled in the art will recognise.

Certain of the compounds of formula I are assymetric and exhibit optical isomerism. Such compounds may be separated into their optical isomers, or may be made by stereospecific syntheses, using conventional techniques know per se.

The invention is illustrated, but in no way limited by the following Examples in which temperatures are in degrees centigrade.

Example 1

3-Methyl 5-(1-methylethyl) 4-(3-chloro-6-fluoro-2-(trifluoromethyl)phenyl)-2-(fluoromethyl)-1,4-dihydro-6-(pyridin-3-yloxymethyl)-3,5-pyridinedicarboxylate hydrochloride

5-Methyl 3-(1-methylethyl) 2-(bromomethyl)-4-(3-chloro-6-fluoro-2-(trifluoromethyl)phenyl)-6-(fluoromethyl)-1,4-dihydro-3,5-pyridinedicarboxylate (2.4g, 4.3mmoles) in acetonitrile (10ml) was added to a stirred solution of pyridin-3-ol (0.814g, 8.6mmoles) and sodium methoxide (0.23g, 4.3mmoles) in acetonitrile (40ml). After stirring for 3 hours, the reaction mixture was poured onto water (150ml). The organic layer was separated, washed with dilute hydrochloric acid, dried ($MgSO_4$) and the solvent was evaporated. Recrystallisation from dichloromethane/ethyl acetate gave the title compound (0.34g), mp 216-7°.

Example 3

3-Methyl 5-(1-methylethyl) 4-(3-chloro-6-fluoro-2-(trifluoromethyl)phenyl)-2-(fluoromethyl-1,4-dihydro-6-((1H-1,2,4-triazol--1-yl)methyl)-3,5-pryridinedicarboxylate

5-Methyl 3-(1-methylethyl) 2-(bromomethyl)-4-(3-chloro-6-fluoro-2-(trifluoromethyl)phenyl)-6-(fluoromethyl)-1,4-dihydro-3,5-pyridinedicarboxylate (1.16g, 2.1mmoles) in acetonitrile (10ml) was added to a stirred solution of 1,2,4-triazole (0.3g, 4.35mmoles) and sodium methoxide (0.235g, 4.35mmoles) in actonitrile (10ml). After 6 hours, the reaction mixture was poured onto ethyl acetate/water. The organic layer was separated, dried ($Na_2SO_4$) and the solvent evaporated. Chromatography on silica eluting with ethyl acetate/petroleum ether (60-80°) and then ethyl acetate/methanol gave the title compound (0.27g), mp 158-60° (dec).

Example 3

5-Methyl 3-(1-methylethyl) 2-((2-aminoethyl) aminomethyl)-4-(3-chloro-6-fluoro-2-(trifluoromethyl) phenyl)-6-(fluoromethyl)-1,4-dihydro-3,5-pyridinedicarboxylate bis-((Z)-2-butenedioate)hemihydrate

5-Methyl 3-(1-methylethyl) 2-(bromomethyl)-4-(3-chloro-6-fluoro-2-(trifluoromethyl)phenyl)-6-(fluoromethyl)-1,4-dihydro-3,5-pyridinedicarboxylate (0.7g, 1.28mmoles) in chloroform (25ml) was added to a solution of 1,2-ethanediamine (2.5g, 42mmoles) in chloroform (25ml). After 1 hour at room temperature, the solution was washed with saturated brine (x 6), dried ($Na_2SO_4$) and the solvent was evaporated. The resulting compound was converted to the title bismaleate salt (0.52g) which was crystallised from ethanol, mp 146-9° (dec).

Example 4

5-Methyl 3-(1-methylethyl) 2-((2-aminoethyl) thiomethyl)-4-(3-chloro-6-fluoro-2-(trifluoromethyl)phenyl)-6-(fluoromethyl)-1,4-dihydro-3,5-pyridinedicarboxylate (Z)-2-butenedioate 1/4 hydrate

The product from Example CT (4.4g. 6.5mmoles), hydrazine hydrate (3ml) and ethanol (100ml) were heated

at reflux for 20 minutes. The ethanol was evaporated and the residue dissolved in ethyl acetate and saturated sodium chloride solution. The organic layer was separated and the aqueous layer was extracted twice with ethyl acetate. The combined organic extracts were dried ($MgSO_4$) and the solvent evaporated. The maleate salt was prepared in ethanol and crystallised on addition of ether to give the title compound (2.25g), mp 148-50°.

Example 5

5-Methyl-3-(1-methylethyl)-2-((2-amino-4,6-dihydroxypyrimidin-5-yl)methyl)-4-(3-chloro-6-fluoro-2-(tri-fluoromethyl)phenyl-6-(fluoromethyl)-1,4-dihydro-3,5-pyridinedicarboxylate

5-methyl 3-(1-methylethyl) 4-(3-chloro-6-fluoro-2-(trifluoromethyl)phenyl)-2-(3-ethoxy-2-(ethoxycarbonyl))-3-oxopropyl)-6-(fluoromethyl)-1,4-dihydro-3,5-pyridinedicarboxylate (0.94g, 1.5mmoles), guanidine hydrochloride (0.435g, 4.5mmoles) and sodium methoxide (0.245g, 4.5mmoles) in methanol (25ml) were heated at reflux for 2 hours. The reaction mixture was poured onto water, made slightly acidic with acetate acid and the product extracted with ethyl acetate (x3). The combined organic extracts were dried ($Na_2SO_4$) and the solvent evaporated. Tituration with ether gave the title compound (0.61g), mp 243-4° (dec).

Example 6

3-Methyl 5-(1-methylethyl) 4-(3-chloro-6-fluoro-2-(trifluormethyl)phenyl)-2-(fluoromethyl)-1,4-dihydro-6-((1H-pyrazol-1-yl)methyl)-3,5-pyridinedicarboxylate

5-Methyl 3-(1-methylethyl) 2-(bromomethyl)-4-(3-chloro-6-fluoro-2-(trifluoromethyl)phenyl)-6-(fluoromethyl)-1,4-dihydro-3,5-pyridinedicarboxylate (2.33g, 4.28mmoles) in acetonitrile (10ml) was added to a stirred mixture of pyrazole (0.575g, 8.6mmoles) and sodium methoxide (0.46g, 8.5mmoles) in acetonitrile (20ml). After stirring for 18 hours, the reaction mixture was poured onto brine and extracted with ethyl acetate. The organic extract was dried ($MgSO_4$) and the solvent evaporated.
Chromatography on silica eluting with ethyl acetate/petroleum ether (60-80°) mixtures followed by crystallisation from petroleum ether (60-80°) gave the title compound (0.87g), mp 102-4°.

Example 7

5-Methyl 3-(1-methylethyl) 2-(((aminocarbonyl)oxy) methyl)-4-(3-chloro-6-fluoro-2-(trifluoromethyl)phenyl)-6-(fluoromethyl)-1,4-dihydro-3,5-pyridinedicarboxylate

Chlorosulphonyl isocyanate (0.25ml, 2.9mmoles) was added to a stirred suspension at -43° of 3-methyl 5-(1-methylethyl) 4-(3-chloro-6-fluoro-2-(trifluoromethyl) phenyl)-2-(fluoromethyl)-1,4-dihydro-6-(hydroxymethyl)-3,5-pyridinedicarboxylate (1.4g, 2.9mmoles) in dichloromethane (100ml). The reaction mixture was allowed to warm to -10° and the stirring continued for 1.5 hours. Brine was added and after 30 minutes, the organic layer was separated. The aqueous layer was extracted with dichloromethane (2 x 100ml) and the combined organic extracts dried ($MgSO_4$). The solvent was evaporated and the residue purified by chromatography on silica eluting with dichloromethane/ethyl acetate mixtures. Crystallisation from acetone/petroleum ether (60-80°) gave the title compound (0.18g), mp 162°.

Example 8

3-Methyl 5-(1-methylethyl) 4-(3-chloro-6-fluoro-2-(trifluoromethyl)phenyl)-2-(fluoromethyl)-1,4-dihydro-6-(pyrimidin-2-yloxymethyl)-3,5-pyridinedicarboxylate

Sodium hydride (1g of 50%, 20mmoles) was added to a solution of 3-methyl 5-(1-methylethyl) 4-(3-chloro-6-fluoro-2-(trifluoromethyl)phenyl)-2-(fluoromethyl)-1,4-dihydro-6-(hydroxymethyl)-3,5-pyridinedicarboxylate (4.9g, 10mmoles) in tetrahydrofuran (150ml) at 0°. After 20 minutes, 2-chloropyrimidine (1.38g, 1.24mmoles) was added. After 2 hours at 0°, dilute hydrochloric acid was added and the product extracted out with ethyl acetate. The organic extract was dried ($MgSO_4$) and the solvent evaporated. HPLC on silica eluting with ethyl acetate/petroleum ether (60-80°) mixtures followed by crystallisation from hexane/propan-2-ol gave the title compound (0.18g), mp 162-3°.

Example 9

5-Methyl 3-(1-methylethyl) 2-((aminoacetyloxy)methyl)-4-(3-chloro-6-fluoro-2-(trifluoromethyl)phenyl)-6-(fluoromethyl)-1,4-dihydro-3,5-pyridinedicarboxylate maleate salt

5-Methyl 3-(1-methylethyl) 4-(3-chloro-6-fluoro-2-(trifluoromethyl)phenyl)-2-(((((1,1-dimethylethoxy) carbonyl)amino)acetyloxy)methyl)-6-(fluoromethyl)-1,4-dihydro-3,5-pyridinedicarboxylate (0.7g, 1.09mmoles) was dissolved in trifluoroacetic acid (1.5ml). After 15 minutes, the solvent was evaporated and the residue dissolved in diethyl ether and saturated sodium bicarbonate solution. The ethereal extract was dried ($Na_2SO_4$)

and the solvent evaporated. Chromatography on silica eluting with ethyl acetate/petroleum ether (60-80°) followed by maleate Salt formation and crystallisation from methanol/diethyl ether gave the title compound (0.05g). M+1+ 541/3; NMR ($D_6$DMSO) 9.05 (s.1H), 3.90 (s,2H), 3.49 (s,3H).

Example 10

3-Methyl 5-(1-methylethyl) 4-(3-chloro-6-fluoro-2-(trifluoromethyl)phenyl)-2-(fluoromethyl)-1,4-dihydro-6-((1H-tetrazol-5-yl)methyl)-3,5-pyridinedicarboxylate

5-Methyl 3-(1-methylethyl) 4-(3-chloro-6-fluoro-2-(trifluoromethyl)phenyl)-2-(cyanomethyl)-6-(fluoromethyl)- 1.4-dihydro-3,5-pyridinedicarboxylate (1.88g, 3.8mmoles), ammonium chloride (0.61g, 11.4mmoles), sodium azide (0.74g, 11.4mmoles) and dimethylformamide (50ml) were heated at 70° for 3 days. The cooled reaction mixture was poured onto water and the product extracted out with ether. The ethereal extract was washed with water, dried ($Na_2SO_4$) and the solvent evaporated. Chromatography on silica eluting with dichloromethane/ethanol mixtures followed by crystallisation from ethyl acetate/petroleum ether (60-80°) gave the title compound (0.15g), mp 186-7° (dec).

Example 11

3-Methyl 5-(1-methylethyl) 4-(3-chloro-6-fluoro-2-(trifluoromethyl)phenyl)-2-(fluoromethyl)-1,4-dihydro-6-(1H-pyrrol-1-ylmethyl)-3,5-pyridinedicarboxylate

5-Methyl 3-(1-methylethyl)2-(aminomethyl)-4-(3-chloro-6-fluoro-2-(trifluoromethyl)phenyl)-6-(fluoromethyl)-1,4-dihydro-3,5-pyridinedicarboxylate (0.8g, 1.65mmoles), 2,5-dimethoxytetrahydrofuran (2ml) and acetic acid (10ml) were heated at 80° for 5 minutes. The solvent was evaporated and the residue dissolved in diethyl ether. This solution was washed with saturated sodium bicarbonate solution, dried ($Na_2SO_4$) and the solvent evaporated. Chromatography on silica eluting with ethyl acetate/petroleum ether (60-80°) mixtures followed by crystallisation gave the title compound (0.32g), mp 130-2°.

Example 12

5-Methyl 3-(1-methylethyl) 2-(((aminoiminomethyl) hyrazono)methyl)-4-(3-chloro-6-fluoro-2-(trifluoromethyl)phenyl)-6-(fluoromethyl)-1,4-dihydro-3,5-pyridinedicarboxylate

3-Methyl 5-(1-methylethyl) 4-(3-chloro-6-fluoro-2-(trifluoromethyl)phenyl)-2-(fluoromethyl)-6-formyl-1,4-dihydro-3,5-pyridinedicarboxylate (0.27g, 0.56mmoles) was added to a solution of aminoguanidine sulphate (0.14g, 0.56mmoles) and the sodium methoxide (0.065g, 1.2mmoles) in ethanol (20ml). The mixture was heated at reflux for 1 hour, cooled and poured into water (100ml). The solid was filtered off and dried to give the title compound (0.2g). M+1+ 538/40; NMR ($D_6$DMSO) delta 9.5 (s,1H), 5.9 (q,1H), 3.5 (s,3H).

Example 13

3-Methyl 5-(1-methylethyl) 4-(3-chloro-6-fluoro-2-(trifluoromethyl)phenyl)-2-(fluoromethyl)-1,4-dihydro-6-(1H-1,2,3-triazol-1-yl)methyl-3.5-pyridinedicarboxylate

5-Methyl 3-(1-methylethyl) 2-(azidomethyl)-4-(3-chloro-6-fluoro-2-(trifluoromethyl)phenyl)-6-(fluoromethyl)-1.4-dihydro-3,5-pyridinedicarboxylate (10.g), ethenyl acetate (10ml) and 1,4-benzenediol (0.02g) in dry toluene (10ml) was kept at 70-75° for 5 days. The solution was washed with water and evaporated and the residue separated by chromatography on silica using ethyl acetate/petroleum ether mixtures as eluant to give the title compound (0.55g), mp 133-4°.

Example 14

5-Methyl 3-(1-methylethyl) 4-(3-chloro-6-fluoro-2-(trifluoromethyl)phenyl)-2-((5-ethoxycarbonyl-3,4-dihydro-4-oxopyrimidin-2-ylthio)methyl)-6-(fluoromethyl)-1,4-dihydro-3,5-pyridinedicarboxylate

5-Methyl 3-(1-methylethyl) 2-(bromomethyl)-4-(3-chloro-6-fluoro-2-(trifluoromethyl)phenyl)-6-(fluoromethyl)-1,4-dihydro-3,5-pyridinedicarboxylate (50.g, 9.1mmoles) in dimethylformamide (20ml) was added to 5-carbethoxy-2-thiouracil (1.8g, 9.0mmoles) and sodium methoxide (0.49g, 9.1mmoles) in dimethylformamide (20ml). The solution was stirred for 4 hours, then poured into water and extracted with ethyl acetate. Evaporation of the extracts and trituration with ether-petroleum ether mixtures afforded the title compound as a complex with 0.33mmoles of dimethylformamide (4.0g), mp 142-4°. Recrystallisation from 95% ethanol gave bright yellow crystals of the title compound hemihydrate (3.3g), mp 118-120° (dec).

Example 15

3-Ethyl 5-methyl 2-((1H-benzoltriazol-1-yloxy) methyl)-4-(2,3-dichlorophenyl)-6-(fluoromethyl)-1,4-dihydro-3,5-pyridinedicarboxylate

3-Ethyl 5-methyl 2-(bromomethyl)-4-(2,3-dichloro-phenyl)-6-(fluoromethyl)-1,4-dihydro-3,5-pyridinedicarboxylate (1.0g, 2.08mmoles), 1H-benzotriazol-ol (0.28g, 2.08mmoles) and triethylamine (0.3ml) in dry dimethyl/formamide (20ml) was stirred at 20° for 20 minutes, then poured into water and ethyl acetate. The organic phase was separated and evaporated and the residue triturated with methanol to give the title compound as a pale yellow solid (0.98g), mp 202-3°.

Example 16

3-Ethyl 5-methyl 2-((aminoiminomethyl)thiomethyl)-4-(2.3-dichlorophenyl)-6-(fluoromethyl)-1,4-dihydro-3,5-pyridinedicarboxylate hydrobromide

3-Ethyl 5-methyl 2-(bromomethyl)-4-(2,3-dichloro-phenyl-6-(fluoromethyl)-1,4dihydro-3,5-pyridinedicarboxylate (1.25g, 2.5mmoles) and thiourea (1.90g, 2.5mmoles) in dry acetonitrile (20ml) were stirred at 20° for 18 hours. The precipitated solid was collected, washed with cold acetonitrile and ether to give the title salt (0.55g), mp 166-7° (dec).

Example 17

5-Ethyl 3-methyl 4-(2-chlorophenyl)-2-(fluoromethyl)-1,4-dihydro-6-((4H-1,2,4-triazol-4-yl)methyl)-3,5-pyridinedicarboxylate

3-Ethyl 5-methyl 2-(aminomethyl)-4-(2-chlorophenyl)-6-(fluoromethyl)-1,4-dihydro-3,5-pyridinedicarboxylate (1.4g, 3.7 mmoles), $N^1$-[(dimethylamino)methylene]-N,N-dimethylmethanehydrazonamide (0.52g, 3.7mmoles) and 4-methylbenzenesulphonic acid(0.01g) in dry toluene (70ml) was heated at reflux for 20 hours. The cooled solution was washed with saturated sodium bicarbonate solution and brine and evaporated. Chromatography of the residue on silica eluting with ethyl acetate/methanol mixture afforded the title compound (0.31g) as cream crystals, mp 197-8°.

Example 18

5-Methyl 3-(1-methylethyl)2-[2-amino-2-(hydroximino) ethyl]-4-(3-chloro-6-fluoro-2-(trifluoromethyl)phenyl)-6-(fluoromethyl)-1,4-dihydro-3,5-pyridinedicarboxylate hydrochloride

A mixture of 5-methyl 3-(1-methylethyl) 4-(3-chloro-6-fluoro-2-(trifluoromethyl)phenyl)-2-(cyanomethyl)-6-(fluoromethyl)-1,4-dihydro-3,5-pyridinedicarboxylate (1.0g, 2.03mmoles), hydroxylamine hydrochloride (10.5g, 2.16mmoles) and sodium methoxide ( 0 11g, 2.03mmoles) in dry methanol (20ml) was heated at reflux for 8 hours. Removal of the solvent under reduced pressure and chromatography of the residue on silica using diethyl ether as eluant afforded a product which after conversion to the hydrochloride in the usual way gave the title compound (0.3g), mp 179-80° (dec).

Example 19

3-Methyl 5-(6-(1-methyl-3-phenylpyrazol-5-yloxy) hexyl) 4-(3-chloro-6-fluoro-2-(trifluoromethyl)phenyl)-2-fluoromethyl)-1,4-dihydro-6-methyl-3,5-pyridinedicarboxylate

Phosphorous pentachloride (0.835g, 4mmoles) was added with stirring to 4-(3-chloro-6-fluoro-2-(trifluoromethyl) phenyl)-6-(fluoromethyl)-1,4-dihydro-5-(methoxycarbonyl)-2-methyl-3-pyridinecarboxylic acid (1.7g, 4mmoles) in dichloromethane (60ml). After 1 hour, 6-(2-methyl-5-phenyl pyrazol-3-yloxy hexan-1-ol (1.1g, 4mmoles) was added and the reaction mixture stirred for 16 hours. Aqueous sodium carbonate was added with stirring. After 1 hour the organic layer was separated, washed with water and brine, and dried ($MgSO_4$). The solvent was evaporated and the residue was chromatographed on silica eluting with ethyl acetate/dichloromethane mixtures to give the title compound (0.3g) as a foam, mp 55-60° (softens).

Example 20

3-Methyl 5-(6-phenoxyhexyl) 4-(3-chloro-6-fluoro-2-(trifluoromethyl)phenyl)-2-(fluoromethyl)-1,4-dihydro-6-methyl-3,5-pyridinedicarboxylate

6-Phenoxyhexyl 3-oxobutanoate (2.78g, 10mmoles), ammonium acetate (1.0g, 13mmoles) and t-butanol) (15ml) were heated at 60° for 3 hours. Methyl 4-fluoro-3-oxobutanoate (1.34g, 10mmoles) and 3-chloro-6-fluoro-2-(trifluoromethyl)benzaldehyde (2.26g, 10mmoles) were added and the heating continued at 50° for 6 days. The solvent was evaporated and the residue purified by chromatography on silica eluting with ethyl acetate/petroleum ether (60-80°) mixtures. Crystallisation from acetone/petroleum ether (60-80°) gave

the title compound (0.9g), mp 126-8°.

Example 21

3-Methyl 5-(6-(6-methyl-2-phenylpyrimidin-4-ylthio) hexyl) 4-(3-chloro-6-fluoro-2-(trifluoromethyl)phenyl)-2-fluoromethyl)-1,4-dihydro-6-methyl-3,5-pyridinedicarboxylate

3-Chloro-6-fluoro-2-(trifluoromethyl)benzaldehyde (0.8g, 3.5mmoles), 6-(6-methyl-2-phenylpyrimidin-4-ylthio)hexyl 3-amino-2-butenoate (1.35g, 3.5mmoles), methyl 4-fluoro-3-oxobutanoate (0.47g, 3.5mmoles) and t-butanol (15ml) were heated at 50° for 5 days. The solvent was evaporated and the residue, dissolved in dichloromethane (15ml), was treated with trifluoroacetic anhydride (0.2ml). After 10 minutes, saturated sodium bicarbonate solution was added. The organic layer was separated, dried ($Na_2SO_4$) and the solvent was evaporated. Chromatography on silica eluting with ethyl acetate/petroleum ether (60-80°) followed by crystallisation from cyclohexane/diethyl ether gave the title compound (0.9g), mp 129-30°.

Example 22

3-Methyl 5-(6-(1-methyl-5-phenylpyrazol-3-yloxy)hexyl) 4-(3-chloro-6-fluoro-2-(trifluoromethyl)phenyl)-2-(fluoromethyl)-1,4-dihydro-6-methyl-3,5-pyridinedicarboxylate

1,2-Dihydro-1-methyl-5-phenyl-3H-pyrazol-3-one (0.46g, 2.64mmoles) was added to a solution of sodium hydride (0.12g of 50%, 2.5mmoles) in dimethylsulphoxide (40ml). After 45 minutes, 3-methyl 5-(6-(((4-methylphenyl) sulphonyl)oxy)hexyl) 4-(3-chloro-6-fluoro-2-(trifluoromethyl)phenyl)-2-(fluoromethyl)-1,4-dihydro-6-methyl-3,5-pyridinedicarboxylate (1.75g, 2.6mmoles) in dimethylsulphoxide (5ml) was added and the resulting solution was stirred for 24 hours. The reaction mixture was poured onto dilute hydrochloric acid and extracted with ethyl acetate. The organic extract was washed with water and brine, dried ($MgSO_4$) and the solvent was evaporated. Purification by chromatography on silica eluting with ethyl acetate/dichloromethane mixtures followed by crystallisation from cyclohexane gave the title compound (0.13g), mp 96-8°

Example 23

5-(7-Carboxyheptyl) 3-methyl 4-(3-chloro-6-fluoro-2-(trifluoromethyl)phenyl)-2-(fluoromethyl)-1,4-dihydro-6-methyl-3,5-pyridinecarboxylate

Sodium hydroxide solution (5ml of 1N) was added to stirred solution of 5-(8-ethoxy-8-oxooctyl) 3-methyl 4-(3-chloro-6-fluoro-2-(trifluoromethyl)phenyl)-2-(fluoromethyl)-1,4-dihydro-6-methyl-3,5-pyridinedicarboxylate (1g, 1.67mmoles) in 1,2-dimethoxy ethane (12ml). After 2 days, the solution was poured onto ice, acidified with 10% aqueous sulphuric acid and the product extracted with ether (x3). The combined organic extracts were washed with brine, dried ($MgSO_4$) and the solvent evaporated. Chromatography on silica eluting with ethyl acetate/petroleum ether (60-80°) followed by crystallisation from ethyl acetate/petroleum ether (60-80°) gave the title compound (0.26g), mp 115.5-7°.

Example 24

5-(8-Methoxy-8-oxooctyl) 3-methyl 4-(3-chloro-6-fluoro--2-(trifluoromethyl)phenyl)-2-(fluoromethyl)-1,4-dihydro-6-methyl-3,5-pyridinedicarboxylate

The product of Example 23 (0.9g, 1.6mmoles) and methanol (30ml) containing conc. sulphuric acid (3 drops) was heated at reflux for 30 minutes. The solvent was evaporated and the residue dissolved in ethyl acetate. This organic solution was washed with brine (x3), dried ($MgSO_4$) and the solvent evaporated. Crystallisation from petroleum ether (60-80°) gave the title compound (0.42g), mp 98-100°.

Example 25

3-Methyl 5-(2-oxo-2-((3-(5-phenylpyrazol-3-yloxy) propyl)amino)ethyl) 4-(3-chloro-6-fluoro-2-(trifluoromethyl)phenyl)-2-(fluoromethyl)-1,4-dihydro-6-methyl-3,5-pyridinedicarboxylate

5-(Carboxymethyl) 3-methyl 4-(3-chloro-6-fluoro-2-(trifluoromethyl)phenyl)-2-(fluoromethyl)-1,4-dihydro-6-methyl-3.5-pyridinecarboxylate (1g, 2mmoles), carbonyldiimidazole (0.37g, 2mmoles) and dichloromethane (50ml) were stirred for 1 hour at room temperature. 3-(5-Phenylpyrazol-3-yloxy propylamine (0.45g, 2mmoles) was added and the stirring continued for 3 hours. The reaction mixture was poured onto dilute hydrochloric acid and the organic extract was washed with water and saturated sodium bicarbonate solution, and then dried ($MgSO_4$). The solvent was evaporated and the residue chromatographed on silica eluting with ethyl acetate/petroleum ether (60-80 ) mixtures. Crystallisation from cyclohexane gave the title compound (0.22g), mp 143-7 .

Example 26

3-Ethyl 5-methyl 2-(2-(2-aminoethoxy)ethoxymethyl)-4-(3-chloro-6-fluoro-2-(trifluoromethyl)phenyl)-6-(fluoromethyl)-1,4-dihydro-3,5-pyridinedicarboxylate oxalate salt

3-Ethyl 5-methyl 4-(3-chloro-6-fluoro-2-(trifluoromethyl)phenyl)-2-((2-(2-(1,3-dihydro-1,3-dioxo-2H-isoindol-2-yl)ethoxy)ethoxy)methyl)-6-(fluoromethyl)-1,4-dihydro-3,5-pyridinedicarboxylate (1g, 1.46mmoles) and hydrazine hydrate (0.5ml, 20mmoles) in ethanol (50ml) were heated at reflux for 1 hour. The solvent was evaporated and ethyl acetate added to the residue. The resulting solid was filtered off and the ethyl acetate filtrate was washed with brine and dried ($MgSO_4$). The solvent was evaporated and the residue was dissolved in ethanol. Addition of oxalic acid gave the oxalate salt which was recrystallised from ethanol to give the title compound (0.2g), mp 136-8°.

Example 27

3-Ethyl 5-methyl 2-(2-aminoethoxymethyl)-4-(2-chlorophenyl)-6-(fluoromethyl)-1,4-dihydro-3,5-pyridinedicarboxylate maleate salt

3-Ethyl 5-methyl 4-(2-chlorophenyl)-2-((2-(1,3-dihydro-1,3-dioxo-2H-isoindol-2-yl)ethoxy)methyl)-6- (fluoromethyl)-1,4-dihydro-3,5-pyridinedicarboxylate (14g, 25mmoles) and hydrazine hydrate (6ml, 120mmoles) in ethanol (100ml) was heated at reflux for 1 hour. The solvent was evaporated and ethyl acetate was added to the residue. The resulting solid was filtered off and the ethyl acetate filtrate was washed with water and dried ($MgSO_4$). The solvent was evaporated, and the residue was dissolved in ethanol. Maleic acid (2.65h) in ethanol was added and then the title compound (3.1g) was filtered off, mp 139-41°.

Example 28

3-Ethyl 5-methyl 2-((2-aminoethoxy)methyl)-4-(3-chloro-2-(trifluoromethyl)phenyl)-6-(fluoromethyl)-1,4-dihydro-3,5-pyridinedicarboxylate maleate salt

3-Ethyl 5-methyl 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate (3.7g, 5.9mmoles) and hydrazine hydrate (1ml, 20mmoles) in ethanol (60ml) was heated at 50° for 2 hours. The cooled reaction mixture was filtered and the solvent evaporated from the filtrate. The residue was purified by chromatography on silica eluting with ethyl acetate/methanol mixtures to give the title compound free base (0.84g). The maleate salt was prepared in ethanol (5ml) with maleic acid (0.2g) and crystallised by addition of ether to give the title compound (0.6g), mp 155-6°.

Example 29

3-Methyl 5-(1-methylethyl) 2-(fluoromethyl)-4-(2-fluoro-6-nitrophenyl)-1,4-dihydro-6-methyl-3,5-pyridinedicarboxylate

2-Fluoro-6-nitrobenzaldehyde (1.26g, 7.5mmoles), methyl 4-fluoro-3-oxobutanoate (1.0g, 7.5mmoles) and (1-methylethyl) 3-amino-2-butenoate (1.06g, 7.5mmoles) were heated at 90° for 2.5 hours in the dark. The cooled residue was chromatographed on silica eluting with petroleum ether (60°-80°)/ethyl acetate mixtures. The title compound (0.25g) was obtained after crystallisation from petroleum ether (60°-80°)/2-propanol, mp 112-3°.

Example 30

3-Methyl 6-(1-methylethyl) 4-(2-chloro-6-methylphenyl)-2-(fluoromethyl)-1,4-dihydro-6-methyl-3,5-pyridinedicarboxylate

2-Chloro-6-methylbenzaldehyde (1.66g, 10.8mmoles), methyl 4-fluoro-3-oxobutanoate (1.45g, 10.8mmoles) and 1-methylethyl 3-amino-2-butenoate (3.55g, 10.8mmoles) were dissolved in t-butanol (50ml). This solution was heated at 45° for 4 days and 55° for 11 days. The solvent was evaporated and the residue chromatographed on silica eluting with ethyl acetate/petroleum ether (60-80°) mixtures. Crystallisation from petroleum ether (60-80°) gave the title compound (0.28g), mp 105-6°.

Example 31

3-Cyclobutyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate maleate (1:1) hydrate (3:2)

Hydrazine hydrate (0.5ml, 10mmoles) was added to a solution of 3-cyclobutyl 5-methyl 4-(3-chloro-6-fluoro-2-(trifluoromethyl)phenyl)-2-((2-((2-(cyclobutyloxycarbonyl)benzoyl)amino)ethoxy)methyl)-6-(fluoromethyl)-1.4-dihydro-3,5-pyridinedicarboxylate (2.4g, 3.2mmoles) in ethanol (35ml) and the mixture was heated at reflux for 2 hours. After cooling, the solid was filtered off and the ethanol evaporated. The ethyl acetate soluble material was chromatographed on silica eluting with ethyl acetate/methanol mixtures to give the title compound freebase (0.47g). The maleate salt (0.35g) was prepared and crystallised from ethanol/ether, mp 144-6° (dec).

Example 32

5-Methyl 3-(3-thietanyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate maleate (1:1) hydrate (1:2)

3-Methyl 5-(3-thietanyl) 4-(3-chloro-6-fluoro-2-(trifluoromethyl)phenyl)-2-(fluoromethyl)-1,4-dihydro--6-((2-((2-(3-thietanyloxycarbonyl)benzoyl)amino)ethoxy) methyl)-3,5-pyridinedicarboxylate(4.1g of crude product) and hydrazine hydrate (1ml) in ethanol (20ml) were stirred at room temperature for 30 minutes. The solvent was evaporated and the residue treated with ethyl acetate/ether. The solution was filtered and the solvent was evaporated. Chromatography twice on silica eluting with ethyl acetate/methanol and then ethyl acetate/methanol/triethylamine mixtures gave the title compound freebase (0.13g). The maleate salt was prepared and crystallised from ethanol/ether to give the title compound (0.085g), mp 140-2°.

Example 33

5-Methyl 3-(1-methylethyl) 2-((2-aminoethoxy)methyl)-6-(fluoromethyl)-4-(pentafluorophenyl)-1,4-dihydro-3,5-pyridinedicarboxylate

1-Methylethyl 4-(2-(1,3-dihydro-1,3-dioxo-2H-isoindol-2-yl)ethoxy)-3-oxobutanoate (4.25g, 12.75mmoles) and ammonium acetate (1.1g, 14.3mmoles) in dry t-butanol (60ml) were heated at 60° for 1.5 hours. Pentafluorobenzaldehyde (2.5g, 12.75mmoles) and methyl 4-fluoro-3-oxobutanoate (1.7g, 12.75mmoles) were added, and heating continued for 1 hour, then formic acid (10ml) was added. After a further 15 minutes the solvent was evaporated and the residue added to ether and water. The organic solution was evaporated and the residue was purified by chromatography (twice) on silica eluting first with ethyl acetate dichloromethane mixtures and then with ethyl acetate/petroleum ether mixtures. The oil obtained was treated with hydrazine hydrate (6ml) in ethanol (50ml) at reflux for 1 hour. The suspension was poured into ether and water and the organic solution evaporated. The residue was purified by chromatography on silica using dichloromethane/methanol mixture to give the title compound (1.1g) as a very pale yellow solid, mp 130-1° (ether-petroleum ether).

Example 34

3-Ethyl 5-methyl 2-((2-aminoethoxy)methyl)-6-(fluoromethyl)-4-(2,3,5,6-tetrafluorophenyl)-1,4-dihydro-3,5-pyridinedicarboxylate

Prepared by the method described in Example 33 except that reaction was allowed to proceed for 7 hours after addition of 2,3,5,6-tetrafluorobenzaldehyde and methyl 4-fluoro-3-oxobutanoate. The product was obtained as a yellow solid, mp 154-6°.

Example 35

3-Ethyl 5-methyl 2-((2-aminoethoxy)methyl)-4-(2,3-dichlorophenyl)-6-(fluoromethyl)-1,4-dihydro-3,5-pyridinedicarboxylate ethanedioate

Titanium tetrachloride (1.36g, 7.16mmoles) was added dropwise to an ice-cooled suspension of sodium borohydride (545mg, 14.32mmoles) in dry dimethoxyethane (40ml) under nitrogen, and 5-ethyl 3-methyl 4-(2,3-dichlorophenyl)-2-(fluoromethyl)-1,4-dihydro-6-((2-hydroxyimino) ethoxy) methyl-3,5-pyridinedicarboxylate (1.7g, 3.58mmoles) was added portionwise. The reaction mixture was allowed to warm to room temperature, stirred for 30 minutes, then poured into water and basified with aqueous ammonia solution. The suspension was extracted with ethyl acetate and the extracts separated, dried and evaporated in vacuo. The residue was purified by chromatography on silica using dichloromethane-ethanol mixtures as eluant and the produce obtained converted to the oxalate salt using oxalic acid in ethanol. The title compound (0.7g) was obtained as pale yellow crystals, mp 154-5°.

Example 36

3-Methyl 5-(1-methylethyl) 2-(fluoromethyl)-1,4-dihydro-6-methyl-4-(2,3,5,6-tetrafluorophenyl)-3,5-pyridinedicarboxylate

2,3,5,6-Tetrafluorobenzaldehyde (10.0g, 56.2mmoles), 1-methylethyl 3-aminobutenoate (8.0g, 56.2mmoles) and methyl 4-fluoro-3-oxobutanoate (7.5g, 56.2mmoles) in dry t-butanol (170ml) were heated to 60° for 17 hours. Formic acid (1ml) was added and the solvent removed by distillation under vacuum. The residue

crystallised from petroleum ether gave the title compound (11.0g), mp 157-9°.

Example 37

3-Methyl 5-(1-methylethyl) 4-(2-fluoro-5-methoxyphenyl)-2-(fluoromethyl)-1,4-dihydro-6-methyl-3,5-pyridinedicarboxylate

2-Fluoro-5-methoxybenzaldehyde (1g), methyl 4-fluoro-3-oxobutanoate (0.87g) and 1-methylethyl 3-amino-2-butenoate (0.93g) in dry t-butanol (3ml) were heated at 60° for 24 hours. The solvent was removed by distillation in vacuum and the residue treated with trifluoroacetic anhydride (0.60ml) in dichloromethane for 1.5 hours. The solvent was evaporated and the residue purified by chromatography on silica using ethyl acetate/petroleum ether as eluant to afford the title compound (0.98g), mp 72-4° (dichloromethane-petroleum ether).

Example 38

5-(6-(2,6-Dimethylpyrimidin-4-yloxy)hexyl) 3-methyl 4-(3-chloro-6-fluoro-2-(trifluoromethyl)phenyl)-2-(fluoromethyl)-1,4-dihydro-6-methyl-3,5-pyridinedicarboxylate

Methyl 4-fluoro-3-oxobutanoate (0.8g), 3-chloro-6-fluoro-2-(trifluoromethyl)benzaldehyde (1.8g), and 6-(2,6-dimethylpyrimidin-4-yloxy)hexyl 3-amino-2-butenoate (1.8g) in dry t-butanol (40ml) were heated at 50° for 6 days. The solvent was removed by distillation and the residue purified by chromatography on silica using successively ethyl acetate-petroleum ether mixtures, ethyl acetate-dichloromethane mixtures, and ether to afford the title compound (0.45g) as an amorphous foam. M+ +1 632/4; NMR ($CDCl_3$) delta 5.95 (q,1H), 6.35 (s,1H).

Example 39

5-Methyl 3-(2,2,2-trichloroethyl) 2-((2-aminoethoxy) methyl)-4-(3-chloro-6-fluoro-2-(trifluoromethyl)phenyl)-6-(fluoromethyl)-1,4-dihydro-3,5-pyridinedicarboxylate Z-butenedioate salt

The product of Example CO (0.6g) and hydrazine hydrate (0.25ml) in ethanol (20ml) were heated at 55° for 3 hours. The suspension was treated with ethyl acetate/methanol (3:1) (20ml), filtered and the filtrate separated by chromatography on silica using ethyl acetate/methanol mixtures as eluant. The free base obtained was converted to the maleate using maleic acid in ethanol giving a pale yellow solid (0.3g), mp 120-2° (ethanol-hexane).

Example 40

3-Ethyl 5-methyl 2-((2-((5-amino-1H-1,2,4-triazol-3-yl)amino)ethoxy)methyl)-6-(fluoromethyl)-1,4-dihydro-4-(2,3,5,6-tetrafluorophenyl)-3,5-pyridinedicarboxylate hemi ethanedioate salt

The product of Example 34 (1g, 2.15mmoles) and S,Sμ-Dimethyl-N-cyanodithioiminocarbonate (315mg, 2.15mmoles) in ether (50ml) were stirred at 20° for 4 days. The solid was filtered off and dissolved in methanol (25ml) containing hydrazine hydrate (0.31ml). The mixture was heated at reflux overnight, then the solvent was removed by distillation and the residue dissolved in ethyl acetate and water. The organic phase was separated, dried and evaporated and the residue was purified by chromatography on silica using dichloromethane-ethanol mixtures as eluant. The product was treated with oxalic acid in ethanol to give the title salt (0.6g), mp 172-4° (ethanol).

Example 41

The following compounds were prepared by the method of Example 2 using appropriate starting materials:

a) 3-Methyl 5-(1-methylethyl) 4-(3-chloro-6-fluoro-2-(trifluoromethyl)phenyl)-2-(fluoromethyl)-1,4-dihydro-6-((1H-imidazol-1-yl)methyl)-3,5-pyridinedicarboxylate oxalate, mp 147-9°.

b) 5-Methyl 3-(1-methylethyl) 2-((6-amino-1,2-dihydro-2-oxopyrid-1-yl)methyl)-4-(3-chloro-6-fluoro--2-(trifluoromethyl)phenyl)-6-(fluoromethyl)-1,4-dihydro-3,5- pyridinedicarboxylate, mp 240-2° (dec).

c) 5-Methyl 3-(1-methylethyl) 2-((2-aminopyrid-3-yloxy) methyl)-4-(3-chloro-6-fluoro-2-(trifluoromethyl)phenyl)-6-(fluoromethyl)-1,4-dihydro-3,5-pyridinedicarboxylate, mp 155-7°.

d) 5-Methyl 3-(1-methylethyl) 2-(((1H-5-amino-1,2,4-triazol-3-yl)thio)methyl)-4-(3-chloro-6-fluoro-2-(trifluoromethyl)phenyl)-6-(fluoromethyl)-1,4-dihydro-3,5-pyridinedicarboxylate ethyl acetate solvate (1:1), mp 115-7°.

e) 5-Methyl 3-(1-methylethyl) 2-((5-amino-1H-1,2,4-triazol-1-yl)methyl)-4-(3-chloro-6-fluoro-2-(trifluoromethyl)phenyl)-6-(fluoromethyl)-1,4-dihydro-3,5-pyridinedicarboxylate, mp 173-5°.

f) 5-methyl 3-(1-methylethyl) 2-((3-amino-1H-1,2,4-triazol-1-yl)methyl)-4-(3-chloro-6-fluoro-2-(trifluoromethyl)phenyl)-6-(fluoromethyl)-1,4-dihydro-3,5-pyridinedicarboxylate, mp 106-8°.

g) 5-methyl 3-(1-methylethyl) 2-((3-amino-4H-1,2,4-triazol-4-yl)methyl)-4-(3-chloro-6-fluoro-2-(trifluoromethyl)phenyl)-6-(fluoromethyl)-1,4-dihydro-3,5-pyridinedicarboxylate, mp 128°.

Example 42

The following compounds were prepared by the method of Example 3 using the appropriate starting materials:

a) 5-Methyl 3-(1-methylethyl) 4-((3-chloro-6-fluoro-2-(trifluoromethyl)phenyl)-6-(fluoromethyl)-1,4-dihydro-2-((1-pyridinium)methyl)-3,5-pyridinedicarboxylate bromide, mp 208° (dec).

b) 5-Methyl 3-(1-methylethyl) 2-((aminoiminomethyl)thiomethyl)-4-(3-chloro-6-fluoro-2-(trifluoromethyl)phenyl)-6-fluoromethyl-1,4-dihydro-3,5-pyridinedicarboxylate hydrochloride. M/e 542/4; M+1+; NMR delta ($D_6$-DMSO) 5.87 (q,1H).

Example 43

The following compound was prepared by the method of Example CU using appropriate starting materials: 5-Methyl 3-(1-methylethyl) 2-((2-aminoethylsulphonyl) methyl)-4-(3-chloro-6-fluoro-2-(trifluoromethyl)phenyl)-6-(fluoromethyl)-1.4-dihydro-3,5-pyridinedicarboxylate oxalate, mp 185° (dec).

Example 44

The following compounds were prepared by the method of Example 4 using appropriate starting materials:

a) 3-Ethyl 5-methyl 2-((2-aminoethylthio)methyl)-4-(3-chloro-6-fluoro-2-(trifluoromethyl)phenyl)-6-(fluoromethyl)-1,4-dihydro-3,5-pyridinedicarboxylate (Z)-2-butenedioate, mp 158-9° (dec).

b) 3-Ethyl 5-methyl 2-((2-aminoethylthio)methyl)-4-(2-chlorophenyl)-6-(fluoromethyl)-1,4-dihydro-3,5-pyridinedicarboxylate (Z)-2-butendioate, mp 123-4°.

c) 5-Methyl 3-(methylethyl) 2-((aminooxy)methyl)-4-(3-chloro-6-fluoro-2-(trifluoromethyl)phenyl)-6-(fluoromethyl)-1,4-dihydro-3,5-pyridinedicarboxylate, mp 125-7°.

d) 5-Methyl 3-(1-methylethyl) 2-((4-(2-aminoethyl)-1H-imidazol-1-yl)methyl)-4-(3-chloro-6-fluoro-2-(trifluoromethyl)phenyl)-6-(fluoromethyl)-1,4-dihydro-3,5-pyridinedicarboxylate bis-(Z)-2-butenedioate, mp 105-8°.

Example 45

The following compounds were prepared by the method of Example 6 using appropriate starting materials:

a) 3-Methyl 5-(1-methylethyl) 4-(3-chloro-6-fluoro-2-(trifluoromethyl)phenyl)-2-(fluoromethyl)-1,4-dihydro-6-((2-(1H-imidazol-4-yl)ethylamino)methyl)-3,5-pyridinedicarboxylate bisoxalate salt, mp 174-6°.

b) 3-Methyl 5-(1-methylethyl) 4-(3-chloro-6-fluoro-2-(trifluoromethyl)phenyl)-2-(fluoromethyl)-1,4-dihydro-6-((1H-1,2,4-triazol-3-ylthio)methyl)-3,5-pyridinedicarboxylate, mp 171-2°.

c) 5-Methyl 3-(1-methylethyl) 4-(3-chloro-6-fluoro-2-(trifluoromethyl)phenyl)-2-((4,6-diaminopyrimidin-2-ylthio) pyridinedicarboxylate, mp 158-9°.

Example 46

The following compounds were prepared by the method of Example 14 using appropriate starting materials:

a) 3-Ethyl 5-methyl 4-(2-chlorophenyl)-2-((5-ethoxycarbonyl-3,4-dihydro-4-oxopyrimidin-2-ylthio)methyl)-6-(fluoromethyl)-1,4-dihydro-3,5-pyridinedicarboxylate, mp 178-9°.

b) 3-Ethyl 5-methyl 4-(2-chlorophenyl)-2-((3,4-dihydro-4-oxopyrimidin-2-ylthio)methyl)-6-(fluoromethyl)-1,4-dihydro-3,5-pyridinedicarboxylate, mp 186-9° (dec).

c) 5-Ethyl 3-methyl 4-(2-chlorophenyl)-2-(fluoromethyl)-1,4-dihydro-6-((pyrimidin-2-ylthio)methyl)-3,5-pyridinedicarboxylate, mp 134-136°.

d) 5-Ethyl 3-methyl 4-(2-chlorophenyl)-2-(fluoromethyl)-1,4-dihydro-6-((pyridin-2-ylthio)methyl)-3,5-pyridinedicarboxylate, mp 136-138°.

e) 3-Ethyl 5-methyl 2-((1H-benzimidazol-2-ylthio)methyl)-4-(2-chlorophenyl)-6-(fluoromethyl)-1,4-dihydro-3,5-pyridinedicarboxylate, mp 115°.

f) 5-Ethyl 3-methyl 4-(2-chlorophenyl)-2-(fluoromethyl)-1,4-dihydro-6-((1H-imidazol--2-ylthio)methyl)-3,5-pyridinedicarboxylate, mp 115° (dec).

g) 3-Ethyl 5-methyl 4-(2,3-dichlorophenyl)-2-((3,4-dihydro-4-oxopyrimidin-2-ylthio)methyl)-6-(fluoromethyl)-1,4-dihydro-3,5-pyridinedicarboxylate, mp 192-4°.

h) 3-Ethyl 5-methyl 4-(2,3-dichlorophenyl)-2-((5-(ethoxycarbonyl)-3,4-dihydro-4-oxopyrimidin-2-ylthio)methyl)-6- (fluoromethyl)-1,4-dihydro-3,5-pyridinedicarboxylate, mp 179-181°.

i) 3-Methyl 5-(1-methylethyl) 4-(3-chloro-6-fluoro-2-(trifluoromethyl)phenyl)-2-(fluoromethyl)-1,4-dihydro-6-((5-methoxy-1H-benzimidazol-2-ylthio)methyl)-3,5-pyridinedicarboxylate, mp 188-190° (dec).

j) 3-Ethyl 5-methyl 2-((6-carboxy-3,4-dihydro-4-oxopyrimidin-2-ylthio)methyl)-4-(2-chlorophenyl)-6-(fluoromethyl)-1,4-dihydro-3,5-pyridinedicarboxylate, mp 158° (dec).

k) 3-Ethyl 5-methyl 4-(2,3-dichlorophenyl)-2-((3,4-dihydro-6-hydroxy-4-oxopyrimidin--2-ylthio)methyl)-6-(fluoromethyl)-1,4-dihydro-3,5-pyridinedicarboxylate, mp 224-226° (dec).

l) 3-Ethyl 5-methyl 4-(2,3-dichlorophenyl)-2-((3,4-dihydro-6-methyl-4-oxopyrimidin-2-ylthio)methyl)-6-(fluoromethyl)-1,4-dihydropyridine-3,5-dicarboxylate, mp 146-50°.

m) 3-Ethyl 5-methyl 2-((5-(ethoxycarbonyl)-3,4-dihydro-4-oxopyrimidin-2-ylthio)methyl)-6-(fluoromethyl)-1,4-dihydro-4-(2-trifluoromethyl)phenyl)-3.5-pyridinedicarboxylate, mp 153-155° (dec).

n) 3-Ethyl 5-methyl 2-((3,4-dihydro-4-oxopyrimidin-2-yl-thio)methyl)-6-(fluoromethyl)-1,4-dihydro-4-(2-trifluoromethyl)phenyl)-3,5-pyridinedicarboxylate, mp 187-189° (dec).

o) 3-Ethyl 5-methyl 2-((4-amino-5-(ethoxycarbonyl)-pyrimidin-2-ylthio)methyl)-4-(2-chlorophenyl)-6-(fluoromethyl)-1,4-dihydro-3,5-pyridinedicarboxylate, mp 209-210° (dec).

p) 5-Ethyl-3-methyl-4-(2,3-dichlorophenyl)-2-(fluoromethyl)-1,4-dihydro-6-(pyrid-2-ylthiomethyl)-3,5-pyridinedicarboxylate, mp 119-21 .

Example 47

The following compounds were prepared by the method of Example 16 using appropriate starting materials:

a) 3-Ethyl 5-methyl 4-(2,3-dichlorophenyl)-2-((((cyanoimino)methylamino)methylthio)methyl)-6-(fluoromethyl)-1,4-dihydro-3,5-pyridinedicarboxylate, mp 191-2° (dec).

b) 3-Ethyl 5-methyl 2-(((acetylimino)aminomethylthio) methyl)-4-(2,3-dichlorophenyl)-6-(fluoromethyl)-1,4-dihydro-3,5-pyridinedicarboxylate, mp 177-180° (dec).

Example 48

following compound was prepared by the method of Example 17 using appropriate starting materials:

5-Methyl 3-(1-methylethyl) 2-((2-aminocarbonyloxy) methyl)-4-(2,3-dichlorophenyl)-6-(fluoromethyl)-1,4-dihydro-3,5-pyridinedicarboxylate, mp 173-5°.

Example 49

The following compound was prepared by the method of Example 11 using appropriate starting materials:

5-Ethyl 3-methyl 4-(2-chlorophenyl)-2-(fluoromethyl)-1,4-dihydro-6-((1H-pyrrol-1-yl)methyl)-3,5-pyridinedicarboxylate, mp 142-4°.

Example 50

The following compound was prepared by the method of Example 17 using appropriate starting materials:

3-Methyl 5-(1-methylethyl) 4-(3-chloro-6-fluoro-2-(trifluoromethyl)phenyl)-2-(fluoromethyl)-1,4-dihydro--6-((4H-1,2,4-triazol-4-yl)methyl)-3,5-pyridinedicarboxylate, mp 175-7° (dec).

Example 51

The following compound was prepared by the method of Example 19 using appropriate starting materials:

3-Methyl 5-(6-(5-phenylpyrazol-3-ylamino)hexyl) 4-(3-chloro-6-fluoro-2-(trifluoromethyl)phenyl)-2-(fluoromethyl)-1,4-dihydro-6-methyl-3,5-pyridinedicarboxylate, mp 90° (dec).

Example 52

The following compounds were prepared by the method of Example 20 using appropriate starting materials:

a) 3-Methyl 5-(6-(pyrid-3-yloxy)hexyl) 4-3-chloro-6-fluoro-2-(trifluoromethyl)phenyl)-2-(fluoromethyl)-1,4-dihydro-6-methyl-3,5-pyridinedicarboxylate, mp 135-7°.

b) 5-(6-(1H-Imidazol-1-yl)hexyl) 3-methyl 4-(3-chloro-6- fluoro-2-(trifluoromethyl)phenyl)-2-(fluoromethyl)-1,4-dihydro-6-methyl-3,5-pyridinedicarboxylate, mp 177-9° (dec).

c) 5-(6-(4,6-Dimethylpyrimidin-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate, mp 83-6°.

d) 5-(6-(1H-Benzimidazol-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate, mp 110-2°.

e) 5-((8-Ethoxy-8-oxo)octyl) 3-methyl 4-(3-chloro-6-fluoro-2-(trifluoromethyl)phenyl)-2-(fluoromethyl)-1,4-dihydro-6-methyl-3,5-pyridinedicarboxylate

Example 53

The following compound was prepared by the method of Example 25 using appropriate starting materials:

5-((8-Amino-8-oxo)octyl) 3-methyl 4-(3-chloro-6-fluoro -2-(trifluoromethyl)phenyl)-2-(fluoromethyl)-1,4-dihydro-6-methyl-3,5-pyridinedicarboxylate, mp 164-6°.

Example 54

The following compounds were prepared by the method of Example 26 using appropriate starting materials:

a) 3-Ethyl 5-methyl 2-((2-aminoethoxy)methyl)-6-(fluoromethyl)-1,4-dihydro-4-(5-nitrothien-2-yl)-3,5-pyridinedicarboxylate ethanedioate salt, mp 166-8°.

b) 3-Ethyl 5-methyl 2-((2-aminoethoxy)methyl)-4-(5-cyanothien-2-yl)-6-(fluoromethyl)-1,4-dihydro-3,5-pyridinedicarboxylate ethanedioate salt, mp 97-9°.

c) 3-Ethyl 5-methyl 2-((2-aminoethoxy)methyl)-4-(3-cyanothien-2-yl)-6-(fluoromethyl)-1,4-dihydro-3,5-pyridinedicarboxylate ethanedioate salt, mp 191-3° (dec).

d) 3-Ethyl 5-methyl 2-((2-aminoethoxy(methyl)-6-(fluoromethyl)-4-(3-methylthien-2-yl)-1,4-dihydro-3,5-pyridinedicarboxylate ethanedioate salt, mp 182-3°.

e) 3-Ethyl 5-methyl 2-((2-(2-aminoethoxy) ethoxy) methyl)-4-(2-chlorophenyl)-6-(fluoromethyl)-1,4-dihydro-3,5-pyridinedicarboxylate, mp 88-90° (ether).

f) Diethyl 2-((2-aminoethoxy)methoxy)-4-(2-chlorophenyl)-1,4-dihydro-6-(trifluoromethyl)-3,5-pyridinedicarboxylate ethanedioate salt, mp 165-7°.

g) 3-Ethyl 5-methyl 4-(3-amino-2,6-difluorophenyl)-2-((2-aminoethoxy)methyl)-6-(fluoromethyl)-1,4-dihydro-3,5-pyridinedicarboxylate, mp 124-6°.

h) 5-Methyl 3-(6-((6-methyl-2-phenylpyrimidin-4-yl)oxy) hexyl) 2-((2-aminoethoxy)methyl)-4-(2-chlorophenyl)-6-(fluoromethyl)-1,4-dihydro-3,5-pyridinedicarboxylate ethanedioate salt, mp 141-3°.

i) 5-Methyl 3-(6-((6-methyl-2-phenylpyrimidin-4-yl)thio) hexyl 2-((2-aminoethoxy)methyl)-4-(2-chlorophenyl)-6- (fluoromethyl)-1,4-dihydro-3,5- pyridinedicarboxylate ethanedioate salt, mp 147° (dec).

j) 3-(6-((4,6-Dimethylpyrimidin-2-yl)thio)hexyl) 5-methyl 2-((2-aminoethoxy)methyl)-4-(2-chlorophenyl)-6-(fluoromethyl)1,4-dihydro-3,5-pyridinedicarboxylate ethanedioate salt, mp 155° (dec).

k) 3-Ethyl 5-methyl 2-((2-aminoethoxy)methyl)-4-(2-chloro-3,5-dimethoxyphenyl)-6-(fluoromethyl)-1,4-dihydro-3,5-pyridinedicarboxylate, mp 108-9°.

Example 55

The following compounds were prepared by the method of Example 27 using appropriate starting materials:

a) 3-Ethyl 5-methyl 2-((2-aminoethoxy)methyl)-4-(2-chloro-3-(trifluoromethyl)phenyl)-6-(fluoromethyl)-1,4-dihydro-3,5-pyridinedicarboxylate maleate salt, mp 142-4°.

b) 3-Ethyl 5-methyl 2-((2-aminoethoxy)methyl)-4-(3-cyanophenyl)-6-(fluoromethyl)-1,4-dihydro-3,5-pyridinedicarboxylate oxalate salt, mp 162-5° (dec).

c) 3-Ethyl 5-methyl 2-((2-aminoethoxy)methyl)-4-(2,5-difluoro-6-(trifluoromethyl)phenyl)-6-(fluoromethyl)-1,4-dihydro-3,5-pyridinedicarboxylate Z-butenedioate salt, mp 140-2°.

d) 3-Ethyl 5-methyl 2-((2-aminoethoxy)methyl)-4-(2-fluoro-5-methoxyphenyl)-6-(fluoromethyl)-1,4-dihydro- 3,5-pyridinedicarboxylate (Z) butenedioate salt, mp 159-61° (dec).

e) 5-Methyl 3-((6-phenoxy)hexyl) 2-((2-aminoethoxy) methyl)-4-(2-chlorophenyl)-6-(fluoromethyl)-1,4-dihydro-3,5-pyridinedicarboxylate Z-butendioate salt, mp 116-8°.

f) 3-Ethyl 5-methyl 2-((2-aminoethoxy)methyl)-4-(6-chloro-2-fluoro-3-methoxyphenyl)-6-(fluoromethyl)-1,4-dihydro-3,5-pyridinedicarboxylate Z-butenedioate salt, mp 158-9°.

g) 5-Methyl 3-(2,2,2-trichloroethyl) 2-((2-aminoethoxy)methyl)-4-(2-chlorophenyl)-6-(fluoromethyl)-1,4-dihydro-3,5-pyridinedicarboxylate Z-butenedioate salt, mp 144-6° (dec).

h) 5-Methyl 3-(2,2,2-trifluoroethyl) 2-((2-aminoethoxy) methyl)-4-(2-chlorophenyl)-6-(fluoromethyl)-1,4-dihydro-3,5-pyridinedicarboxylate Z-butenedioate salt, mp 157-8°.

Example 56

The following compounds were prepared by the method of Example 29 using appropriate starting materials:

a) 3-Methyl 5-(1-methylethyl) 4-(3-chloro-2-fluorophenyl)-2-(fluoromethyl)-1,4-dihydro-6-methyl3,5-pyridinedicarboxylate, mp 135-6.5°.

b) 3-Methyl 5-(1-methylethyl) 4-(2,6-difluorophenyl)-2-(fluoromethyl)-1,4-dihydro-6-methyl-3,5-pyridinedicarboxylate, mp 164-5°.

c) 3-Methyl 5-(1-methylethyl) 2-(fluoromethyl)-1,4-dihydro-6-methyl-4-(3-nitro-2-(trifluoromethyl)phenyl)-3,5-pyridinedicarboxylate, mp 169-70°.

d) 3-Methyl 5-(1-methylethyl) 4-(2-fluoro-3-(trifluoromethyl)phenyl)-2-(fluoromethyl)-1,4-dihydro-6-methyl-3,5-pyridinedicarboxylate, mp 119-20°.

e) 3-Methyl 5-(1-methylethyl) 4-(3-chloro-2-(trifluoromethyl)phenyl)-2-(fluoromethyl)-1,4-dihydro-6-methyl-3,5-pyridinedicarboxylate, mp 133-4°.

f) 3-Methyl 5-(1-methylethyl) 4-(3-chloro-2-nitrophenyl)-2-(fluoromethyl)-1,4-dihydro-6-methyl-3,5-pyridinedicarboxylate, mp 165-6°.

g) 3-Methyl 5-(1-methylethyl) 4-(2,3-difluorophenyl)-2-(fluoromethyl)-1,4-dihydro-6-methyl-3,5-pyridinedicarboxylate, mp 53-4°.

h) 3-Methyl 5-(1-methylethyl) 2-(fluoromethyl)-1,4-dihydro-4-(5-methoxy-2-nitrophenyl)-6-methyl-3,5-pyridinedicarboxylate, mp 119.5-121°.

Example 57

The following compounds were prepared by the method of Example 30 using appropriate starting materials:

a) 3-Methyl 5-(1-methylethyl) 2-fluoromethyl-4-(2-fluoro-6-(pentafluoroethyl)-phenyl)-1,4-dihydro-6-methyl-3,5-pyridinedicarboxylate, mp 109-11°.

b) 3-Methyl 5-(1-methylethyl) 2-(fluoromethyl)-4-(4- fluoro-2-(trifluoromethyl)phenyl)-1,4-dihydro-6-methyl-3,5-pyridinedicarboxylate, mp 92-3°.

c) 3-Methyl 5-(1-methylethyl) 2-(fluoromethyl)-4-(8-fluoronaphthalen-1-yl)-1,4-dihydro-6-methyl-3,5-pyridinedicarboxylate, mp 158-60°.

Example 58

The following compounds were prepared by the method of Example 35 using appropriate starting materials:

a) 5-Methyl 3-(2,2,2-trifluoroethyl) 2-((2-aminoethoxy) methyl)-6-(fluoromethyl)-1,4-dihydro-4-(2-(trifluoromethyl)phenyl)-3,5-pyridinedicarbxylate Z-butenedioate salt, mp 165-7 .

b) 5-Methyl 3-(1-methylethyl) 2-((2-aminoethoxy)methyl)-6-(fluoromethyl)-1,4-dihydro-4-(2,3,5,6-tetrafluorophenyl)-3,5-pyridinedicarboxylate, mp 175-7 .

c) 5-Methyl 3-(1-methylethyl) 2-((2-aminoethoxy)methyl)-6-(fluoromethyl)-1,4-dihydro-4-(2,4,6-trifluorophenyl)-3,5-pyridinedicarboxylate Z-butenedioate salt, mp 146-8° (dec).

Example 59

The following compounds were prepared by the method of Example 36 using appropriate starting materials:

a) 3-Methyl 5-(2,2,2-trifluoroethyl) 2-(fluoromethyl)-1,4-dihydro-6-methyl-4-(2-(trifluoromethyl)phenyl)-3,5-pyridinedicarboxylate, mp 72°.

b) 3-Methyl 5-(1-methylethyl) 2-(fluoromethyl)-1,4-dihydro-6-methyl-4-(2,4,6-trifluorophenyl)-3,5-pyridinedicarboxylate, mp 194-5°.

c) 3-Methyl 5-(1-methylethyl) 2-(fluoromethyl)-1,4-dihydro-6-methyl-4-(pentafluorophenyl)-3,5-pyridinedicarboxylate, mp 132-3°.

Example 60

The following compound was prepared by the method of Example 37 using appropriate starting material: 3-Methyl 5-(1-methylethyl) 4-(5-fluoro-2-methoxyphenyl)-2-(fluoromethyl)-1,4-dihydro-6-methyl-3,5-pyridinedicarboxylate, mp 98-100°.

Example 61

The following compounds were prepared by the method of Example 21 using appropriate starting material:

a) 3-Methyl 5-(1-methylethyl) 4-(2-chloro-3,5-dimethoxyphenyl)-2-(fluoromethyl)-1,4-dihydro-6-methyl-3,5-dihydropyridine, mp 135-6°.

b) 3-Methyl 5-(1-methylethyl) 4-(6-chloro-2-fluoro-3-methoxyphenyl)-2-(fluoromethyl)-1,4-dihydro-6-methyl-3,5-pyridinedicarboxylate, mp 136-7°.

Example 62

The following compound was prepared by the method of Example 39 using appropriate starting material: Diethyl 2-((2-aminoethoxy)methyl)-4-(3-chloro-6- fluoro-2-(trifluoromethyl)phenyl)-1,4-dihydro-6-(trifluoromethyl)-3,5-pyridinedicarboxylate Z-butenedioate salt, mp 108-110°.

Example 63

The following compounds were prepared by the method of Example 40 using appropriate starting materials:

a) 5-Methyl 3-(2,2,2-trichloroethyl) 2-((2-((5-amino-1H-1,2,4-triazol-3-yl)amino)ethoxy)methyl)-4-(2-chlorophenyl)-6-(fluoromethyl)-1,4-dihydro-3,5-pyridinedicarboxylate ethanedioate salt, mp 143-5°.

b) 3-Ethyl 5-methyl 2-((2-((5-amino-1H-1,2,4-triazol-3-yl)amino)ethoxy)methyl)-6-(fluoromethyl)-1,4-dihydro-4-(pentafluorophenyl)-3,5-pyridinedicarboxylate Z-butenedioate salt, mp 110-3°.

c) 3-Ethyl 5-methyl 2-((2-(2-(5-amino-1H-1,2,4-triazol-3-yl)aminoethoxy)ethoxy)methyl)-4-(2-chlorophenyl)-6-(fluoromethyl)-1,4-dihydro-3,5-pyridinedicarboxylate hemi-ethanedioate salt, mp 173-5°.

d) 3-Ethyl 5-methyl 2-((2-((5-amino-1H-1,2,4-triazol-3-yl)amino)ethoxy)methyl)-4-(3-cyanothien--2-yl)-6-(fluoromethyl)-1,4-dihydro-3,5-pyridinedicarboxylate ethanedioate salt, mp 150-2°.

Example 64

3-Methyl 5-(1-methylethyl) 4-(5-amino-2-chlorophenyl)-2-(fluoromethyl)-1,4-dihydro-6-methyl-3,5-pyridinedicarboxylate

Prepared by the method of Example BN, mp 125-7°.

Intermediates

Example A

5-Methyl 3-(1-methylethyl) 2-(bromomethyl)-4-(3-chloro--6-fluoro-2-(trifluoromethyl)phenyl)-6-(fluoromethyl)-1,4-dihydro-3,5-pyridinedicarboxylate

Pyridinium bromide perbromide (0.16g, 0.5mmoles) was added to a stirred solution at 3° of 3-methyl 5-(1-methylethyl) 4-(3-chloro-6-fluoro-2-(trifluoromethyl) phenyl)-2-(fluoromethyl)-1,4-dihydro-6-methyl-3,5-pyridinedicarboxylate (0.2g, 0.43mmoles) and pyridine (0.057ml, 0.71mmoles) in chloroform (5ml). After 20 minutes, the solution was poured onto saturated sodium chloride. The organic layer was separated, washed with 10% sulphuric acid and and saturated sodium chloride, dried ($MgSO_4$) and the solvent was evaporated to give the title bromomethyl compound (0.24g). NMR delta ($CDCl_3$) 6.0 (q,1H), 4.65(q,2H).

Example B

3-Ethyl 5-methyl 2-(bromomethyl)-4-(3-chloro-6-fluoro-2-(trifluoromethyl)phenyl)-6-(fluoromethyl)-1,4-dihydro-3,5-pyridinedicarboxylate

Prepared by the method of Example A.

Example C

5-Methyl 3-(1-methylethyl) 4-(3-chloro-6-fluoro-2-(trifluoromethyl)phenyl)-2-(3-ethoxy-2-(ethoxycarbonyl)-3-oxopropyl-6-(fluoromethyl-1,4-dihydro-3,5-pyridinedicarboxylate

Prepared by the method of Example 1 using sodium methoxide and diethyl 1.3-propanedioate, mp 146-9°.

Example D

Ethyl 4-(2-(1,3-dihydro-1,3-dioxo-2H-isoindol-2-yl) ethylthio)-3-oxobutanoate

Ethyl 4-chloro-3-oxobutanoate (3ml, 22mmoles) was added to a stirred solution of potassium carbonate (6.2g. 44mmoles) and 1,3-dihydro-1.3-dioxo-2H-isoindol-2-ethanethiol (4.6g, 22mmoles) in tetrahydrofuran (50ml). The mixture was heated at 50° for 1 hour, cooled and then water was added. The mixture was acidified to pH1 with hydrochloric acid and the product was extracted with diethyl ether. The organic extract was dried ($Na_2SO_4$) and the solvent evaporated to give the title compound (7.6g).

Example E

Ethyl 3-amino-4-(2-(1,3-dihydro-1,3-dioxo-2H-isoindol-2-yl)ethylthio)-2-butenoate

Ethyl 4-(2-(1,3-dihydro-1.3-dioxo-2H-isoindol-2-yl) ethylthio)-3-oxobutanoate (7.6g, 22.7mmoles) and ammonium acetate (2.0g, 26mmoles) in t-butanol (100ml) was heated at 60° for 1 hour followed by 70 hours at room temperature. The solvent was evaporated and the residue dissolved in diethyl ether and water. The organic layer was separated, dried ($Na_2SO_4$) and the solvent was evaporated. Chromatography on silica eluting with ethyl acetate/petroleum ether (60-80°) gave the title compound (2.5g), mp 79-80°.

Example F

5-Methyl 3-(1-methylethyl) 4-(3-chloro-6-fluoro-2-(trifluoromethyl)phenyl)-2-(cyanomethyl)-6-(fluoromethyl)-1,4-dihydro-3,5-pyridinedicarboxylate

5-Methyl 3-(1-methylethyl) 2-(bromomethyl)-4-(3-chloro--6-fluoro-2-(trifluoromethyl)phenyl)-6-(fluoromethyl)-1,4-dihydro-3,5-pyridinedicarboxylate (2.34g, 4.28mmoles) in dichloromethane (10ml) was added to a stirred solution at 0° of tetraethylammonium cyanide (1.33g, 8.56mmoles) in dichloromethane (100ml). After stirring for 16 hours at room temperature, the organic solution was washed well with water, dried ($Na_2SO_4$) and the solvent evaporated. Chromatography on silica eluting with ethyl acetate/dichloromethane mixtures gave the title compound (0.18g), mp 146-7°.

Example G

3-Methyl 5-(1-methylethyl) 4-(3-chloro-6-fluoro-2-(trifluoromethyl)phenyl)-2-(fluoromethyl)-1,4-dihydro-6-(hydroxymethyl)-3,5-pyridinedicarboxylate

Prepared by the method of Example F using 10% aqueous sulphuric acid in tetrahydrofuran, mp 194-6°.

Example H

5-Methyl 3-(1-methylethyl) 2-(aminomethyl)-4-(3-chloro-6-fluoro-2-(trifluoromethyl)phenyl)-6-(fluoromethyl)-1,4-dihydro-3,5-pyridinedicarboxylate 4-methylbenzenesulphonate salt

Prepared by the method of Example F using ammonia gas, mp 210-2°.

Example I

5-Methyl 3-(1-methylethyl) 4-(3-chloro-6-fluoro-2-(trifluoromethyl)phenyl)-2-(((((1,1-dimethylethoxy) carbonyl)amino)acetyloxy)methyl)-6-(fluoromethyl)-1,4-dihydro-3,5-pyridinedicarboxylate

N-((1,1-Dimethylethoxy)carbonyl)glycine (0.5g, 2.85mmoles) and carbonyldiimidazole (0.46g, 2.85mmoles) in dichloromethane (75ml) were stirred at room temperature for 15 minutes. 3-Methyl 5-(1-methylethyl) 4-(3-chloro-6-fluoro-2-(trifluoromethyl)phenyl)-2-(fluoromethyl)-1,4-dihydro-6-(hydroxymethyl)-3,5-pyridinedicarboxylate (1.38g. 2.85mmoles) and triethylamine (0.8ml, 5.67mmoles) in dichloromethane (10ml) was added and the mixture stirred at room temperature for 3 hours and at reflux for 3 hours. The solvent was evaporated and the residue purified by chromatography on silica eluting with ethyl acetate/dichloromethane mixture to give the title compound (0.7g). M+1+ 641/3: NMR ($CDCl_3$) delta 6.03 (q,1H), 3.58 (s,3H), 1.46 (s.9H).

Example H

5-Methyl 3-(1-methylethyl) 2-(dibromomethyl)-4-(3-chloro-6-fluoro-2-(trifluoromethyl)phenyl)-6-(fluoromethyl)-1,4-dihydro-3,5-pyridinedicarboxylate

Pyridinium bromide perbromide (2.74g, 8.55mmoles) was added to a stirred solution of pyridine (0.7ml, 8.7mmoles) and 3-methyl 5-(1-methylethyl) 4-(3-chloro-6-fluoro-2-(trifluoromethyl)phenyl)-2-(fluoromethyl)-1,4-dihydro-6-methyl-3,5-pyridinedicarboxylate (2g, 4.28mmoles) in chloroform (100ml). After 4 hours, the solution was washed with dilute hydrochloric acid and water, dried ($Na_2SO_4$) and the solvent evaporated. Chromatography on silica eluting with ethyl acetate/petroleum ether (60-80°) mixtures followed by crystallisation from petroleum ether (60-80°) gave the title compound (0.4g), mp 108-10°.

Example K

3-Methyl 5-(1-methylethyl) 4-(3-chloro-6-fluoro-2-(trifluoromethyl)phenyl)-2-(fluoromethyl)-6-formyl-1,4-dihydro-3,5-pyridinedicarboxylate

5-Methyl 3-(1-methylethyl) 2-(dibromomethyl)-4-(3- chloro-6-fluoro-2-(trifluoromethyl)phenyl)-6-(fluoromethyl)-1,4-dihydro-3,5-pyridinedicarboxylate (1.1g, 1.8mmoles), silver nitrate (0.644g, 3.8mmoles), ethanol (50ml) and water (10ml) were heated at reflux for 2 hours. After cooling the solid was filtered off and the filtrate evaporated. The residue in ether was washed with water, dried ($Na_2SO_4$) and the solvent evaporated. Chromatography on silica eluting with petroleum ether (60-80°)/dichloromethane mixtures followed by crystallisation from petroleum ether (60-80°) gave the title compound (0.27g). M+ 481/3; NMR ($CDCl_3$) delta 10.35 (s,1H), 6.16 (q,1H), 3.58 (s,3H).

Example L

5-Methyl 3-(1-methylethyl) 2-azidomethyl-4-(3-chloro-6-fluoro-2-(trifluoromethyl)phenyl)-6-(fluoromethyl)-1,4-dihydro-3,5-pyridinedicarboxylate

5-Methyl 3-(1-methylethyl)-2-(bromomethyl)-4-(3-chloro-6-fluoro-2-(trifluoromethyl)phenyl)-6-(fluoromethyl)-1,4-dihydro-3,5-pyridinedicarboxylate (2.0g, 3.66mmoles) and sodium azide (0.25g, 3.85mmoles) in dry dimethylformamide (10ml) were stirred at 20° for 3 hours. The mixture was poured into brine and ethyl acetate and the organic phase separated and evaporated. The residue was separated by chromatography on silica using ethyl acetate/petroleum ether mixtures as eluants to give the title compound (1.2g) as pale yellow prisms, mp 80-82°.

Example M

3-Ethyl 5-Methyl 2-(aminomethyl)-4-(2-chlorophenyl)-6-(fluoromethyl)-1,4-dihydro-3,5-pyridinedicarboxylate

Prepared by the method of Example H, mp 157-9°.

Example N

3-Ethyl 5-methyl 2-(bromomethyl)-4-(2-chlorophenyl)-6-(fluoromethyl)-1,4-dihydro-3,5-pyridinedicarboxylate

Prepared by the method of Example A.

Example O

3-Ethyl 5-methyl 2-(bromomethyl)-4-(2,3-dichlorophenyl)-6-(fluoromethyl)-1,4-dihydro-3,5-pyridinedicarboxylate

Prepared by the method of Example A. Mass m/e 480/82/84 (M+ +1)

Example P

3-Ethyl 5-methyl 2-(bromomethyl)-4-(2-(trifluoromethyl)phenyl)-6-(fluoromethyl)-1,4-dihydro-3,5-pyridinedicarboxylate, mp 105-7° (dec).

Example Q

5-(2-Cyanoethyl) 3-methyl 4-(3-chloro-6-fluoro-2-(trifluoromethyl)phenyl)-2-(fluoromethyl)-1,4-dihydro-6-methyl-3,5-pyridinedicarboxylate

3-Chloro-6-fluoro-2-(trifluoromethyl)benzaldehyde (68g), 0.3mmoles), methyl 4-fluoro-3-oxobutanoate (40.2g, 0.3mmoles) and 2-cyanoethyl 3-amino-2-butenoate (46g, 0.3mmoles) were heated in dry t-butanol (600ml) under $N_2$ at 55 for 8 days. The reaction mixture was cooled and the product filtered off. Crystallisation

from 2-propanol gave the title compound (34g), mp 167-8°.

Example R

4-(3-Chloro-6-fluoro-2-(trifluoromethyl)phenyl)-6-(fluoromethyl)-1,4-dihydro-5-(methoxycarbonyl)-2-methyl-3-pyridinedicarboxylic acid

Sodium hydroxide (3.75g, 0.094mmoles) in water (120ml) was added to a stirred solution of 5-(2-cyanoethyl) 3-methyl 4-(3-chloro-6-fluoro-2-(trifluoromethyl)phenyl)-2-(fluoromethyl)-1,4-dihydro-6-methyl-3,5-pyridinedicarboxylate (17.6g, 0.037mmoles) in 1,2-dimethoxy ethane (60ml). After stirring for 16 hours, the solution was poured into dilute hydrochloric acid (350ml) and the solid was filtered off, washed with water and methanol and dried in vacuo to give the title acid (14.0g), mp 209-210°.

Example S

3-Methyl 5-(6-(((4-methylphenyl)sulphonyl)oxy)hexyl) 4-(3-chloro-6-fluoro-2-(trifluoromethyl)phenyl)-2-(fluoromethyl)-1,4-dihydro-6-methyl-3,5-pyridinedicarboxylate

Phosphorous pentachloride (4.88g, 23.5mmoles) was added to a stirred suspension of 4-(3-chloro-6-fluoro-2-(trifluoromethyl)phenyl)-6-(fluoromethyl)-1,4-dihydro-5-(methoxycarbonyl)-2-methyl-3-pyridinedicarboxylic acid (10g, 23.5mmoles) in dichloromethane (350ml). After 1 hour, 6-hydroxyhexyl-1-(4-methylbenzene)sulphonate (5.64g, 23.5mmoles) in dichloromethane (100ml) was added slowly. After 16 hours, 5% aqueous sodium carbonate was added with vigorous stirring until the aqueous layer was pH 7-8. The organic layer was separated, washed with brine, dried ($MgSO_4$) and the solvent was evaporated. Purification by HPLC on silica eluting with ethyl acetate/petroleum ether (60-80°) mixtures gave the title compound as an oil (11.8g). NMR delta ($CDCl_3$) 2.32 (s,3H), 2.45 (s,3H), 3.56 (s,3H), 5.93 (q,1H).

Example T

5-(2-Methoxy-2-oxoethyl) 3-methyl 4-(3-chloro-6-fluoro-2-(trifluoromethyl)phenyl)-2-(fluoromethyl)-1,4-dihydro-6-methyl-3,5-pyridinedicarboxylate

2-Methoxy-2-oxoethyl 3-oxobutanoate (8g, 46mmoles), ammonium acetate (3.54g, 46mmoles) and t-butanol (100ml) were heated at 60° for 4 hours. 3-Chloro-6-fluoro-2-(trifluoromethyl)benzaldehyde (10.4g, 46mmoles) and methyl 4-fluoro-3-oxobutanoate (6.5g, 46mmoles) were added and the heating continued for 6 days at 45°. The solvent was evaporated and the residue purified by chromatography on silica eluting with ethyl acetate/petroleum ether (60-80°) mixtures followed by crystallisation from dichloromethane/petroleum ether (60-80°)/2-propanol to give the title compound (3.5g), mp 138-40°.

Example U

5-(Carboxymethyl) 3-methyl 4-(3-chloro-6-fluoro-2-(trifluoromethyl)phenyl)-2-(fluoromethyl)-1,4-dihydro-6-methyl-3,5-pyridinedicarboxylate

Sodium hydroxide solution (0.5g, 12.5mmoles in 10ml water) was added to a stirred solution of 5-(2-methoxy-2-oxoethyl) 3-methyl 4-(3-chloro-6-fluoro-2-(trifluoromethyl)phenyl)-2-(fluoromethyl)-1,4-dihydro-6-methyl-3,5-pyridinedicarboxylate (3.11g, 6.25mmoles) in 1,2-dimethoxy ethane (200ml). After 1.5 hours, the reaction mixture was poured onto dilute hydrochloric acid and extracted with ethyl acetate. The organic extract was washed with water, dried ($MgSO_4$) and the solvent evaporated. The residue was crystallised from dichloromethane/petroleum ether (60-80°) to give the title compound (2.3g), mp 111-4°.

Example V

6-Methyl-2-phenylpyrimidin-4-ylthiol

Phosphorous pentasulphide (3.5g, 7.9mmoles) was added to a stirred mixture of 6-methyl-2-phenylpyrimidin-4-ol (3.5g, 19mmoles) in pyridine (15ml). The reaction mixture was heated at reflux for 1 hour, cooled in ice and then water (30ml) was added. The solvent was evaporated and the residue titurated with water. The title compound (3g) was filtered off and dried in vacuo.

Example W

6-(4,6-Dimethylpyrimidin-2-ylthio)hexan-1-ol

Sodium hydride (3.42g of 50%, 71mmoles) was added to a stirred solution of 4,6-dimethylpyrimidin-2-ylthiol (10g, 71mmoles) in dimethylsulphoxide (100ml). After 1 hour, 6-chlorohexan-1-ol (9.71g, 71mmoles) in dimethylsulphoxide (10ml) was added and the stirring continued for 16 hours. The reaction mixture was poured onto water, neutralised with diluted hydrochloric acid and extracted with ethyl acetate. The organic

extract was dried ($MgSO_4$) and the solvent evaporated. Chromatography on silica eluting with dichloromethane/methanol mixtures gave the title compound (14:5g). M+ 240; NMR ($CDCl_3$) delta 6.7 (s,1H), 2.4 (s,6H).

Example X

6-(1H-Benzimidazol-2-ylthio)hexan-1-ol

Prepared by the method of Example W. M+ 250: NMR ($CDCl_3$) delta 3.7 (t,2H), 3.4 (t,2H).

Example Y

6-(6-Methyl-2-phenylpyrimidin-4-ylthio)hexan-1-ol

Prepared by the method of Example W. NMR ($CDCl_3$) delta 6.85 (s,1H), 2.43 (s,3H).

Example Z

6-(2-Methyl-5-phenylpyrazol-3-yloxy)hexan-1-ol

Prepared by the method of Example W using 6-hydroxyhexyl 4-methylbenzenesulphonate in place of 6-chlorohexan-1-ol. NMR ($CDCl_3$) delta 5.80 (s,1H), 3.70 (s,3H).

Example AA

6-Phenoxyhexyl 3-oxobutanoate

6-Phenoxyhexan-1-ol (2.4g, 12.4mmoles) and 5-acetyl-2,2-dimethyl-1,3-dioxane-4,6-dione (2.3g, 12.4mmoles) in benzene (50ml) were heated at reflux for 4 hours. After cooling the solution was washed with water, dried ($MgSO_4$) and the solvent evaporated to give the title compound (3.1g). M+ 278.

Example AB

6-(Pyridin-3-yloxy)hexyl 3-oxobutanoate

Prepared by the method of Example AA. M+ 279.

Example AC

6-(1H-Imidazol-1-yl)hexyl 3-oxobutanoate

Prepared by the method of Example AA. M+ 252; NMR ($CDCl_3$) delta 7.48 (s,1H), 7.31 (s,1H), 7.06 (s,1H), 3.45 (s,2H), 2.27 (s,3H).

Example AD

Ethyl 8-(1,3-dioxobutyloxy)octanoate

Prepared by the method of Example AA. M+ 272; NMR ($CDCl_3$) delta 3.46 (s,2H), 2.3 (s,3H).

Example AE

6-(6-Methyl-2-phenylpyrimidin-4-ylthio)hexyl 3-oxobutanoate

Diketene (0.92g, 11mmoles) was added dropwise to 6-(6-methyl-2-phenylpyrimidin-4-ylthio)hexan-1-ol (3g, 10mmoles) in toluene (3ml) containing triethylamine (2 drops) keeping the temperature at 85-90°. The reaction was heated at 95° for a further 4 hours, cooled and the solvent was evaporated to give the title compound (3.9g). M+ 385; NMR ($CDCl_3$) delta 6.85 (s,1H), 3.4 (s,2H), 2.45 (s,3H), 2.20 (s,3H).

Example AF

6-(4,6-Dimethylpyrimidin-2-ylthio)hexyl 3-oxobutanoate

Prepared by the method of Example AE. M+ 324; NMR ($CDCl_3$) delta 6.7 (s,1H), 3.5 (s,2H), 2.5 (s,6H), 2.25 (s,3H).

Example AG

6-(1H-Benzimidazol-2-ylthio)hexyl 3-oxobutanoate

Prepared by the method of Example AE. M+ 334: NMR ($CDCl_3$) delta 3.49 (s,2H), 2.28 (s,3H).

Example AH

6-(6-Methyl-2-phenylpyrimidin-4-ylthio)hexyl 3-amino-2-butenoate

6-(6-Methyl-2-phenylpyrimidin-4-ylthio)hexyl 3-oxobutanoate (3.86g, 10mmoles), ammonium acetate (1g, 13mmoles) and t-butanol (20ml) were heated at 50 for 4 hours. The solvent was evaporated and the residue dissolved in ethyl acetate and saturated sodium bicarbonate solution. The organic layer was separated, dried ($Na_2SO_4$) and the solvent evaporated. Crystallisation from cyclohexane gave the title compound (2.7g). M+ 385; NMR ($CDCl_3$) delta 6.93 (s,1H), 4.54 (s,1H), 2.48 (s,3H), 1.89 (s,3H).

Example AI

1,3-Dihydro-2-(3-(5-phenylpyrazol-3-yloxy)propyl)-2H-isoindole-1,3-dione

Prepared by the method of Example W using 1,3-dihydro-2-(3-((4-methylphenylsulphonyl)oxy)propyl)-2H-isoindol-1,3-dione in place of 6-chloro-1-hexanol. M+ 347; NMR ($CDCl_3$) delta 6.03 (s,1H), 4.15 (t,2H), 3.72 (t,2H).

Example AJ

3-(5-Phenylpyrazol-3-yloxy)propylamine

1,3-Dihydro-2-(3-(5-phenylpyrazol-3-yloxy)propyl)-2H-isoindole-1,3-dione (4g, 11.5mmoles), hydrazine hydrate (2.23ml, 45mmoles) and the ethanol (100ml) were heated at reflux for 45 minutes. The reaction mixture was cooled and the solid filtered off. The solid was taken up in dichloromethane/water and the aqueous layer basified to pH10. The organic layer was separated, dried ($MgSO_4$) and the solvent evaporated to give the title compound (0.65g). M+ 218; NMR ($CDCl_3$) delta 7.15 (s,1H), 4.24 (t,2H), 3.0 (t,2H).

Example AK

6-(5-Phenylpyrazol-3-ylamino)hexan-1-ol

3,3-Di(methylthio)-1-phenyl-2-propen-1-one (2.24g, 10mmoles), 6-aminohexan-1-ol (2.83g, 10mmoles) and ethanol (25ml) were heated at reflux for 8 hours. Hydrazine hydrate (0.5g, 10mmoles) was added and the reflux continued for a further 6 hours. The solvent was evaporated and the residue dissolved in chloroform and water. The organic extract was dried ($MgSO_4$) and the solvent evaporated. Purification by chromatography on silica eluting with dichloromethane/methanol mixtures gave the title compound (1.0g). M+ 259; NMR ($CDCl_3$) delta 3.4 (m,2H), 3.02 (m,2H).

Example AL

Ethyl 4-(2-(2-(1,3-dihydro-1,3-dioxo-2H-isoindol-2-yl)ethoxy)ethoxy)-3-oxobutanoate

Sodium hydride (5.7g of 50%, 120mmoles) was added to a solution of 2-(2-ethoxyethyl)-1,3-dihydro-2H-isoindole-1,3-dione (14g, 59mmoles) in dry tetrahydrofuran (300ml). After stirring for 1 hour at room temperature, the mixture was cooled at 0° and, keeping the temperature at 0°, ethyl 4-chloro-3-oxobutanoate (8.14g, 59mmoles) in dry tetrahydrofuran (300ml) was added over 1.5 hours. After stirring at room temperature for 16 hours, ethanol was added to quench the reaction. The reaction mixture was poured onto concentrated hydrochloric acid (12ml)/water/ice and extracted with ethyl acetate (x2). The combined extracts were washed with water and brine, dried ($Na_2SO_4$) and solvent was evaporated. Chromatography on silica eluting with ethyl acetate/petroleum ether (60-80°) mixtures gave the title compound (3.7g). M+ 363; NMR ($CDCl_3$) delta 7.7 (m,4H), 1.2 (t,3H).

Example AM

2-Fluoro-3-(trifluoromethyl)benzaldehyde

Butyl lithium (43ml of 1.6M in hexane) was added to a stirred solution at -78° of 1-fluoro-2-(trifluoromethyl) benzene (10g, 61mmoles) in dry tetrahydrofuran (90ml). After 0.5 hour, N-methyl-N-phenylformamide (8.34g, 61mmoles) was added over 0.5 hour. After a further 0.5 hour at -78°, the mixture was poured into ice (150g)/dilute sulphuric acid (10ml). The product was extracted with ether (X2) and the combined extracts were washed with water (X4), sodium bicarbonate solution and brine, dired ($MgSO_4$) and the solvent removed <u>in vacuo</u>. The residue was distilled (12-15mm Hg) to give a colourless oil (7.9g) which could be further purified by crystallisation from hexane at -30°. M+ 192; NMR ($CDCl_3$) delta 10.4 (s,1H).

Example AN

1-Methyl-3-nitro-2-(trifluoromethyl)benzene

2-Iodo-1-methyl-3-nitrobenzene (8g, 30mmoles), activated copper (8g), trifluoromethyl iodide (25g) and dimethylformamide (30ml) were heated at 130° for 1 day in a pressure bottle. Purification by chromatography on silica eluting with ethyl acetate/petroleum ether (60-80°) gave the title compound (2.4g) as a colourless solid. M+ 205; NMR ($CDCl_3$) delta 7.5(m,3H), 2.65 (q,3H).

Example AO

1-(Dibromomethyl)-3-nitro-2-(trifluoromethyl)benzene

1-Methyl-3-nitro-2-(trifluoromethyl)benzene (2.5g, 12.2mmoles), N-bromosuccinimide (3.88g, 21.8mmoles) and dibenzoyl peroxide (catalytic amount) were heated at reflux in carbon tetrachloride (60ml) for 3 days. N-Bromosuccinimide (1.0g, 5.6mmoles) was added and the heating continued for 1 day. The cooled reaction mixture was filtered and the solvent was removed. The residue was chromatographed on silica eluting with petroleum ether (60-80°)/ethyl acetate mixtures to give the title compound (2.3g). NMR ($CDCl_3$) delta 8.4-7.5(m).

Example AP

3-Nitro-2-(trifluoromethyl)benzaldehyde

1-(Dibromomethyl)-3-nitro-2-(trifluoromethyl)benzene (3.0g. 8.3mmoles) was dissolved in dichloromethane (20ml) under nitrogen and then fuming sulphuric acid (2.0ml) was added dropwise. After 4 hours, further fuming sulphuric acid (1.0ml) was added and the mixture stirred overnight. The reaction mixture was poured onto ice/water, neutralised with sodium bicarbonate and the aldehyde extracted with ether. The organic extract was dried ($Na_2SO_4$) and the solvent removed. The aldehyde (0.9g) crystallised on addition of petroleum ether (60-80°).

A small sample further purified by chromatography on silica eluting with petroleum ether (60-80°)/ ethyl acetate and crystallised from petroleum ether (60-80°), mp 61-2°.

Example AQ

N-(4-Chloro-2-methyl-3-(trifluoromethyl)phenyl)-2,2-dimethylpropanamide

Butyl lithium (115ml of 1.6M in hexane, 0.185mmoles) was added with stirring to a solution of N-(4-chloro-3-(trifluoromethyl)phenyl)-2,2-dimethylpropanamide (21.5g, 0.077moles) in tetrahydrofuran (250ml) at 5°. After 1.5 hours at 0-5°, iodomethane (12g, 0.08mmoles) in petroleum ether (60-80°) (20ml) was added below 5°. After 1 hour at 0-5°, ether and water were added. The ethereal layer was separated and the aqueous layer was extracted with ether (X2). The organic extracts were washed with brine, dried ($Na_2SO_4$) and the solvent evaporated. The title compound (14.5g) was obtained after crystallisation from ether/petroleum ether (60-80°). M+ 295/293; NMR ($CDCl_3$) delta 2.35 (q,3H).

Example AR

4-Chloro-2-methyl-3-(trifluoromethyl)benzenamine hydrochloride

N-(4-Chloro-2-methyl-3-(trifluoromethyl)-phenyl)-2,2-dimethylpropanamide (13g, 44 mmoles) was heated at reflux in ethanol (100ml) and c.hydrochloric acid (100ml) for 8 hours. The solvent was removed in vacuo and the residue titurated with ether to give the aniline hydrochloride (8.9g). M+ 211/209; NMR ($D_6DMSO$) delta 2.31 (q,3H).

Example AS

1-Chloro-3-methyl-2-(trifluoromethyl)benzene

A solution of sodium nitrite (2.16g, 31mmoles) in water (5ml) was added slowly between -5° and 0° to a stirred suspension of 4-chloro-2-methyl-3-(trifluoromethyl) benzenamine hydrochloride (7.0g, 28.5mmoles) in concentrated hydrochloric acid (11.25ml) and water (40ml). After stirring for 1 hour at 0°, the solution was poured onto hypophosphorous acid (45ml, d 1.2) at 0° and left overnight at room temperature. The product was extracted with ether, the extracts washed with water and sodium bicarbonate solution, dried ($Na_2SO_4$) and the solvent was evaporated. Distillation (bath temperature 100° at 15mm Hg) gave the title compound (5.0g). M+ 196/194: NMR ($CDCl_3$) delta 2.54 (q,3H).

Example AT

3-Chloro-2-(trifuoromethyl)benzaldehyde

1-Chloro-3-methyl-2-(trifluoromethyl)benzene (2.5g, 13mmoles), N-bromosuccinimide (5.72g, 32mmoles) and benzoyl peroxide (catalytic amount) in carbon tetrachloride (50ml) was heated at reflux with stirring for 24 hours. The reaction mixture was cooled diluted with dichloromethane (50ml) and filtered. The filtrate was washed with water (X2), dried ($Na_2SO_4$) and the solvent removed to give 1-(dibromomethyl)-3-chloro-2-(trifluoromethyl)benzene. This compound was dissolved in dichloromethane (30ml) and the fuming sulphuric acid (4ml) was added dropwise with stirring. After stirring for 16 hours, ice and water were added. The organic layer was separated, washed with water and sodium bicarbonate solution, dried ($Na_2SO_4$) and the solvent removed to give the aldehyde. M+ 210/208; NMR ($CDCl_3$) delta 10.4 (q,1H).

Example AU

2-Fluoro-6-(pentafluoroethyl)benzaldehyde

Butyl lithium (13.2ml of 1.45M in hexane, 19mmoles) was added to a stirred solution at -78° of 1-fluoro-3-(pentafluoroethyl)benzene (4.55g, 21mmoles) in dry tetrahydrofuran (170ml). After 4.5 hours at -78°, methyl formate (13.5g, 210mmoles) was added. After a further 1 hour at -78°, the mixture was poured onto ice (150g)/dilute sulphuric acid (10ml). The product was extracted with ether (x3) and the combined extracts were washed with brine (x3). Distillation of the solvent at atmospheric pressure followed by chromatography on silica eluting with ether/petroleum ether (60-80°) mixtures gave the desired aldehyde. Further purification by distillation gave the aldehyde (1.63g). M+ 242; NMR ($CDCl_3$) delta 10.3 (t,1H).

Example AV

1-Bromo-8-fluoronaphthalene

A solution of sodium nitrite (4g, 58mmoles) in water (10ml) was added slowly between -5° and 0° to a stirred mixture of 8-bromo-1-naphthalenamine (12g, 54mmoles), water (150ml) and c.hydrochloric acid (150ml). After stirring for 1 hour at 0°, fluoroboric acid (48ml of 40%) was added. After 20 minutes, the solid was filtered off, washed with water and dried over $P_2O_5$, washed with ether and redried to give 8-bromo-1-naphthalenediazonium tetrafluoroborate (15g).

The diazonium salt (15g) was heated at 110° until decomposition was complete. The residue was dissolved in ether and this solution washed with 10% sodium hydroxide solution and water, dried ($MgSO_4$) and the solvent evaporated. Chromatography on silica eluting with petroleum ether (60-80°) gave the title compound (3.4g). M+ 226/224; NMR ($CDCl_3$) delta 7.75 (d,2H), 7.61 (d,1H).

Example AW

8-Fluoro-1-naphthalenecarboxaldehyde

Butyl lithium (17.5ml of 1.6M in hexane, 27mmoles) was added to a stirred solution at -78° of 1-bromo-8-fluoronaphthalene (3.1g, 14mmoles) in dry ether (50ml). After 5 minutes, methyl formate (15ml) was added. After a further 10 minutes, the mixture was poured onto ether/dilute hydrochloric acid. The ethereal layer was separated, dried ($MgSO_4$) and the solvent evaporated. Crystallisation from petroleum ether (60-80°) gave the desired aldehyde (1.2g). M+ 174; NMR ($CDCl_3$) delta 11.0 (d,1H).

Example AX

3-Cyclobutyl 5-methyl 4-(3-chloro-6-fluoro-2-(trifluoromethyl)phenyl)-2-((2-((2-(cyclobutyloxycarbonyl)benzoyl)amino)ethoxy)methyl)-6-(fluoromethyl)-1,4-dihydro-3,5-pyridinedicarboxylate

2-((2-((2-Carboxybenzoyl)amino)ethoxy)methyl)-4-(3-chloro-6-fluoro-2-(trifluoromethyl)phenyl)-6-(fluoromethyl)-1,4-dihydro-5-(methoxycarbonyl)-3-pyridinecarboxylic acid (2.5g, 4mmoles) and phosphorous pentachloride (2.46g, 12mmoles) in dichloromethane (150ml) were stirred for 45 minutes and then cyclobutanol (0.625g, 8.7mmoles) was added. After stirring overnight, potassium carbonate (10g) and water were added. After vigorous stirring for 1.5 hours, the layers were separated and the aqueous extract re-extracted with dichloromethane. The organic extracts were washed with water and brine, dried ($MgSO_4$) and the solvent evaporated. Chromatography on silica eluting with dichloromethane/methanol mixtures gave the title diester (2.4g). M/e 742/4 M+1+; NMR delta ($CDCl_3$) 3.57 (s,3H), 4.90 (m,1H), 5.20 (m,1H), 6.03 (q,1H), 6.2 (t,1H), 8.15 (d,1H).

Example AY

3-Methyl 5-(3-thietanyl) 4-(3-chloro-6-fluoro-2-(trifluoromethyl)phenyl)-2-(fluoromethyl)-1,4-dihydro-6-((2-((2-(3-thietanyloxycarbonyl)benzoyl)amino)ethoxy) methyl)-3,5-pyridinedicarboxylate

2-((2-((2-Carboxybenzoyl)amino)ethoxy)methyl)-4-(3-chloro-6-fluoro-2-(trifluoromethyl)phenyl)-6-(fluoromethyl)-1,4-dihydro-5-(methoxycarbonyl)-3-pyridinedicarboxylic acid (3.0g, 4.9mmoles), dicyclohexylcarbodiimide (2.2g, 11mmoles), thietan-3-ol (1.0g, 11mmoles) and 4-(dimethylamino)pyridine (0.13g, 11mmoles) in dichloromethane (80ml) were stirred at room temperature for 40 hours. The solvent was evaporated and the residue treated with 4:1 ethyl acetate/ether. The solid was filtered off and the solvent evaporated to give the crude diester (4.1g).

Example AZ

2-Cyanoethyl 4-(2-(1,3-dihydro-1,3-dioxo-2H-isoindol-2-yl)ethoxy)-3-oxobutanoate

Ethyl 4-(2-(1,3-dihydro-1,3-dioxo-2H-isoindol-2-yl) ethoxy)-3-oxobutanoate (8g, 25mmoles) and 3-hydroxypropionitrile (12g, 170mmoles) were heated at 100 under $N_2$ for 10 hours in toluene (100ml) containing a catalytic amount of 4-methylphenylsulphonic acid. Ethyl acetate and water were added; the organic layer was

separated, washed with water, dried ($MgSO_4$) and the solvent evaporated. Chromatography on silica eluting with ethyl acetate/petroleum ether (60-80°) mixtures gave the title ester (8.9g). M/e 345 M+1+; NMR delta ($CDCl_3$) 4.14 (s,2H), 3.53 (s,2H).

Example BA

2-Cyanoethyl 2((3-chloro-6-fluoro-2-(trifluoromethyl) phenyl)methylene)-4-(2-(1,3-dihydro-1,3-dioxo-2H-isoindol-2-yl)ethoxy)-3-oxobutanoate

2-Cyanoethyl 4-(2-(1,3-dihydro-1,3-dioxo-2H-isoindol-2-yl)ethoxy)-3-oxobutanoate (8.9g, 26mmoles) and 3-chloro-6-fluoro-2-(trifluoromethyl)benzaldehyde (5.84g, 26mmoles) in ethanol(300ml) containing hexanoic acid (35 drops) and piperidine (35 drops) was stirred at room temperature for 24 hours. The ethanol was evaporated and the residue in ethyl acetate was washed with dilute hydrochloric acid, 2% aqueous sodium carbonate, water and brine. The organic extract was dried ($MgSO_4$) and the solvent evaporated to give the crude title compound (14.4g).

Example BB

2-((2-((2-Carboxybenzoyl)amino)ethoxy)methyl)-4-(3-chloro-6-fluoro-2-(trifluoromethyl)phenyl)-6-(fluoromethyl)-1,4-dihydro-5-(methoxycarbonyl)-3-pyridinecarboxylic acid

2-Cyanoethyl 2-((3-chloro-6-fluoro-2-(trifluoromethyl)phenyl)methylene)-4-(2-(1,3-dioxo-1,3-dihydro-2H-isodinol-2-yl)ethoxy)-3-oxobutanoate, methyl 4-fluoro-3-oxobutanoate and ammonia (8ml, d 0.88) in propan-2-one (200ml) were stirred at room temperature for 24 hours. The solvent was evaporated and the residue in ethyl acetate was washed with water and dried ($MgSO_4$). The solvent was evaporated and the residue in dichloromethane (50ml) was treated with trifluoroacetic anhyride (12ml). After 20 minutes methanol was added and then the solvent was evaporated. The residue was dissolved in 1,2-dimethoxyethane (85ml) and neutralised with 2N sodium hydroxide. Sodium hydroxide (35ml of 2N) was then added and the mixture stirred for 1 hour. Ethyl acetate and water were added, the aqueous layer was separated and re-extracted with ethyl acetate. The aqueous layer was acidified with dilute hydrochloric acid and the resulting solid filtered off. After drying under vacuum at room temperature the title diacid (9.0g) was obtained. M/e 634/6 M+1+; NMR delta ($D_6$-DMSO) 4.63 (q,2H), 5.88 (q,1H), 8.36 (d,1H), 8.43 (t,1H).

Example BC

3-Ethyl 5-methyl 2-((2-(1,3-dihydro-1,3-dioxo-2H-isoindol-2-yl)ethoxy)methyl)-6-(fluoromethyl)-1,4-dihydro-4-(5-nitrothien-2-yl)-3,5-pyridinedicarboxylate

Ethyl 4-(2-(1,3-dihydro-1,3-dioxo-2H-isoindol-2-yl)ethoxy)-3-oxobutanoate (6.38g, 20mmoles) and ammonium acetate (1.7g, 22 mmoles) in dry t-butanol (20ml) were warmed at 60°C for 2 hours and 5-nitrothiphene-2-carboxaldehyde (3.41g, 20mmoles) and methyl 4-(fluoro-3- osobutanoate (2.68g 20mmoles) added. The resulting solution was stirred at 40°C for 4 days then evaporated and separated by chromatography on silica using ethyl acetate/petroleum ether mixtures as eluant, followed by purification by chromatography on silica using dichloromethane/methanol mixtures. The product was obtained as a viscous red syrup (4.7g). M+ +1 574/6; NMR ($CDCL_3$) delta 3.73 (3H,s), 3.84 (2H,t), 3.99 (2H,t), 5.28 (1H,s).

Example BD

3-Ethyl 5-methyl 4-(5-cyanothien-2-yl)-2-((2-(1,3-dihydro-1,3-dioxo-2H-isoindol-2-yl)ethoxy)methyl)-6-(fluoromethyl)-1,4-dihydro-3,5-pyridinedicarboxylate

5-Formylthiophene-2-carbonitrile (1.0g, 7.3mmoles), methyl 4-fluoro-3-oxobutanoate (0.98g, 7.3mmoles) and ethyl 3-amino-4-(2-(1,3-dihydro-1,3-dioxo-2H-isoindol 2-yl)ethoxy)-but-2-enoate (2.32g, 7.3mmoles) in dry t-butanol (10ml) were stirred at 40° for 6 days. Evaporation of the solvent and trituration of the residue with petroleum ether afforded 3-ethyl 5-methyl 4-(5-cyanothietan-2-yl) (((1,3-dihydro-1,3-2H-isoindol-2-yl)ethoxy)methyl)-6-(fluoromethyl)-6-hydroxy-1,4,5,6,-tetrahydro-3,5-pyridinedicarboxylate (1.2g) as pale buff crystals. mp 141-3°. This was converted to the title compound by treatment with 1 equivalent of trifluoroacetic anhydride and 2 equivalents of pyridine in dichloromethane at 20°.

Example BE

3-Ethyl 5-methyl 2-((2-(1,3-dihydro-1,3-dioxo-2H-isoindol-2-yl)ethoxy)methyl)-4-(3-(1,3-dioxolan-2-yl)-thien-2-yl)-6-(fluoromethyl)-1,4-dihydro-3,5-pyridinedicarboxylate

Prepared by the method of Example BC using appropriate starting materials and obtained as an oil. M+ +1 601; NMR ($CDCL_3$) delta 6.1 (s,1H), 5.4 (s,1H).

Example BF

3-Ethyl 5-methyl 2-((2-(1,3-dihydro-1,3-dioxo-2H-isoindol-2-yl)ethoxy)methyl)-6-(fluoromethyl)-4-(3-formylthien-2-yl)-1,4-dihydro-3,5-pyridinedicarboxylate

The product of Example BE (7.0g) in tetrahydrofuran (100ml) and 10% hydrochloric acid solution (15ml) was stirred at 20° for 1 hour. The solution was poured into ethyl acetate (300ml) and water (300ml) and the organic solution separated, washed and dried. Evaporation of solvent andrecrystallisation of the residue from acetone-petroleum ether gave golden yellow crystals (4.2g) M+ +1 557; NMR ($CDCl_3$) delta 10.21 (s,1H), 5.88 (s,1H).

Example BG

3-Ethyl 5-methyl 2-((2-(1,3-dihydro-1,3-dioxo-2H-isoindol-2-yl)ethoxy)methyl)-6-(fluoromethyl)-4-(3-(hydroxyimino)methylthien-2-yl)-1,4-dihydro-3,5- pyridinedicarboxylate

The product of Example BF (2.0g, 3.6mmoles), hydroxylamine hydrochloride (0.35g, 5.1mmoles) and sodium acetate (0.55g, 6.6mmoles) in acetic acid (100ml) were stirred at 20° for 3 hours. The mixture was diluted with water and neutralised with solid sodium hydrogen carbonate. The product was extracted into ethyl acetate and the organic solution was washed, dried and evaporated to give the title oxime (mixture of E and Z isomers) as an amorphous foam (2.01g). M+ +1 572; NMR ($CDCl_3$) delta 8.40 (s,1H), 5.35 (s,1H).

Example BH

3-Ethyl 5-methyl 4-(3-cyanothien-2-yl)-2-(((1,3-dihydro-1,3-dioxo-2H-isoindol-2-yl)ethoxy)methyl)-6-(fluoromethyl)-1,4-dihydro-3,5-pyridinedicarboxylate

The product of Example BG and N-(2,2,2-trifluoroacetyl)imidazole (1.23g, 7.5mmoles) in dry tetrahydrofuran (200ml) were heated at reflux overnight, evaporated and the residue dissolved in ethyl acetate and water. The organic solution was separated, washed, dried and evaporated. The residue was purified by chromatography on silica using dichloromethane/ethyl acetate as eluant to give the title compound as a yellow oil (1.6g). M+ +1 554; NMR ($CDCl_3$) delta 7.13 (d,1H), 7.01 (d,1H), 5.58 (s,1H).

Example BI

3-Ethyl 5-methyl 2-((2-(1,3-dihydro-1,3-dioxo-2H-isoindol-2-yl)ethoxy)methyl)-6-(fluoromethyl)-1,4-dihydro-4-(3-methylthien-2-yl)-3,5-pyridinedicarboxylate

Prepared as an oil by the method of Example BC using appropriate starting materials.

Example BJ

3-Ethyl 5-methyl 4-(2,5-difluoro-6-(trifluoromethyl) phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Example BC as a pale yellow solid. M+ +1 627; NMR ($CDCl_3$) delta 4.55 (d,2H), 5.80 (m,1H).

Example BK

3-Ethyl 5-methyl 4-(2-chlorophenyl)-2-((2-(2-(1,3-dihydro-1,3-dioxo-2H-isoindol-2-yl)ethoxy)ethoxy)methyl)--6-(fluoromethyl)-1,4-dihydro-3,5-pyridinedicarboxylate

Prepared by the method of Example 30 and obtained as a yellow solid, mp 119-20°.

Example BL

Diethyl 4-(2-chlorophenyl)-2-((2-(1,3-dihydro-1,3-dioxo-2H-isoindol-2-yl)ethoxy)methyl)-1,4-dihydro)-6-(trifluoromethyl)-3,5-pyridinedicarboxylate

2-Chlorobenzaldehyde (1.4g, 10mmoles), ethyl 4,4,4-trifluoro-3-oxobutanoate (1.84g, 10mmoles) and ethyl 3-amino-4-(2-(1,3-dihydro-1,3-dioxo-2H-isoindol-2-yl)ethoxy)-but-2-enoate (3.18g, 10mmoles) in dry t-butanol (20ml) were stirred at 60° for 6 days. The solvent was evaporated and the residue dissolved in dichloromethane (150ml) and concentrated sulphuric acid (20ml) and stirred vigorously for 5 minutes. Water (200ml) was then added cautiously and the organic solution separated, dried and evaporated. The residue was

purified by chromatography on silica using ethyl acetate-petroleum ether mixtures to give the title compound as a pale yellow oil, which solidifies on prolonged standing (1.4g). M+ 606/8; NMR ($CDCl_3$) delta 5.49 (s,1H), 4.76 (AB pattern,2H).

Example BM

3-Ethyl 5-methyl 4-(2,6-difluoro-3-nitrophenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Example BC as an orange solid. M + 1 + 604; NMR ($CDCl_3$) delta, 6.92 (1H,t), 3.60 (3H,s).

Example BN

3-Ethyl 5-methyl 4-(3-amino-2,6-difluorophenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

The product of Example BM (2.5g, 4.15mmoles) and stannous chloride dihydrate (4.67g, 5 equiv) in ethanol (50ml) were boiled at reflux for 30 minutes, and the resulting solution poured onto ice and made slightly basic with aqueous sodium hydroxide. The resulting suspension was filtered and the filtrate extracted with ethyl acetate. The organic extract was dried and evaporated and the residue purified by chromatography on silica using ethyl acetate-petroleum ether mixtures as eluant to give the title compound. M+ +1 574; NMR ($CDCl_3$) delta 3.58 (s,3H), 7.98 (d,1H).

Example BO

3-Ethyl 5-methyl 4-(2,3-dichlorophenyl)-2-(((2,2-diethoxy)ethoxy)methyl)-6-(fluoromethyl)-1,4-dihydro-3,5-pyridinedicarboxylate

Sodium hydride (0.96g, 0.02mmoles), 50% oil dispersion, was added to a solution of 2,2-diethoxyethanol (10ml) in dry tetrahydrofuran (25ml). When the initial reaction had subsided the product of Example O (4.81g, 0.01mmoles) was added and the solution stirred for 30 minutes, poured into water and ether and acidified with dilute sulphuric acid solution. The organic phase was separated, dried and evaporated and the residue purified by chromatography on silica using ethyl acetate-petroleum ether as eluant to give the title compound (3.5g) as crystals, mp 75-7° (petroleum ether).

Example BP

5-Ethyl 3-methyl 4-(2,3-dichlorophenyl)-2-(fluoromethyl)-1,4-dihydro-6-((2-(hydroxyimino)ethoxy)methyl)-3,5-pyridinedicarboxylate

The product of Example BO (2.5g, 4.68mmoles) and hydroxylamine hydrochloride (0.97g, 14 mmoles) in ethanol (50ml) and water (2ml) were heated at reflux for 3 hours. The solvent was removed in vacuo and the residue partitioned between ether and water. The organic solution was separated, dried and evaporated to give the title compound as a mixture of E and Z isomers (1.7g). M+ +1 479/7/5.

Example BQ

5-Methyl 3-(2,2,2-trifluoroethyl) 2-(bromoethyl) 6-(fluoromethyl)-1,4-dihydro-4-(2-trifluoromethyl)phenyl)-3,5-pyridinedicarboxylate

Prepared by the method of Example A using the compound of Example 68 a).

Example BR

5-Methyl 3-(2,2,2-trifluoroethyl) 2-((2-hydroxyimino)ethoxy)methyl)-6-(fluoromethyl)-1,4-dihydro-4-(2-trifluoromethyl)phenyl)-3,5-pyridinedicarboxylate

Prepared from the product of Example BQ using the methods of Examples BO and BP without purification of the intermediate acetal. The product was obtained as a yellow solid. M+ +1 529.

Example BS

5-Methyl 3-(1-methylethyl)2-(bromomethyl)-6-(fluoromethyl)-4-(2,3,5,6-tetrafluorophenyl)-1,4-dihydro-3,5-pyridinedicarboxylate

Prepared by the method of Example A, mp 134-5° (dec).

Example BT

5-Methyl 3-(1-methylethyl) 2-((2.2-diethoxy)ethoxy)methyl)-6-(fluoromethyl)-4-(2,3,5,6-tetrafluorophenyl)-1,4-dihydro-3,5-pyridinedicarboxylate.

Prepared by the method of Example BO as pale yellow needles from petroleum ether, mp 109-10°.

Example BU

3-Methyl 5-(1-methylethyl) 2-(fluoromethyl)-6-((2-(hydroxyimino)ethoxy)methyl)-1.4-dihydro-4-(2,3,5,6-tetrafluorophenyl)-3,5-pyridinedicarboxylate

Prepared by the method of Example BP as a mixture of E and Z isomers, mp 130-4°.

Example BV

3-Methyl 5-(1-methylethyl) 2-(fluoromethyl)-6-((2-(hydroxyimino)ethoxy)methyl)-1,4-dihydro-4-(2,4,6-trifluorophenyl)-3,5-pyridinedicarboxylate

Prepared from the product of Example 68 b) by using the methods of Examples A, BO and BP without purification of intermediate compounds. The product was obtained as a pale solid. M+ 474; NMR ($CDCl_3$) delta 7.5 (t,1H), 6.94 (t,1H), 5.4 (s,1H).

Example BW

2-Fluoro-5-methoxybenzaldehyde and 5-fluoro-2-methoxybenzaldehyde

sec-Butyl lithium (200ml, 1.4M solution in cyclohexane) was added to a 4-fluoroanisole (30g) and tetramethylethylenediamine (27g) in dry tetrahydrofuran (400ml) at -45° under nitrogen and the solution was stirred at -45° for 3 hours. Methyl formate (40ml) in dry tetrahydrofuran (40ml) was added at -60° and the resulting solution stirred at -60° for 1.5 hours and then poured into ice and dilute hydrochloric acid. The mixture was extracted with ethyl acetate and the extract separated, washed and dried. The residue was separated by chromatography on silica using ethyl acetate/petroleum ether as eluant into two components:-

i) Less polar - 2-fluoro-5-methoxybenzaldehyde (3.8g). M+ 154; NMR ($CDCl_3$) delta 10.31 (s,1H).

ii) More polar - 5-fluoro-2-methoxybenzaldehyde (3.2g). M+ 154; NMR ($CDCl_3$) delta 10.41 (d,1H).

Example BX

3-Ethyl 5-methyl 2-((2-(1,3-dihydro-1,3-dioxo-2H-isoindol-2-yl)ethoxy)methyl)-4-(2-fluoro-5-methoxyphenyl)-6-(fluoromethyl)-1,4-dihydro-3,5-pyridinedicarboxylate

Prepared by the method of Example BC and obtained as an oil. M+ 570; NMR ($CDCl_3$) delta 5.2 (s,1H), 3.72 (s,3H), 3.62 (s,3H).

Example BY

6-(2,6-dimethylpyrimidin-4-yloxy)hexanol

Prepared by the method of Example W. M+ +1 297 (Trimethylsilyl derivative); NMR ($CDCl_3$) delta 6.2 (s,1H), 4.25 (t,2H).

Example BZ

6-(2,6-dimethylpyrimidin-4-yloxy)hexyl 3-amino buten-2-oate

Diketene (1.0ml) was added dropwise to a mixture of the product of Example BY (2.7g) and triethylamine (0.02ml) at 80°, and the resulting solution stirred at 80° for 3 hours. Ammonium acetate (1.0g) and dry t-butanol (30ml) were added to the cooled mixture and the resulting solution warmed at 60° for 6 hours. The solvent was removed by distillation and the residue dissolved in dichloromethane and aqueous sodium bicarbonate. The organic solution after concentration afforded the title compound as a yellow solid. M+ 307; NMR ($CDCl_3$) delta 6.4 (s,1H), 4.6 (s,1H).

Example CA

6-(6-Methyl-2-phenylpyrimidin-4-ylthio)hexyl 4-(2-(1,3-dihydro-1,3-dioxo-2H-isoindol-2-yl)ethoxy)-3-oxobutanoate

Ethyl 4-(2-(1,3-dihydro-1,3-dioxo-2H-isodinol-2-yl)ethoxy)-3-oxobutanoate (3.8g) and 6-((6-methyl-2-phenyl

pyrimidin-4-yl)thio)hexan-1-ol (4.2g) in dry toluene containing 4-methylbenzenesulphonic acid (0.1g) were heated under reflux for 21 hours. The solvent was removed by distillation and the residue purified by chromatography on silica using tetrahydrofuran-petroleum ether mixtures to give the title compound as a pale orange oil (6.6g).

The compounds of Examples CB and CD were made using the method of Example CA.

Example CB

6-Phenoxyhexyl 4-(2-(1,3-dihydro-1,3-dioxo-2H-isoindol-2-yl)ethoxy)-3-oxobutanoate. M+ 467.

ExampleCC

6-(6-methyl-2-phenylpyrimidin-4-yloxy)hexyl 4-(2-(1,3-dihydro-1,3-dioxo-2H-isoindol-2-yl)ethoxy)-3-oxobutanoate.

M+ +1 560.

Example CD

6-(4,6-dimethylpyrimidin-2-ylthio)hexyl 4-(2-(1,3-dihydro-1,3-dioxo-2H-isoindol-2-yl)ethoxy)-3-oxobutanoate.

Example CE

5-Methyl 3-(6-(6-methyl-2-phenylpyrimidin-4-ylthio)hexyl) 4-(2-chlorolphenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

The product of Example CA (5.13g, 10mmoles) and ammonium acetate (0.8g, 10.4mmoles) in dry t-butanol were stirred at 60° for 5 hours and then 2-chlorobenzaldehyde (1.40g, 10mmoles) and methyl 4-fluoro-3-oxobutanoate (1.34g, 10 mmoles) were added. The mixture was heated at 55° for 14 days and then the solvent was removed by distillation. Separation of the residue by chromatography on silica using ethyl acetate-petroleum ether mixture as eluant afforded the title compound as an oil (2.4g). M+ +1 813/5.

The compounds of Examples CF and CH were made by the method of Example CE.

Example CF

5-Methyl 3-(6-phenoxyhexyl) 4-(2-chlorophenyl) 2-((2-(1,3-dihydro-1,3-dioxo-2H-isoindol-2-yl)ethoxy)methyl)-6-(fluoromethyl)-1,4-dihydro-3,5-pyridinedicarboxylate.

M+ 705/7.

Example CG

5-Methyl 3-(6-(6-methyl-2-phenylpyrimidin-4-yloxy)hexyl) 4-(2-chlorophenyl)-2-((2-(1,3-dihydro-1,3-dioxo-2H-isoindol-2-yl)ethoxy)methyl)-6-(fluoromethyl)-1,4-dihydro- 3,5-pyridinedicarboxylate. M+ +1 797/9.

Example CH

3-(6-(4,6-dimethyl-2-pyrimidinylthio)hexyl) 5-methyl 4-(2-chlorophenyl)-2-((2-(1,3-dihyro-1,3-dioxo-2H-isoindol-2-yl)ethoxy)methyl)-6-(fluoromethyl)-1,4-dihydro-3,5-pyridinedicarboxylate. M+ +1 751/3.

Example CI

2-(2-Chloro-3,5-dimethoxyphenyl)-4,5-dihydro-4,4-dimethyloxazole

sec-Butyl lithium (44ml, 1.3M in cyclohexane) was added dropwise to 2-(3,5-dimethoxyphenyl)-4,5-dihydro-4,4-dimethyloxazole (11.5g, 48mmoles) and tetramethylethylenediamine (8.1ml) in 1,2-dimethoxyethane (100ml) at -78°. The solution was stirred at -70° for 1.5 hours and then 4-methylbenzenesulphonylchloride (9.8g, 51mmoles) in 1,2-dimethoxyethane (20ml) was added over 20 minutes. The mixture was stirred at 20° for 16 hours, then poured into water and ether. The organic phase was washed with 1M sodium hydroxide solution and brine, dried and evaporated. The residue was purified by high pressure liquid chromatography on silica eluting with tetrahydrofuran-hexane to give the title compound (11.6g). M/e, M+ 269/71; NMR ($CDCl_3$) delta 1.41 (6H,s), 6.57 (1H,d), 6.78 (1H,d).

Example CJ

2-Chloro-3,5-dimethoxybenzaldehyde

The product of Example CI (10g, 37mmoles) and iodomethane (20ml) were kept at 20° for 8 days. Excess iodomethane was distilled offand the residue triturated with ether. The resulting crystalline methiodide salt (13g, 32mmoles) in ethanol (250ml) was treated portionwise with sodium borohydride (1.2g, 32mmoles) keeping the temperature below 5°, and the mixture was then stirred at 20° for 3 hours. The solution was

acidified with 2M hydrochloric acid solution and after 30 minutes was poured into brine and ethyl acetate. The organic phase was separated, dried and evaporated to give the title compound (3.5g). M+ 200/2; NMR ($CDCl_3$) delta 10.50 (1H,s), 7.02 (1H,d), 6.73 (1H,d).

Example CK

4-Chloro-2-fluoro-1-methoxybenzene

Sodium nitrite (14g, 0.2mmoles) in water (30ml) was added dropwise with vigorous stirring to 5-chloro-2-methoxybenzeneamine hydrochloride (37.7g, 0.2mmoles) in concentrated hydrochloric acid (60ml) and water (60 ml ) at 0-5°. After 1 hour at 0°, fluoroboric acid (40% aqueous solution, 45ml) was added. The precipitated diazonium tetrafluoroborate salt was filtered, washed with cold water and ether, and dried under vacuum. This salt (32.7g) was heated at 180° until evolution of gas was complete. Ether and saturated sodium hydrogen carbonate solution were added to the cooled product and the ether layer separated, dried and concentrated. The residue was purified by bulb to bulb distillation to give the title compound (9.7g) base peak 100° (bath temperature)/20mm Hg. M+ 160/2; NMR ($CDCl_3$) delta 6.88 (1H,t), 7.05 (1H,octet), 7.09 (1H,dd).

Example CL

6-Chloro-2-fluoro-3-methoxybenzaldehyde

n-Butyl lithium (50ml, 1.55M in hexane) was added to the product of Example CK (8.7g) in dry tetrahydrofuran (100ml) at -70°. The solution was maintained at -70° for 1.5 hours, then methyl formate (20ml) was added. After a further 1.5 hours, the mixture was poured into 1M hydrochloric acid and ether. The organic phase was separated, washed with brine, dried and evaporated to give the title aldehyde (8.0g). M+ 188/90; NMR ($CDCl_3$) delta 10.42 (1H,s), 7.20 (1H,dd), 7.11 (1H,t).

Example CM

3-Ethyl 5-methyl 4-(6-chloro-2-fluoro-3-methoxyphenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Example 24 using appropriate starting materials. M+ 604/6.

Example CN

3-Ethyl 5-methyl 4-(2-chloro-3,5-dimethoxyphenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Example 32 using appropriate starting materials. M+ 616/8.

Example CO

5-Methyl 3-(2,2,2-trichloroethyl) 4-(3-chloro-6-fluoro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

2,2,2-Trichloroethyl 4-(2-(1,3-dihydro-1,3-dioxo-2H-isoindol-2-yl)ethyl)-3-oxobutanoate (2.65g), 3-chloro-6-fluoro-2-(trifluoromethyl)benzaldehyde (1.41g), piperidine (0.2ml) and hexanoic acid (0.2ml) in ethanol (100ml) were stirred at 20° for 24 hours. The solvent was removed by distillation and the residue added to methyl 4-fluoro-3-oxobutanoate (0.765g) and concentrated aqueous ammonia (6.5ml) in acetone (200ml) and the solution stirred at 20° for 16 hours. The solvent was removed by distillation and the residue treated with trifluoroacetic anhydride (1ml) in dry dichloromethane (200ml) at 0° for 15 minutes. The solvent was evaporated and the residue dissolved in ethyl acetate, and then the solution was washed with brine, dried and concentrated. The crude material was purified by chromatography on silica using ethyl acetate/petroleum ether mixtures as eluant, giving the title compound (0.87g). M+ 744/6/8; NMR ($CDCl_3$) delta 6.05 (q,1H), 4.74 (q,2H).

Example CP

Diethyl 4-(3-chloro-6-fluoro-2-(trifluoromethyl)phenyl)-2-((2-(1,3-dihydro-1,3-dioxo-2H-isoindol-2-yl)ethoxy)methyl)-1,4-dihydro -6-(trifluoromethyl)-3,5-pyridinedicarboxylate

Prepared by the method of Example BD.

Example CQ

5-Methyl 3-(2,2,2-trichloroethyl) 4-(2-chlorophenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Example 32 as a yellow gum. M+ 659/661/663; NMR ($CDCl_3$) delta 5.48 (s,1H), 4.71 (q,2H).

Example CR

5-Methyl 3-(2,2,2-trifluoroethyl) 4-(2-chlorophenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Prepared by the method of Example 32. M+ +1 611/13; NMR ($CDCl_3$) delta 5.40 (s,1H), 4.39 (m,2H).

Example CS

2,2,2-trifluoroethyl 4-(2-(1,3-dihydro-1,3-dioxo-2H-isoindol-2-yl)ethoxy)-3-oxobutanoate

2,2,2-trichloroethyl 4-(2-(1,3-dihydro-1,3-dioxo-2H-isoindol-2-yl)ethoxy)-3-oxobutanoate (4.2g) and 2,2,2-trifluoroethanol (25ml) were heated at reflux for 15 hours, then poured into ethyl acetate and water. The organic phase was dried and evaporated, giving the title compound as an oil. M+ +1 374; NMR ($CDCl_3$) delta 3.61 (2H,s), 4.47 (2H,m).

Example CT

5-Methyl 3-(1-methylethyl) 4-(3-chloro-6-fluoro-2-(trifluoromethyl)phenyl)-2-(((1,3-dihydro-1,3-dioxo-2H-isoindol--2-yl)ethylthio)methyl)-6-(fluoromethyl(-1,4-dihydro-3,5-pyridinedicarboxylate

5-Methyl 3-(1-methylethyl) 2-(bromomethyl)-4-(3-chloro-6-fluoro-2-(trifluoromethyl)phenyl)-6-(fluoromethyl)-1,4-dihydro-3,5-pyridinedicarboxylate (5.84g, 10.7mmoles) and 1,3-dihydro-1,3-dioxo-2H-isoindole-2-ethanethiol (2.2g, 10.7mmoles) were stirred in acetonitrile (50ml). Potassium carbonate (1.47g, 10.7mmoles) was added and after 3 hours, the reaction mixture was poured onto saturated sodium chloride. The product was extracted into ethyl acetate, the organic extract washed with saturated sodium chloride, dried ($MgSO_4$) and the solvent was evaporated. Chromatography on silica eluting with ethyl acetate/dichloromethane mixtures gave the title compound (4.4g) as an oil. M/e 673/5 (M+ +1)

Example CU

5-Methyl 3-(1-methylethyl) 4-(3-chloro-6-fluoro-2-(trifluoromethyl)phenyl)-2-(((1,3-dihydro-1,3-dioxo-2H-isoindol-2-yl)ethylsulphonyl)methyl)-6-(fluoromethyl)-1,4-dihydro-3,5-pyridinedicarboxylate

A solution of potassium peroxymonosulphate (Oxone) (1.5g, 4.9mmoles) in water (10ml) was added to the product from Example 4 (1.1g, 1.6mmoles) in ethanol (150ml) and water (75ml). After stirring for 16 hours, the reaction mixture was poured onto brine and extracted with ethyl acetate. The organic extract was washed with water, dried ($Na_2SO_4$) and the solvent was evaporated. Chromatography on silica eluting with dichloromethane/ ethyl acetate gave the title compound (1.1g). M+1+ 705/7; NMR delta ($CDCl_3$) 3.6 (s,3H), 1.3 and 1.05 (d,3H(x2)).

Example CV

3-Ethyl 5-methyl 4-(2-chlorophenyl)-2-(((1,3-dihydro-1,3-dioxo-2H-isoindol-2-yl)ethylthio)methyl)-6-(fluoromethyl)-1,4-dihydro-3,5-pyridinedicarboxylate

Ethyl 3-amino-4-(2-(1,3-dihydro-1,3-dioxo-2H- isoindol-2-yl)ethylthio)-2-butanoate (2.5g, 7.5mmoles), 2-chlorobenzaldehyde (1.1g, 7.8mmoles) and methyl 4-fluoro-3-oxobutanoate (1.05g, 7.8mmoles) in t-butanol (25ml) was heated at 50° for 20 hours. The solvent was evaporated and the residue purified by chromatography on silica eluting with ethyl acetate/petroleum ether (60-80°) mixtures to give the title compound (2.3g) as an oil. M+ 572/4.

Example CW

3-Ethyl 5-methyl 4-(3-chloro-6-fluoro-2-(trifluoromethyl)phenyl)-2-((2-(2-(1,3-dihydro-1,3-dioxo-2H-isoindol-2-yl)ethoxy)methyl)-6-(fluoromethyl)-1,4-dihydro-3,5-pyridinedicarboxylate

Ethyl 4-(2-(2-(1,3-dihydro-1,3-dioxo-2H-isoindol-2-yl)ethoxy)ethoxy)-3-oxobutanoate (2.7g, 7.4mmoles) and ammonium acetate (0.6g, 8.1mmoles) in t-butanol (50ml) were heated at 60° for 4 hours. 3-Chloro-6-fluoro-2-(trifluoromethyl)benzaldehyde (1.6g, 7.4mmoles) and methyl 4-fluoro-3-oxobutanoate (0.95g,

7.4mmoles) were added and the heating at 60° continued for 6 days. The solvent was evaporating and the residue, in dichloromethane (50ml), was treated with trifluoroacetic anhydride (0.5ml). After 10 minutes, methanol (5ml) was added and the solvent was evaporated. The residue in dichloromethane was washed with water, dried ($Na_2SO_4$) and the solvent evaporated. Purification by HPLC on silica eluting with ethyl acetate/petroleum ether (60-80°) mixtures gave the title compound (1g). M+ + 687/9; NMR ($CDCl_3$) delta 6.0 (q,1H), 5.54 (octet,2H).

Example CX

3-Ethyl 5-methyl 4-(2-chlorophenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Ethyl 4-(2-(1,3-dihydro-1,3-dioxo-2H-isoindol-2-yl)ethoxy)-3-oxobutanoate (11g, 33mmoles) and ammonium acetate (2.8g, 36mmoles) in t-butanol (250ml) were heated at 60° for 2 hours. 2-Chlorobenzaldehyde (4.62g, 33mmoles) and methyl 4-fluoro-3-oxo-butanoate (4.42g, 33mmoles) were added and the heating at 60° continued for 4 days. The solvent was evaporated and the residue, in dichloromethane (250ml), was treated with trifluoroacetic anhydride (2.0ml). After 0.5 hours, methanol (20ml) was added and the solvent was evaporated. The residue in dichloromethane was washed with water, dried ($MgSO_4$) and the solvent evaporated. The residue was purified by HPLC on silica eluting with ethyl acetate/petroleum ether (60-80°) mixtures to give the title compound (14g). M+1+ 557/9; NMR ($CDCl_3$) delta 4.77 (q,2H), 3.6 (s,3H).

Example CY

3-Ethyl 5-methyl 4-(3-chloro-2-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Ethyl 4-(2-(1,3-dihydro-1,3-dioxo-2H-isoindol-2-yl)ethoxy-3-oxobutanoate (3.55g, 11mmoles) and ammonium acetate (0.86g, 11mmoles) in t-butanol (70ml) were heated at 50° for 4 hours. 3-Chloro-2-(trifluoromethyl)benzaldehyde (2.3g, 11mmoles) and methyl 4-fluoro-3-oxobutanoate (1.55g, 11mmoles) were added and the heating continued at 50° for 5 days. The solvent was evaporated and the residue purified by chromatography on silica eluting with ethyl acetate/petroleum ether (60-80°) mixtures to give the title compound (3.7g). M+1+ 625.7; NMR ($CDCl_3$) delta 5.73 (q,1H), 3.44 (s,3H).

Example CZ

The following compound was prepared by the method of Example CT using appropriate starting materials: 3-Ethyl 5-methyl 4-(3-chloro-6-fluoro-2-(trifluoromethyl)phenyl)-2-((2-(1,3-dihydro-1,3-dioxo-2H-isoindol-2-yl)ethylthio)methyl)-6-(fluoromethyl)-1,4-dihydro-3,5-pyridinedicarboxylate, mp 100-6°.

Example DA

The following compound was prepared by the method of Example 15 using appropriate starting materials: 5-Methyl 3-(1-methylethyl) 4-(3-chloro-6-fluoro-2-(trifluoromethyl)phenyl)-2-((1,3-dihydro-1,3-dioxo-2H-isoindol-2-yloxy)methyl)-6-(fluoromethyl)-1,4-dihydro-3,5-pyridinedicarboxylate

Example DB

The following compounds were prepared by the method of Example CX using appropriate starting materials: a) 3-Ethyl 5-methyl 4-(2-chloro-3-(trifluoromethyl)phenyl)-2-((2-(1,3-dihydro-1,3-dioxo-2H-isoindol-2-yl)ethoxy)methyl)-6-(fluoromethyl)-1,4-dihydro-3,5-pyridinedicarboxylate. M+1+ 625/7; NMR ($CDCl_3$) delta 7.6 (d,1H), 7.5 (d,1H), 7.3 (t,1H), 3.6 (s,3H). b) 3-Ethyl 5-methyl 4-(3-cyanophenyl)-2-((2-(1,3-dihydro-1,3-dioxo-2H-isoindol-2-yl)ethoxy)methyl)-6-(fluoromethyl)-1,4-dihydro-3,5-pyridinedicarboxylate

Example DC

Prepared by the method of Example 14.

5-Methyl 3-(1-methylethyl) 4-(3-chloro-6-fluoro-2-(trifluoromethyl)phenyl)-2-((4-(2-(1,3-dihydro-1,3-dioxo2H-isoindol-2-yl)ethyl)-1H-imidazol-1-yl)methyl)-6-(fluoromethyl)-1,4-dihydro-3,5-pyridinedicarboxylate, mp 80-83°.

Example DD

| | % w/w | Range % w/w |
|---|---|---|
| Compound of formula I | 5 | 1-20 |
| Microcrystalline cellulose | 50 | 10-80 |
| Spray dried lactose | 37.75 | 10-80 |
| Magnesium stearate | 1 | 0.25-2 |
| Colloidal silicon dioxide | 0.25 | 0.1-1 |
| Cross linked sodium carboxy methyl cellulose | 3 | 1-5 |
| Hydroxypropylmethylcellulose (coating) | 3 | 1-5 |

This formulation is made up as a direct compression tablet, or without compression or coating, may be filled into a gelatine capsule.

Example DE

| | % w/w | Range % w/w |
|---|---|---|
| Compound of formula I | 5 | 1-20 |
| Microcrystalline cellulose | 50 | 10-80 |
| Lactose | 35.75 | 10-80 |
| Polyvinylpyrrolidone | 2 | 1-5 |
| Magnesium stearate | 1 | 0.25-2 |
| Colloidal silicon dioxide | 0.25 | 0.1-1 |
| Cross linked sodium carboxy methyl cellulose | 3 | 1-5 |
| Hydroxypropyl methyl cellulose (coating) | 3 | 1-5 |

This formulation is made up as a granulate and then compressed into a tablet. Alternatively the granules may be filled into a gelatine capsule.

## Claims

1. A compound of formula I,

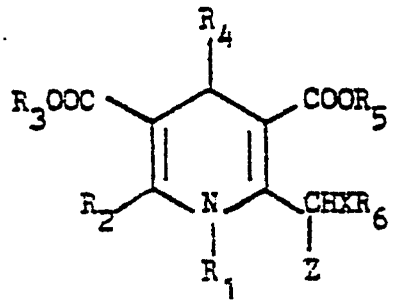

I

in which $R_2$ is alkyl C 1 to 6 optionally substituted by one or more fluorine atoms,
$R_3$ is alkyl C 1 to 6,
$R_4$ is phenyl, naphthyl or an unsaturated sulphur containing heterocyclic ring, each of which is optionally substituted by one or more groups selected from $-CF_3$, -CN, $-NO_2$, $NH_2$, halogen, alkyl C 1 to 6, alkoxy C 1 to 6 or $C_2F_5$,
$R_5$ is alkyl C 1 to 10 optionally interrupted by $-CONR_8-$ and optionally substituted by one or more halogen atoms or by a group $-YR_7$
Y is -O-, $-S(O)_m$. $-NR_9$ or a bond,
$R_7$ is -COOH or a group $R_{10}$,
X is -O-, -NR, $-S(O)_{m_1}$, or a bond,
Z is hydrogen or together with R forms a bond,
R is hydrogen, alkyl C 1 to 6 or together with Z forms a bond,
$R_6$ is a group $R_{11}$,
$R_{10}$ and $R_{11}$, which may be the same or different, represent a 5 or 6 membered unsaturated nitrogen containing heterocyclic ring which is optionally fused to a phenyl ring, $R_{10}$ and $R_{11}$ optionally being substituted by one or more groups selected from oxo, hydroxy, alkyl C 1 to 6, $-(CH_2)_{n_1}NH_2$, alkloxy C 1 to 6, phenyl or -COOH,
and when X is -NR- or $-S(O)_{m_1}-$, $R_6$ may in addition be hydrogen or a group $-CH_2CH_2NH_2$, $-CH_2CH_2R_{11}$ or $NHC(=NH)NH_2$,
when X is -O-, $R_6$ may also be a group $-NH_2$, $-C(=O)\ (CH_2)_nNH_2$, $-CH_2CH_2NR_{14}R_{15}$ or $CH_2CH_2OCH_2CH_2NR_{14}R_{15}$,
when X is -NR-, $-S(O)_{m_1}-$, or a bond, $R_6$ may also be hydrogen or a group $-C(=NR_{12})NHR_{13}$,
when Y is -O-, $R_{10}$ may in addition be phenyl,
and when $-CHZXR_6$ is $-CH_2OCH_2CH_2NH_2$ then $R_5$ may be thietanyl,
$R_1$, $R_8$, $R_9$, $R_{13}$ and $R_{14}$, which may be the same or different, are each hydrogen or alkyl C 1 to 6,
$R_{12}$ is hydrogen, alkylcarbonyl C2 to 7, nitrile or hydroxy,
$R_{15}$ is hydrogen or a group $R_{11}$,
m and $m_1$, which may be the same or different, are each 0, 1 or 2,
n is 0 or 1,
$n_1$ is an integer from 0 to 6
and pharmaceutically acceptable salts, esters, amides and protected derivatives thereof,
with the provisos that:-

i) when $R_2$ is unsubstituted alkyl then

a) the group $-CHZXR_6$ is not methyl, $-CH_2OCONH_2$, $-CH_2OCH_2CH_2NH_2$, $-CH=NCH_2CH_2NH_2$ or the groups of formulae XXV and XXVI,

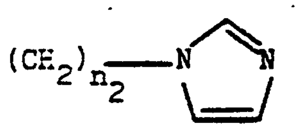

XXV

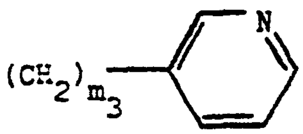

XXVI

in which $n_2$ is 1 or 2, and
$m_3$ is 0, 1 or 2, and
b) $R_4$ is not a group of formula XXVII,

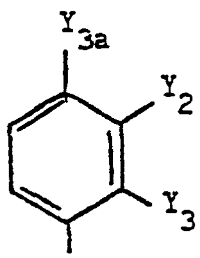

XXVII

in which $Y_2$ is hydrogen, -Cl or $-CF_3$, and $Y_3$ and $Y_{3a}$, which may be same or different, are each hydrogen or -Cl,

ii) when $-CHZXR_6$ is methyl and

A. $R_5$ is unsubstituted alkyl, then $R_4$ is not a monosubstituted phenyl group, 2-fluoro-6-(trifluoromethyl) phenyl, a group of formula XXVIII,

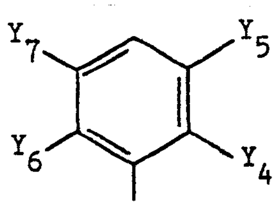

XXVIII

in which, either
a) $Y_4$ is -Cl, methyl or methoxy, $Y_5$ is -Cl, $-NO_2$, $-CF_3$, methyl or methoxy, and
$Y_6$ and $Y_7$ are both hydrogen or
b) $Y_5$ is $-NO_2$, $Y_6$ is -Cl or methyl and $Y_4$ and $Y_7$ are both hydrogen;
or a 2,3,6-trisubstituted phenyl group other than 6-chloro-2-fluoro-3-methoxyphenyl; and
B. $R_5$ is methyl then $R_4$ is not a pentafluorophenyl group,

iii) when $R_2$ is $-CH_2F$ and

A. $R_5$ is unsubstituted alkyl, other than cycloalkyl, and the group $-CHZXR_6$ is $-CH_2OCH_2CH_2NR_{14}R_{15}$ then $R_4$ is not a group of formula XXIX,

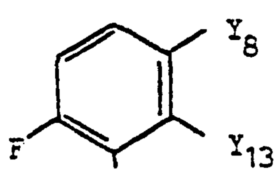

XXIX

in which,
a) $Y_{13}$ is $-CF_3$ and $Y_8$ is -Cl, or

b) $Y_{13}$ is -F and $Y_8$ is either -F or $-NO_2$, and
B. $R_5$ is alkyl substituted by halogen then $R_4$ is not 2,3,6-trisubstituted phenyl group, and
C. $R_5$ is 1-methylethyl then
a) $R_4$ is not a group of formula XXX,

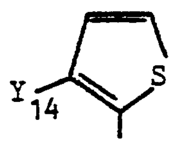

XXX

in which $Y_{14}$ is nitro. nitrile or methyl, and
b) $R_4$ is not a group of formula XXXI.

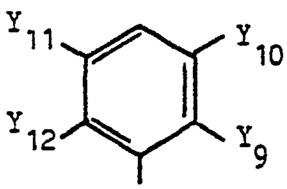

XXXI

in which $Y_9$ and $Y_{11}$, which may be the same or different, are each -Cl or -F,
$Y_{10}$ is -Cl or $-CF_3$, and
$Y_{12}$ is $-CF_3$ or hydrogen;
iv) when $R_5$ is an uninterrupted alkyl group and Y is -O-, then $R_7$ is not a monosubstituted pyrazol-3-yl group, a 2,5-disubstituted pyrimidin-4-yl group,
or protected derivatives thereof.

2. A compound according to Claim 1, wherein $R_2$ is fluoromethyl.

3. A compound according to Claim 1, wherein $R_4$ is a phenyl group substituted in one or more of the 2-, 3- or 6- positions.

4. A compound according to Claim 1, wherein $R_5$ is a group $-YR_{10}$ and $-CHZXR_6$ is methyl.

5. A compound according to Claim 1, wherein $R_5$ is alkyl C 1 to 6 and $R_6$ is $-CH_2CH_2NH_2$ or a group $R_{11}$.

. A compound according to Claim 1, wherein the group $-CHZXR_6$ is selected from (2-aminoethylthio)methyl; (2-aminoethylsulphonyl)methyl; (aminoiminomethyl) thiomethyl; 1H-pyrrol-1-ylmethyl; (4H-1,2,4-triazol-4-yl) methyl; (5-3-ethoxycarbonyl-3,4-dihydro-4-oxopyrimidin-2-ylthio)methyl; (3,4-dihydro-4-oxopyrimidin-2-ylthio)methyl; (pyridin-2-ylthio)methyl; (pyridin-3-yloxy)methyl; methyl; (2-aminoethoxy)methyl and (2-((5-amino-1H-1,2,4-triazol-3-yl)amino)ethoxy)methyl.

7. 3-Methyl 5-(1-methylethyl) 4-(3-chloro-6-fluoro-2-(trifluoromethyl)phenyl)-2-(fluoromethyl)-1,4-dihydro-6-(pyridin-3-yloxymethyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-1,2,4-triazol-1-yl)methyl)-3,5-pyridinedicarboxylate,
5-Methyl 3-(1-methylethyl) 2-((2-aminoethyl) aminoethyl)-4-(3-chloro-6-fluoro-2-(trifluoromethyl) phenyl)-6-(fluoromethyl)-1,4-dihydro-3,5-pyridinedicarboxylate,
5-Methyl 3-(1-methylethyl) 2-((2-aminoethyl) thiomethyl)-4-(3-chloro-6-fluoro-2-(trifluoromethyl)phenyl)6-(fluoromethyl)-1,4-dihydro-3,5-pyridinedicarboxylate,
5-Methyl-3-(1-methylethyl)-2-((2-amino-4,6-dihydroxypyrimidin-5-yl)methyl)-4-(3-chloro-6-fluoro-2-(trifluoromethyl)phenyl-6-(fluoromethyl)-1,4-dihydro-3,5-pyridinedicarboxylate,
3-Methyl 5-(1-methylethyl) 4-(3-chloro-6-fluoro-2- (trifluoromethyl)phenyl)-2-(fluoromethyl)-1,4-dihydro-6-((1H-pyrazol-1-yl)methyl)-3,5-pyridinedicarboxylate,
5-Methyl 3-(1-methylethyl) 2-(((aminocarbonyl)oxy)methyl)-4-(3-chloro-6-fluoro-2-(trifluoromethyl)phenyl)-6-(fluoromethyl)-1,4-dihydro-3,5-pyridinedicarboxylate,
3-Methyl 5-(methylethyl) 4-(3-chloro-6-fluoro-2--(trifluoromethyl)phenyl)-2-(fluoromethyl)-1,4-dihydro--6-(pyrimidin-2-yloxymethyl)-3,5-pyridinedicarboxylate,
5-Methyl 3-(1-methylethyl) 2-((aminoacetyloxy)methyl)-4-(3-chloro-6-fluoro-2-(trifluoromethyl)phenyl)-6-(fluoromethyl)-1,4-dihydro-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-tetrazol-5-yl)methyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-(1H-pyrrol-1-ylmethyl)-3,5-pyridinedicarboxylate.
5-Methyl 3-(1-methylethyl) 2-(((aminoiminomethyl)hydrazono)methyl)-4-(3-chloro-6-fluoro-2-(trifluoromethyl)phenyl)-6-(fluoromethyl)-1,4-dihydro-3.5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-(1H-1,2,3-triazol-1-yl)methyl-3,5-pyridinedicarboxylate,
5-Methyl 3-(1-methylethyl) 4-(3-chloro-6-fluoro-2- (trifluoromethyl)phenyl)-2-((5-ethoxycarbonyl-3,4-dihydro-4-oxopyrimidin-2-ylthio)methyl)-6-(fluoromethyl)-1,4-dihydro-3,5-pyridinedicarboxylate,
3-Ethyl 5-methyl 2-((1H-benzotriazol-1-yloxy) methyl)-4-(2,3-dichlorophenyl)-6-(fluoromethyl)-1,4-dihydro-3,5-pyridinedicarboxylate,
3-Ethyl 5-methyl 2-((aminoiminomethyl)thiomethyl)-4-(2,3-dichlorophenyl)-6-(fluoromethyl)-1,4-dihydro-3,5-pyridinedicarboxylate.
5-Ethyl 3-methyl 4-(2-chlorophenyl)-2-(fluoromethyl)-1,4-dihydro-6-((4H-1,2,4-triazol-4-yl)methyl)-3,5-pyridinedicarboxylate,
5-Methyl 3-(1-methylethyl)2-[2-amino-2-(hydroximino) ethyl]-4-(3-chloro-6-fluoro-2-(trifluoromethyl)phenyl)-6-(fluoromethyl)-1,4-dihydro-3,5-pyridinedicarboxylate,
3-Methyl 5-(6-(1-methyl-3-phenylpyrazol-5-yloxy) hexyl 4-(3-chloro-6-fluoro-2-(trifluoromethyl)phenyl)-2-(fluoromethyl)-1,4-dihydro-6-methyl-3,5-pyridinedicarboxylate,
3-Methyl 5-(6-phenoxyhexyl) 4-(3-chloro-6-fluoro-2-(trifluoromethyl)phenyl)-2-(fluoromethyl)-1,4-dihydro-6-methyl-3,5-pyridinedicarboxylate,
3-Methyl 5(-6-(6-methyl-2-phenylpyrimidin-4-ylthio) hexyl 4-(3-chloro-6-fluoro-2-(trifluoromethyl)phenyl)-2-(fluoromethyl)-1,4-dihydro-6-methyl-3,5- pyridinedicarboxylate,
3-Methyl 5-(6-(1-methyl-5-phenylpyrazol-3-yloxy)hexyl) 4-(3-chloro-6-fluoro-2-(trifluoromethyl)phenyl)-2-(fluoromethyl)-1,4-dihydro-6-methyl-3,5-pyridinedicarboxylate,
5-(7-Carboxyheptyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(8-Methoxy-8-oxooctyl) 3-methyl 4-(3-chloro-6-fluoro--2-(trifluoromethyl)phenyl)-2-(fluoromethyl)-1,4-dihydro-6-methyl-3,5-pyridinedicarboxylate,
3-Methyl 5-(2-oxo-2-((3-(5-phenylpyrazol-3-yloxy) propyl)amino)ethyl) 4-(3-chloro-6-fluoro-2-(trifluoromethyl)phenyl)-2-(fluoromethyl)-1,4-dihydro-6-methyl-3,5-pyridindedicarboxylate,
3-Ethyl 5-methyl 2-(2-(2-aminoethoxy)ethoxymethyl)-4-(3-chloro-6-fluoro-2-(trifluoromethyl)phenyl)-6-(fluoromethyl)-1,4-dihydro-3,5-pyridinedicarboxylate,
3-Ethyl 5-methyl 2-(2-aminoethoxymethyl)-4-(2-chlorophenyl)-6-(fluoromethyl)-1,4-dihydro-3,5-pyridinedicarboxylate,
3-Ethyl 5-methyl 2-((2-aminoethoxy)methyl)-4-(3-chloro-2-(trifluoromethyl)phenyl)-6-(fluoromethyl)-1,4-dihydro-3,5-pyridinedicarboxylate,
3-Methyl 5-(1-methylethyl) 2-(fluoromethyl)-4- (2-fluoro-6-nitrophenyl)-1,4-dihydro-6-methyl-3,5-pyridinedicarboxylate,
3-Methyl 5-(1-methylethyl) 4-(2-chloro-6-methylphenyl)-2-(fluoromethyl)-1,4-dihydro-6-methyl-3,5-pyridinedicarboxylate,
3-Cyclobutyl 5-methyl 2-((2-aminoethoxy)methyl)-4-(3-chloro-6-fluoro-2-(trifluoromethyl)phenyl)-6-(fluoromethyl)-1,4-dihydro 3,5-pyridinedicarboxylate,
5-Methyl 3-(3-thietanyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-((2-aminoethoxy)methyl)-6-(fluoromethyl)-4-(pentafluorophenyl)-1,4-dihydro-3,5-pyridinedicarboxylate,
3-Ethyl 5-methyl 2-((2-aminoethoxy)methyl)-6-(fluoromethyl)-4-(2,3,5,6-tetrafluorophenyl)-1,4-dihydro-3,5-pyridinedicarboxylate.
3-Ethyl 5-methyl 2-((2-aminoethoxy)methyl)-4-(2,3-dichlorophenyl)-6-(fluoromethyl)-1,4-dihydro-3,5-pyridinedicarboxylate,
3-Methyl 5-(1-methylethyl) 2-(fluoromethyl)-1,4-dihydro-6-methyl-4-(2,3,5,6-tetrafluorophenyl)-3,5-pyridinedicarboxylate,
3-Methyl 5-(1-methylethyl) 4-(2-fluoro-5-methoxyphenyl)-2-(fluoromethyl)-1,4-dihydro-6-methyl-3,5- pyridinedicarboxylate,
5-(6-(2,6-Dimethylpyrimidin-4-yloxy(hexyl) 3-methyl 4-(3-chloro-6-fluoro-2(trifluoromethyl)phenyl)-2-(fluoromethyl)-1,4-dihydro-6-methyl-3,5-pyridinedicarboxylate,
5-Methyl 3-(2,2,2-trichloroethyl) 2-((2-aminoethoxy) methyl)-4-(3-chloro-6-fluoro-2-(trifluoromethyl)phenyl)-6-(fluoromethyl)-1,4-dihydro-3,5-pyridinedicarboxylate,
3-Ethyl 5-methyl 2-((2-((5-amino-1H-1,2,4-triazol-3-yl)amino)ethoxy)methyl)-6-(fluoromethyl)-1,4-dihydro-4-(2,3,5,6-tetrafluorophenyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-imidazol-1-yl)methyl)-3,5-pyridinedicarboxylate,
5-Methyl 3-(1-methylethyl) 2-((6-amino-1,2-dihydro-2-oxopyrid-1-yl)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-((2-aminopyrid-3-yloxy) methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-(((1H-5-amino-1,2,4-triazol-3-yl)thio)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-((5-amino-1H-1,2,4- triazol-1-yl)methyl)-4-(3-chloro-6-fluoro-2-(trifluoromethyl)phenyl)-6-(fluoromethyl)-1,4-dihydro-3,5-pyridinedicarboxylate,

5-methyl 3-(1-methylethyl) 2-((3-amino-1H-1,2,4-triazol-1-yl)methyl)-4-(3-chloro-6-fluoro-2-(trifluoromethyl)phenyl)-6-(fluoromethyl)-1,4-dihydro-3,5-pyridinedicarboxylate,

5-methyl 3-(1-methylethyl) 2-((3-amino-4H-1,2,4-triazol-4-yl)methyl)-4-(3-chloro-6-fluoro-2-(trifluoromethyl)phenyl)-6-(fluoromethyl)-1,4-dihydro3,5-pyridinedicarboxylate.

5-Methyl 3-(1-methylethyl) 4-(3-chloro-6-fluoro-2-(trifluoromethyl)phenyl)-6-(fluoromethyl)-1,4-dihydro-2-((1-pyridinium)methyl)-3,5-pyridinedicarboxylate bromide,

5-Methyl 3-(1-methylethyl) 2-((aminoiminomethyl)-thiomethyl)-4-(3-chloro-6-fluoro-2-(trifluoromethyl)phenyl)-6-fluoromethyl-1,4-dihydro-3,5-pyridinedicarboxylate,

5-Methyl 3-(1-methylethyl) 2-((2-aminoethylsulphonyl) methyl)-4-(3-chloro-6-fluoro-2-(trifluoromethyl)phenyl)-6-(fluoromethyl)-1,4-dihydro-3,5-pyridinedicarboxylate,

3-Ethyl 5-methyl 2-((2-aminoethylthio)methyl)-4-(3-chloro-6-fluoro-2-(trifluoromethyl)phenyl)-6-(fluoromethyl)-1,4-dihydro-3,5-pyridinedicarboxylate.

3-Ethyl 5-methyl 2-((2-aminoethylthio)methyl)-4-(2-chlorophenyl)-6-(fluoromethyl)-1,4-dihydro-3,5- pyridinedicarboxylate,

5-Methyl 3-(methylethyl) 2-((aminooxy)methyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 2-((4-(2-aminoethyl)-1H-imidazol-1-yl)methyl)-4-(3-chloro-6-fluoro-2-(trifluoromethyl)phenyl)-6-(fluoromethyl)-1,4-dihydro-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-((2-(1H-imidazol-4-yl)ethylamino)methyl)-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-((1H-1,2,4-triazol-3-ylthio)methyl)-3,5-pyridinedicarboxylate,

5-Methyl 3-(1-methylethyl) 4-(3-chloro-6-fluoro-2-(trifluoromethyl)phenyl)-2-(4,6-diaminopyrimidin-2-ylthio) methyl)-6-(fluoromethyl)-1,4-dihydro-3,5-pyridinedicarboxylate,

3-Ethyl 5-methyl 4-(2-chlorophenyl)-2-((5-ethoxycarbonyl-3,4-dihydro-4-oxopyrimidin--2-ylthio)methyl)-6-(fluoromethyl)-1,4-dihydro-3,5-pyridinedicarboxylate,

3-Ethyl 5-methyl 4-(2-chlorophenyl)-2-((3,4-dihydro-4-oxopyrimidin-2-ylthio)methyl)-6-(fluoromethyl)-1,4-dihydro-3,5-pyridinedicarboxylate,

5-Ethyl 3-methyl 4-(2-chlorophenyl)-2-(fluoromethyl)-1,4-dihydro-6-(pyrimidin-2-ylthio)methyl)-3,5-pyridinedicarboxylate,

5-Ethyl 3-methyl 4-(2-chlorophenyl)-2-(fluoromethyl)-1,4-dihydro-6-((pyridin-2-ylthio)methyl)-3,5-pyridinedicarboxylate,

3-Ethyl 5-methyl 2-((1H-benzimidazol-2-ylthio)methyl)-4-(2-chlorophenyl)-6-(fluoromethyl)-1,4-dihydro-3,5-pyridinedicarboxylate,

5-Ethyl 3-methyl 4-(2-chlorophenyl)-2-(fluoromethyl)-1,4-dihydro-6-((1H-imidazol-2-ylthio)methyl)-3,5-pyridinedicarboxylate,

3-Ethyl 5-methyl 4-(2,3-dichlorophenyl)-2-((3,4-dihydro-4-oxopyrimidin-2-ylthio)methyl)-6-(fluoromethyl)-1,4-dihydro-3,5-pyridinedicarboxylate,

3-Ethyl 5-methyl 4-(2,3-dichlorophenyl)-2-((5-(ethoxycarbonyl)-3,4-dihydro-4-oxopyrimidin-2-ylthio)methyl)-6-(fluoromethyl)-1,4-dihydro-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-((5-methoxy-1H-benzimidazol-2-ylthio)methyl)-3,5-pyridinedicarboxylate.

3-Ethyl 5-methyl 2-((6-carboxy-3,4-dihydro-4-oxopyrimidin-2-ylthio)methyl)-4-(2-chlorophenyl)-6-(fluoro methyl)-1,4-dihydro-3,5-pyridinedicarboxylate,

3-Ethyl 5-methyl 4-(2,3-dichlorophenyl)-2-((3,4-dihydro-6-hydroxy-4-oxopyrimidin-2-ylthio)methyl)-6-(fluoromethyl)-1,4-dihydro-3,5-pyridinedicarboxylate,

3-Ethyl 5-methyl 4-(2,3-dichlorophenyl)-2-((3,4-dihydro-6-methyl-4-oxopyrimidin-2-ylthio)methyl)-6-(fluoromethyl)-1,4-dihydropyridine-3,5-dicarboxylate,

3-Ethyl 5-methyl 2-((5-(ethoxycarbonyl)-3,4-dihydro-4-oxopyrimidin-2-ylthio)methyl)-6-(fluoromethyl)-1,4-dihydro-4-(2-trifluoromethyl)phenyl)-3,5-pyridinedicarboxylate,

3-Ethyl 5-methyl 2-((3,4-dihydro-4-oxopyrimidin-2-yl-thio)methyl)-6-(fluoromethyl)-1,4-dihydro-4-(2-trifluoromethyl)phenyl)-3,5-pyridinedicarboxylate,

3-Ethyl 5-methyl 2-((4-amino-5-(ethoxycarbonyl)-pyrimidin-2-ylthio)methyl)-4-(2-chlorophenyl)-6-(fluoromethyl)-1,4-dihydro-3,5-pyridinedicarboxylate,

5-Ethyl-3-methyl-4-(2,3-dichlorophenyl)-2-(fluoromethyl)-1,4-dihydro-6-(pyrid-2-ylthiomethyl)-3,5-pyridinedicarboxylate.

3-Ethyl 5-methyl 4-(2,3-dichlorophenyl)-2-((((cynaoimino)methylamino)methylthio)methyl)-6-(fluoromethyl)-1,4-dihydro-3,5-pyridinedicarboxylate.

3-Ethyl 5-methyl 2-(((acetylimino)aminomethylthio) methyl)-4-(2,3-dichlorophenyl)-6-(fluoromethyl)-1,4-dihydro-3,5-pyridinedicarboxylate.

5-Methyl 3-(1-methylethyl) 2-((2-aminocarbonyloxy) methyl)-4-(2,3-dichlorophenyl)-6-(fluoromethyl)-1,4-dihydro-3,5-pyridinedicarboxylate.

5-Ethyl 3-methyl 4-(2-chlorophenyl)-2-(fluoromethyl)-1,4-dihydro-6-((1H-pyrrolo-1-yl)methyl)-3,5-pyridinedicarboxylate.

3-Methyl 5-(1-methylethyl) 4-(3-chloro-6-fluoro-2-(trifluoromethyl)phenyl)-2-(fluoromethyl)-1,4-dihydro-6-((4H-1,2,4-triazol-4-yl)methyl)-3,5-pyridinedicarboxylate,
3-Methyl 5-(6-(5-phenylpyrazol-3-ylamino)hexyl) 4-(3-chloro-6-fluoro-2-(trifluoromethyl)phenyl)-2-(fluoromethyl)-1,4-dihydro-6-methyl-3,5-pyridinedicarboxylate,
3-Methyl 5-(6-(pyrid-3-yloxy)hexyl) 4-(3-chloro-6-fluoro-2-(trifluoromethyl)-phenyl)-2-(fluoromethyl)-1,4-dihydro-6-methyl-3,5-pyridinedicarboxylate,
5-(6-(1H-Imidazol-1-yl)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(6-(4,6-Dimethylpyrimidin-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(6-(1H-Benzimidazol-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-((8-Ethoxy-8-oxo)octyl) 3-methyl 4-(3-chloro-6-fluoro-2-(trifluoromethyl)phenyl)-2-(fluoromethyl)-1,4-
5-((8-Amino-8-oxo)octyl) 3-methyl 4-(3-chloro-6-fluoro-2-(trifluoromethyl)phenyl)-2-(fluoromethyl)-1,4-dihydro-6-methyl-3,5-pyridinedicarboxylate,
3-Ethyl 5-methyl 2-((2-aminoethoxy)methyl)-6-(fluoromethyl)-1,4-dihydro-4-(5-nitrothien-2-yl)-3,5-pyridinedicarboxylate,
3-Ethyl 5-methyl 2-((2-aminoethoxy)methyl)-4-(5-cyanothien-2-yl)-6-(fluoromethyl)-1,4-dihydro-3,5-pyridinedicarboxylate,
3-Ethyl 5-methyl 2-((2-aminoethoxy)methyl)-4-(3-cyanothien-2-yl)-6-(fluoromethyl)-1,4-dihydro-3,5-pyridinedicarboxylate,
3-Ethyl 5-methyl 2-((2-aminoethoxy(methyl)-6-(fluoromethyl)-4-(3-methylthien-2-yl)-1,4-dihydro-3,5-pyridinedicarboxylate,
3-Ethyl 5-methyl 2-((2-(2-aminoethoxy)ethoxy)methyl)-4-(2-chlorophenyl)-6-(fluoromethyl)-1,4-dihydro-3,5-pyridinedicarboxylate,
Diethyl 2-((2-aminoethoxy)methyl)-4-(2-chlorophenyl)-1,4 dihydro-6-(trifluoromethyl)-3,5-pyridinedicarboxylate,
3-Ethyl 5-methyl 4-(3-amino-2,6-difluorophenyl)-2-((2-aminoethoxy)methyl)-6-(fluoromethyl)-1,4-dihydro-3,5-pyridinedicarboxylate,
5-Methyl 3-(6-((6-methyl-2-phenylpyrimidin-4-yl)oxy)hexyl) 2-((2-aminoethoxy)methyl)-4-(2-chlorophenyl)-6-(fluoromethyl)-1,4-dihydro-3,5-pyridinedicarboxylate,
5-Methyl 3-(6-((6-methyl-2-phenylpyrimidin-4-yl)thio)hexyl) 2-((2-aminoethoxy)methyl)-4-(2-chlorophenyl)-6-(fluoromethyl)-1,4-dihydro-3,5-pyridinedicarboxylate,
3-(6-((4,6-Dimethylpyrimidin-2-yl)thio)hexyl) 5-methyl 2-((2-aminoethoxy)methyl)-4-(2-chlorophenyl)-6-(fluoromethyl)-1,4-dihydro-3,5-pyridinedicarboxylate,
3-Ethyl 5-methyl 2-((2-aminoethoxy)methyl)-4-(2-chloro-3,5-dimethoxyphenyl)-6-(fluoromethyl)-1,4-dihydro-3,5-pyridinedicarboxylate,
3-Ethyl 5-methyl 2-((2-aminoethoxy)methyl)-4-(2-chloro-3-(trifluoromethyl)phenyl)-6-(fluoromethyl)-1,4-dihydro-3,5-pyridinedicarboxylate,
3-Ethyl 5-methyl 2-((2-aminoethoxy)methyl)-4-(3-cyanophenyl)-6-(fluoromethyl)-1,4-dihydro-3,5-pyridinedicarboxylate,
3-Ethyl 5-methyl 2-((2-aminoethoxy)methyl)-4-(2,5-difluoro-6-(trifluoromethyl)phenyl)-6-(fluoromethyl)-1,4-dihydro-3,5-pyridinedicarboxylate,
3-Ethyl 5-methyl 2-((2-aminoethoxy)methyl)-4-(2- fluoro-5-methoxyphenyl)-6-(fluoromethyl)-1,4-dihydro-3,5-pyridinedicarboxylate,
5-Methyl 3-((6-phenoxy)hexyl) 2-((2-aminoethoxy)methyl)-4-(2-chlorophenyl)-6-(fluoromethyl)-1,4-dihydro-3,5-pyridinedicarboxylate,
3-Ethyl 5-methyl 2-((2-aminoethoxy)methyl)-4-(6-chloro-2-fluoro-3-methoxyphenyl)-6-(fluoromethyl)-1,4-dihydro-3,5-pyridinedicarboxylate,
5-Methyl 3-(2,2,2-trichloroethyl) 2-((2-aminoethoxy)methyl)-4-(2-chlorophenyl)-6-(fluoromethyl)-1,4-dihydro-3,5-pyridinedicarboxylate,
5-Methyl 3-(2,2,2-trifluoroethyl) 2-((2-aminoethoxy)methyl)-4-(2-chlorophenyl)-6-(fluoromethyl)-1,4-dihydro-3,5-pyridinedicarboxylate,
3-Methyl 5-(1-methylethyl) 4-(3-chloro-2-fluorophenyl)-2-(fluoromethyl)-1,4-dihydro-6-methyl-3,5-pyridinedicarboxylate,
3-Methyl 5-(1-methylethyl) 4-(2,6-difluorophenyl)-2-(fluoromethyl)-1,4-dihydro-6-methyl-3,5-pyridinedicarboxylate,
3-Methyl 5-(1-methylethyl) 2-(fluoromethyl)-1,4-dihydro-6-methyl-4-(3-nitro-2-(trifluoromethyl)phenyl)-3,5-pyridinedicarboxylate,
3-Methyl 5-(1-methylethyl) 4-(2-fluoro-3-(trifluoromethyl)phenyl)-2-(fluoromethyl)-1,4-dihydro-6- methyl-3,5-pyridinedicarboxylate.
3-Methyl 5-(1-methylethyl) 4-(3-chloro-2-(trifluoromethyl)phenyl)-2-(fluoromethyl)-1,4-dihydro-6-methyl-3,5-pyridinedicarboxylate,
3-Methyl 5-(1-methylethyl) 4-(3-chloro-2-nitrophenyl)--2-(fluoromethyl)-1,4-dihydro-6-methyl-3,5-pyridinedicarboxylate,
3-Methyl 5-(1-methylethyl) 4-(2,3-difluorophenyl)-2-(fluoromethyl)-1,4-dihydro-6-methyl-3,5-pyridinedicarboxylate.
3-Methyl 5-(1-methylethyl) 2-(fluoromethyl)-1,4-dihydro-4-(5-methoxy-2-nitrophenyl)-6-methyl-3,5-pyridinedicarboxylate,
3-Methyl 5-(1-methylethyl) 2-fluoromethyl-4-(2-fluoro-6-(pentafluoroethyl)phenyl)-1,4-dihydro-6-methyl-3,5-pyridinedicarboxylate,
3-Methyl 5-(1-methylethyl) 2-(fluoromethyl)-4-(4-fluoro-2-(trifluoromethyl)phenyl)-1,4-dihydro-6-methyl-3,5-pyridinedicarboxylate.
3-Methyl 5-(1-methylethyl) 2-(fluoromethyl)-4-(8-fluoronaphthalen-1-yl)-1,4-dihydro-6-methyl-3,5-pyridinedicarboxylate,
5-Methyl 3-(2,2,2-trifluoroethyl) 2-((2-aminoethoxy)methyl)-6-(fluoromethyl)-1,4-dihydro-4-(2-(trifluoromethyl)phenyl)-3,5-pyridinedicarboxylate,
5-Methyl 3-(1-methylethyl) 2-((2-aminoethoxy)methyl)-6-(fluoromethyl)-1,4-dihydro-4-(2,3,5,6-tetrafluorophenyl)-3,5-pyridinedicarboxylate,
5-Methyl 3-(1-methylethyl) 2-((2-aminoethoxy)methyl)-6-(fluoromethyl)-1,4-dihydro-4-(2,4,6-trifluorophenyl)-3,5-pyridinedicarboxylate,
3-Methyl 5-(2,2,2-trifluoroethyl) 2-(fluoromethyl)-1,4-dihydro-6-methyl-4-(2-(trifluoromethyl)phenyl)-3,5-pyridinedicarboxylate,
3-Methyl 5-(1-methylethyl) 2-(fluoromethyl)-1,4-dihydro-6-methyl-4-(2,4,6-trifluorophenyl)-3,5-pyridinedicarboxylate,
3-Methyl 5-(1-methylethyl) 2-(fluoromethyl)-1,4-dihydro-6-methyl-4-(pentafluorophenyl)-3,5-pyridinedicarboxylate,
3-Methyl 5-(1-methylethyl) 4-(5-fluoro-2-methoxyphenyl)-2-(fluoromethyl)-1,4-dihydro-6-methyl-3,5-pyridinedicarboxylate,
3-Methyl 5-(1-methylethyl) 4-(2-chloro-3,5-dimethoxyphenyl)-2-(fluoromethyl)-1,4-dihydro-6-methyl-3,5-dihydropyridine,
3-Methyl 5-(1-methylethyl) 4-(6-chloro-2-fluoro-3-methoxyphenyl)-2-(fluoromethyl)-1,4-dihydro-6-methyl-3,5-pyridinedicarboxylate,
Diethyl 2-((2-aminoethoxy)methyl)-4-(3-chloro-6- fluoro-2-(trifluoromethyl)phenyl)-1,4-dihydro-6-(trifluoromethyl)-3,5-pyridinedicarboxylate,
5-Methyl 3-(2,2,2-trichloroethyl) 2-((2-(5-amino-1H-1,2,4-triazol-3-yl)amino)methyl)-4-(2-chlorophenyl)-6-(fluoromethyl)-1,4-dihydro-3,5-pyridinecarboxylate,
3-Ethyl 5-methyl 2-((2-((5-amino-1H-1,2,4-triazol-3-yl)amino)ethoxy)-6-(fluoromethyl)-1,4-dihydro-4-(pentafluorophenyl)-3,5-pyridinedicarboxylate,
3-Ethyl 5-methyl 2-((2-(2-(5-amino-1H-1,2,4-triazol-3-yl)aminoethoxy)methyl)-4-(2-chlorophenyl)-6-(fluoromethyl)-1,4-dihydro-3,5-pyridinedicarboxylate,
3-Ethyl 5-methyl 2-((2-((5-amino-1H-1,2,4-triazol-3-yl)amino)ethoxy)methyl)-4-(3-cyanothien-2-yl)-6-(fluoromethyl)-1,4-dihydro-3,5-pyridinedicarboxylate,
3-Methyl 5-(1-methylethyl) 4-(5-amino-2-chlorophenyl)-2-(fluoromethyl)-1,4-dihydro-6-methyl-3,5-pyridinedicarboxylate,
and pharmaceutically acceptable salts, esters, amides and protected derivatives thereof.

8. A pharmaceutical formulation comprising a compound of formula I, as defined in Claim 1, in accordance with a pharmaceutically acceptable adjuvent, diluent or carrier.

9. The use of a compound of formula I, as defined in Claim 1, in the production of a pharmaceutical formulation for the treatment of a hypertensive condition.

10. A process for the production of a compound of formula I, as defined in Claim 1, or a pharmaceutically acceptable salt, ester, amide or protected derivative thereof, which comprises

a) production of a compound of formula I in which X is -O-, -NR-, -S- or a bond other than a carbon carbon bond, by reaction of a compound of formula II,

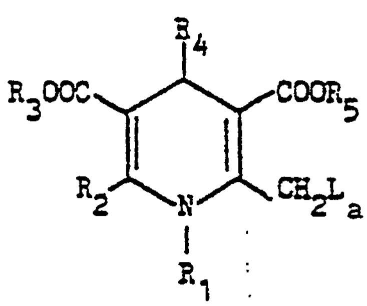

II

in which $R_1$, $R_2$, $R_3$, $R_4$ and $R_5$ are as defined above, and
$L_a$is a good leaving group,
with a compound of formula III.

$R_{11}X_aH$ III

in which $R_{11}$ is as defined above, and

$X_a$ represents -O-, -NR-, -S- or a bond

b) production of a compound of formula I in which at least one of m or $m_1$ is 1 or 2 by selective oxidation of a corresponding compound of formula I in which at least one of m or $m_1$ is 0 or 1 respectively,

c) production of a compound of formula I in which at least one of $R_5$, $R_6$, $R_{10}$ and $R_{11}$ carries an $NH_2$ group by deprotection of a corresponding compound of formula I in which at least one of $R_5$ $R_6$, $R_1$ and $R_{11}$ carries a protected -NH- group;

d) production of a compound of formula I in which X is a bond and $R_{11}$ is a group of formula IV,

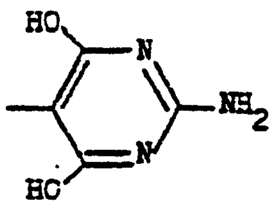

IV

by reacting guanidine with a compound of formula V,

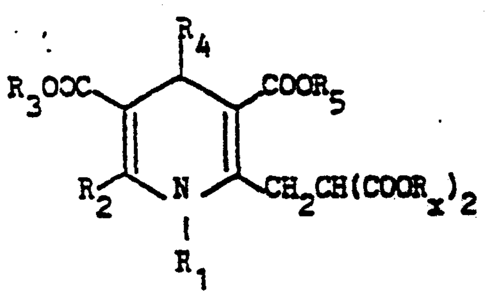

V

in which $R_1$, $R_2$, $R_3$, $R_4$ and $R_5$ are as defined above, and

$R_x$ is alkyl C 1 to 6,

e) production of a compound of formula I in which X is -O-, -S- or -NR- and $R_{11}$ is a pyrimidin-2-yl group by reaction of a corresponding compound of formula I in which X is -O-, -S- or -NR- and $R_{11}$ is hydrogen with a compound of formula VI,

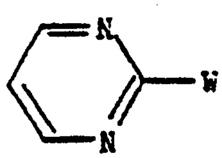

VI

in which W is a good leaving group,

f) production of a compound of formula I in which X is a bond and $R_{11}$ is a 1H-tetrazol-5-yl group by reacting a corresponding compound of formula I in which $R_{11}$ is nitrile with an azide,

g) production of a compound of formula I in which X is a bond and $R_{11}$ is 1H-pyrrol-1-yl group by reaction of a corresponding compound of formula I in which the group $XR_{11}$ is $-NH_2$ with 2,5-dimethoxytetrahydrofuran,

h) production of a compound of formula I in which X is -O- and $R_6$ is $-CONNH_2$ by hydrolysis of the product of the reaction of a corresponding compound of formula I in which X is -O- and $R_6$ is hydrogen with a compound of formula VII

$L_h-N=C=O$ VII

in which $L_h$ is a good leaving group,

i) production of a compound of formula I in which R and Z together form a bond and $R_6$ is a group $-NHC(=NH)NH_2$ by reacting a compound of formula VIII,

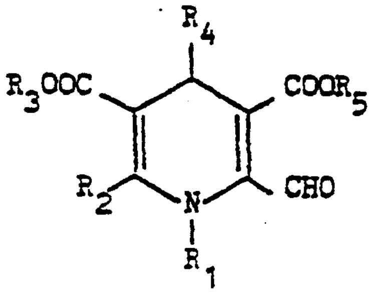

VIII

in which $R_1$, $R_2$, $R_3$. $R_4$ and $R_5$ are as defined above,
with aminoguanidine or a salt thereof.

j) production of a compound of formula I in which $R_{11}$ is a 1,2,3-triazol-1-yl group by reaction of a compound of formula I in which $R_{11}$ is an azide group,
with a compound of formula IX,

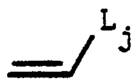

IX

in which $L_j$ is a good leaving group,

k) production of a compound of formula I in which $R_{11}$ is a 1,2,4-triazol-4-yl group by reaction of a compound of formula I in which $R_{11}$ is an amino group,
with a compound of formula XI,
$L_kHC=N-N=CHL_{k'}$ XI
in which $L_k$ and $L_{k'}$, which may be the same or different, are good leaving groups,

l) production of a compound of formula I in which $R_6$ is a group -C(=NOH)$NH_2$ by reaction of a compound of formula I in which $R_6$ is nitrile,
with hydroxylamine, or a salt thereof,

m) production of a compound of formula I in which at least one of $R_5$, $R_{10}$ and $R_{11}$ carries a -COOH group by reductive cleavage or hydrolysis of a corresponding compound of formula I in which at least one of $R_5$, $R_{10}$ and $R_{11}$ carries a carboxylic acid ester group,

n) production of a compound of formula I in which at least one of $R_5$, $R_{10}$ and $R_{11}$ carries a carboxylic acid ester group by esterification or transesterification of a corresponding compound of formula I in which at least one of $R_5$, $R_{10}$ and $R_{11}$ carries a -COOH group,

o) production of a compound of formula I in which $R_1$ is alkyl C 1 to 6, by C 1 to 6 alkylation of a corresponding compound of formula I in which $R_1$ is hydrogen,

p) production of a compound of formula I in which $R_6$ contains a 3-amino-4H-1,2,4-triazol-5-yl group by reaction of a corresponding compound of formula I in which $R_6$ contains an amine group, with a compound of formula XII,

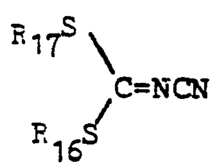

XII

in which $R_{17}$ and $R_{16}$, which may be the same or different are each alkyl C 1 to 6, and
hydrazine or a derivative thereof,

q) production of a compound of formula I in which Z is hydrogen, X is -O- and $R_6$ is $-CH_2CH_2NH_2$ by reduction of a compound of formula XIII,

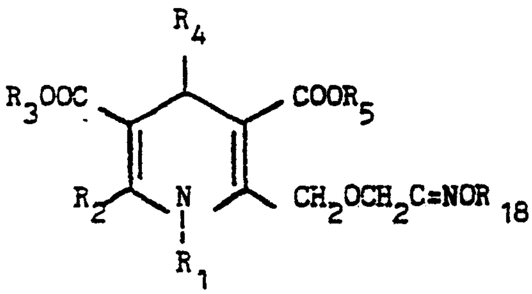

XIII

in which $R_1$, $R_2$, $R_3$, $R_4$ and $R_5$ are as defined above,
$R_{18}$ is hydrogen or alkyl C 1 to 10,

r) production of a compound of formula I in which $R_5$ contains an amide group by reaction of a corresponding compound of formula I in which $R_5$ is a group $-(CH_2)_{n_r}$ $CO_2H$ with the corresponding amine,

in which $n_r$ is an integer from 1 to 8,

s) production of an optical isomer of a compound of formula I by resolution of a mixture of optical isomers of the compound,

t) production of a pharmaceutically acceptable salt of a compound of formula I, by treating a compound of formula I, or another salt, with a compound containing an available pharmaceutically acceptable ion and capable of converting the compound of formula I or the other salt to a pharmaceutically acceptable salt of the compound of formula I,

u) reaction of the compounds of formulae XIV, XV, XVI and XVII,

$R_4CHO$ XIV
$R_5OOCCH_2COCH_2XR_6$ XV
$R_3OOCCH_2COR_2$ XVI
$R_1NH_2$ XVII

or their partial condensates, or protected derivatives thereof,
in which $R_1$, $R_2$, $R_3$, $R_4$, $R_5$, X and $R_6$ are as defined above,
and where desired or necessary converting the resulting compound of formula I to a pharmaceutically acceptable salt, ester, amide or protected derivative thereof or vice versa.

11. A compound of formula I in which $R_1$ is hydrogen for use as an intermediate.
12. A compound of formula I in which $-CHZXR_6$ is a group $-CH_2Br$ for use as an intermediate.

Claims for the following Contracting States: AT, ES

1. A process for the production of a compound of formula I,

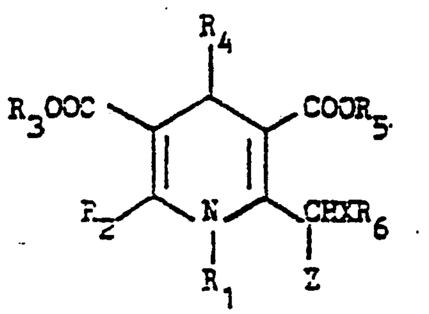

I

in which $R_2$ is alkyl C 1 to 6 optionally substituted by one or more fluorine atoms,
$R_3$ is alkyl C 1 to 6,
$R_4$ is phenyl, naphthyl or an unsaturated sulphur containing heterocyclic ring, each of which is optionally substituted by one or more groups selected from $-CF_3$, -CN, $-NO_2$, $NH_2$, halogen, alkyl C 1 to 6, alkoxy C 1 to 6 or $C_2F_5$,
$R_5$ is alkyl C 1 to 10 optionally interrupted by $-CONR_8-$ and optionally substituted by one or more halogen atoms or by a group $-YR_7$
Y is -O-, $-S(O)_m$, $-NR_9$ or a bond,
$R_7$ is -COOH or a group $R_{10}$,
X is -O-, -NR-, $-S(O)_{m_1}$, or a bond,
Z is hydrogen or together with R forms a bond,
R is hydrogen, alkyl C 1 to 6 or together with Z forms a bond,

$R_6$ is a group $R_{11}$,
$R_{10}$ and $R_{11}$, which may be the same or different, represent a 5 or 6 membered unsaturated nitrogen containing heterocyclic ring which is optionally fused to a phenyl ring, $R_{10}$ and $R_{11}$ optionally being substituted by one or more groups selected from oxo, hydroxy, alkyl C 1 to 6, $-(CH_2)_{n_1}NH_2$, alkoxy C 1 to 6, phenyl or -COOH,
and when X is -NR- or $-S(O)_{m_1}$, $R_6$ may in addition be hydrogen or a group $-CH_2CH_2NH_2$, $-CH_2CH_2R_{11}$ or $NHC(=NH)NH_2$,
when X is -O-, $R_6$ may also be a group $-NH_2$, $-C(=O)\ (CH_2)_nNH_2$, $-CH_2CH_2NR_{14}R_{15}$ or $CH_2CH_2OCH_2CH_2NR_{14}R_{15}$,
when X is -NR-, $-S(O)_{m_1}$, or a bond, $R_6$ may also be hydrogen or a group $-C(=NR_{12})NHR_{13}$,
when Y is -O-, $R_{10}$ may in addition be phenyl,
and when $-CHZXR_6$ is $-CH_2OCH_2CH_2NH_2$ then
$R_5$ may be thietanyl.
$R_1$, $R_8$, $R_9$, $R_{13}$ and $R_{14}$, which may be the same or different, are each hydrogen or alkyl C 1 to 6,
$R_{12}$ is hydrogen, alkylcarbonyl C2 to 7, nitrile or hydroxy,
$R_{15}$ is hydrogen or a group $R_{11}$,
m and $m_1$, which may be the same or different, are each 0, 1 or 2,
n is 0 or 1,
$n_1$ is an integer from 0 to 6
and pharmaceutically acceptable salts, esters, amides and protected derivatives thereof,
with the provisos that:-
i) when $R_2$ is unsubstituted alkyl then
a) the group $-CHZXR_6$ is not methyl, $-CH_2OCONH_2$, $-CH_2OCH_2CH_2NH_2$, $-CH=NCH_2CH_2NH_2$ or the groups of formulae XXV and XXVI,

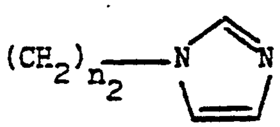

XXV

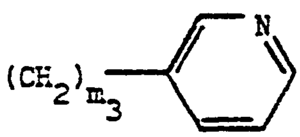

XXVI

in which $n_2$ is 1 or 2, and
$m_3$ is 0, 1 or 2, and
b) $R_4$ is not a group of formula XXVII,

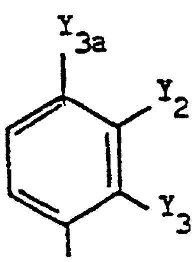

XXVII

in which $Y_2$ is hydrogen, -Cl or $-CF_3$, and $Y_3$ and $Y_{3a}$, which may be same or different, are each hydrogen or -Cl,
ii) when $-CHZXR_6$ is methyl and
A. $R_5$ is unsubstituted alkyl, then $R_4$ is not a monosubstituted phenyl group, 2-fluoro-6-(trifluoromethyl) phenyl, a group of formula XXVIII,

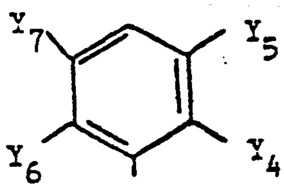

XXVIII

in which, either
a) $Y_4$ is -Cl, methyl or methoxy,
$Y_5$ is -Cl. $-NO_2$, $-CF_3$, methyl or methoxy,
and
$Y_6$ and $Y_7$ are both hydrogen or
b) $Y_5$ is $-NO_2$, $Y_6$ is -Cl or methyl and $Y_4$ and $Y_7$ are both hydrogen;
or a 2,3,6-trisubstituted phenyl group other than 6-chloro-2-fluoro-3-methoxyphenyl; and
B. $R_5$ is methyl then $R_4$ is not a pentafluorophenyl group,
iii) when $R_2$ is $-CH_2F$ and
A. $R_5$ is unsubstituted alkyl, other than cycloalkyl, and the group $-CHZXR_6$ is $-CH_2OCH_2CH_2NR_{14}R_{15}$ then $R_4$ is not a group of formula XXIX,

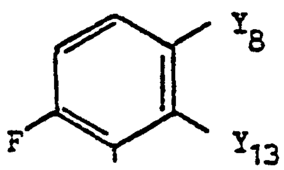

XXIX

in which,
a) $Y_{13}$ is $-CF_3$ and $Y_8$ is -Cl, or
b) $Y_{13}$ is -F and $Y_8$ is either -F or $-NO_2$, and
B. $R_5$ is alkyl substituted by halogen then $R_4$ is not 2,3,6-trisubstituted phenyl group, and
C. $R_5$ is 1-methylethyl then
a) $R_4$ is not a group of formula XXX,

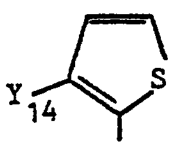

XXX

in which $Y_{14}$ is nitro, nitrile or methyl, and
b) $R_4$ is not a group of formula XXXI,

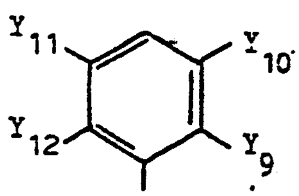

XXXI

in which $Y_9$ and $Y_{11}$, which may be the same or different, are each -Cl or -F,
$Y_{10}$ is -Cl or $-CF_3$, and
$Y_{12}$ is $-CF_3$ or hydrogen;
iv) when $R_5$ is an uninterrupted alkyl group and Y is -O-, then $R_7$ is not a monosubstituted pyrazol-3-yl group, a 2,5-disubstituted pyrimidin-4-yl group, or protected derivatives thereof,
which comprises

a) production of a compound of formula I in which X is -O-, -NR-, -S- or a bond other than a carbon carbon bond, by reaction of a compound of formula II,

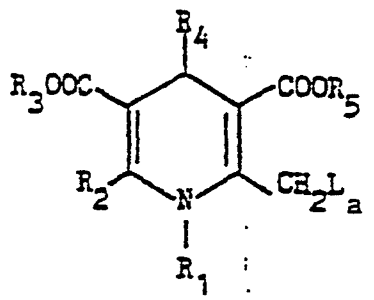

II

in which $R_1$, $R_2$, $R_3$, $R_4$ and $R_5$ are as defined above, and
$L_a$ is a good leaving group,
with a compound of formula III,
$R_{11}X_aH$ III
in which $R_{11}$ is as defined above, and
$X_a$ represents -O-, -NR-, -S- or a bond

b) production of a compound of formula I in which at least one of m or $m_1$ is 1 or 2 by selective oxidation of a corresponding compound of formula I in which at least one of m or $m_1$ is 0 or 1 respectively,

c) production of a compound of formula I in which at least one of $R_5$, $R_6$, $R_{10}$ and $R_{11}$ carries an $NH_2$ group by deprotection of a corresponding compound of formula I in which at least one of $R_5$, $R_6$, $R_{10}$ and $R_{11}$ carries a protected -NH- group,

d) production of a compound of formula I in which X is a bond and $R_{11}$ is a group of formula IV,

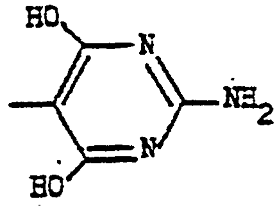

IV

by reacting guanidine with a compound of formula V,

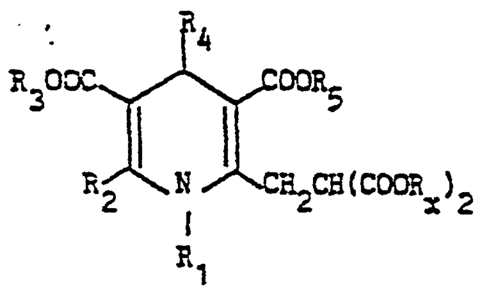

V

in which $R_1$, $R_2$, $R_3$, $R_4$ and $R_5$ are as defined above, and
$R_x$ is alkyl C 1 to 6,

e) production of a compound of formula I in which X is -O-, -S- or -NR- and $R_{11}$ is a pyrimidin-2-yl group by reaction of a corresponding compound of formula I in which X is -O-, -S- or -NR- and $R_{11}$ is hydrogen with a compound of formula VI,

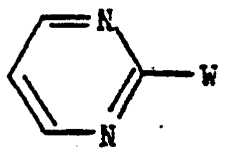

VI

in which W is a good leaving group,

f) production of a compound of formula I in which X is a bond and $R_{11}$ is a 1H-tetrazol-5-yl group by reacting a corresponding compound of formula I in which $R_{11}$ is nitrile with an azide,

g) production of a compound of formula I in which X is a bond and $R_{11}$ is 1H-pyrrol-1-yl group by reaction of a corresponding compound of formula I in which the group $XR_{11}$ is $-NH_2$ with 2,5-dimethoxytetrahydrofuran,

h) production of a compound of formula I in which X is -O- and $R_6$ is $-CONH_2$ by hydrolysis of the product of the reaction of a corresponding compound of formula I in which X is -O- and $R_6$ is hydrogen with a compound of formula VII

$L_h-N=C=O$ VII

in which $L_h$ is a good leaving group,

i) production of a compound of formula I in which R and Z together form a bond and $R_6$ is a group $-NHC(=NH)NH_2$ by reacting a compound of formula VIII,

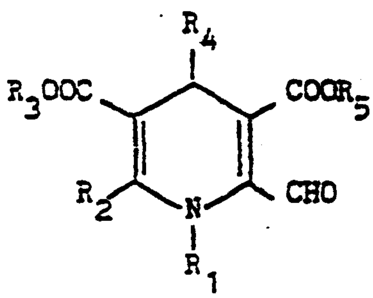

VIII

in which $R_1$, $R_2$, $R_3$, $R_4$ and $R_5$ are as defined above,
with aminoguanidine or a salt thereof,

j) production of a compound of formula I in which $R_{11}$ is a 1,2,3-triazol-1-yl group by reaction of a compound of formula I in which $R_{11}$ is an azide group,
with a compound of formula IX,

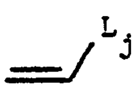

IX

in which $L_j$ is a good leaving group,

k) production of a compound of formula I in which $R_{11}$ is a 1,2,4-triazol-4-yl group by reaction of a compound of formula I in which $R_{11}$ is an amino group,
with a compound of formula XI,

$L_kHC=N-N=CHL_{k'}$ XI

in which $L_k$ and $L_{k'}$, which may be the same or different, are good leaving groups,

l) production of a compound of formula I in which $R_6$ is a group $-C(=NOH)NH_2$ by reaction of a compound of formula I in which $R_6$ is nitrile,
with hydroxylamine, or a salt thereof,

m) production of a compound of formula I in which at least one of $R_5$, $R_{10}$ and $R_{11}$ carries a -COOH group by reductive cleavage or hydrolysis of a corresponding compound of formula I in which at least one of $R_5$, $R_{10}$ and $R_{11}$ carries a carboxylic acid ester group,

n) production of a compound of formula I in which at least one of $R_5$, $R_{10}$ and $R_{11}$ carries a carboxylic acid ester group by esterification or transesterification of a corresponding compound of formula I in which at least one of $R_5$, $R_{10}$ and $R_{11}$ carries a -COOH group,

o) production of a compound of formula I in which $R_1$ is alkyl C 1 to 6, by C 1 to 6 alkylation of a corresponding compound of formula I in which $R_1$ is hydrogen,

p) production of a compound of formula I in which $R_6$ contains a 3-amino-4H-1,2,4-triazol-5-yl group by reaction of a corresponding compound of formula I in which $R_6$ contains an amine group,

with a compound of formula XII,

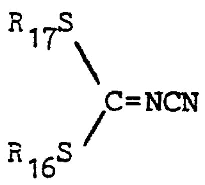

XII

in which $R_{17}$ and $R_{16}$, which may be the same or different are each alkyl C 1 to 6, and hydrazine or a derivative thereof,

q) production of a compound of formula I in which Z is hydrogen, X is -O- and $R_6$ is $-CH_2CH_2NH_2$ by reduction of a compound of formula XIII,

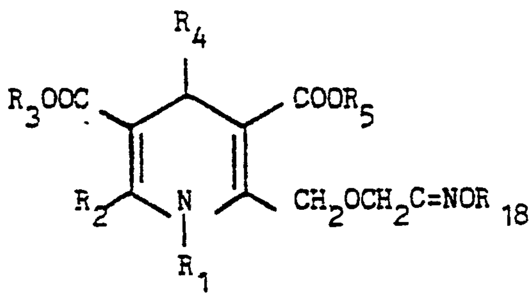

XIII

in which $R_1$, $R_2$, $R_3$, $R_4$ and $R_5$ are as defined above,
$R_{18}$ is hydrogen or alkyl C 1 to 10,

r) production of a compound of formula I in which $R_5$ contains an amide group by reaction of a corresponding compound of formula I in which $R_5$ is a group $-(CH_2)_{n_r}$ $CO_2H$ with the corresponding amine,

in which $n_r$ is an integer from 1 to 8,

s) production of an optical isomer of a compound of formula I by resolution of a mixture of optical isomers of the compound,

t) production of a pharmaceutically acceptable salt of a compound of formula I, by treating a compound of formula I, or another salt, with a compound containing an available pharmaceutically acceptable ion and capable of converting the compound of formula I or the other salt to a pharmaceutically acceptable salt of the compound of formula I,

u) reaction of the compounds of formulae XIV, XV, XVI and XVII,
$R_4CHO$ XIV
$R_5OOCCH_2COCH_2XR_6$ XV
$R_3OOCCH_2COR_2$ XVI
$R_1NH_2$ XVII
or their partial condensates, or protected derivatives thereof,
in which $R_1$, $R_2$, $R_3$, $R_4$, $R_5$ and $R_6$ are as defined above,
and where desired or necessary converting the resulting compound of formula I to a pharmaceutically acceptable salt, ester, amide or protected derivative thereof or vice versa.

2. A process according to Claim 1, wherein $R_2$ is fluoromethyl.

3. A process according to Claim 1, wherein $R_4$ is a phenyl group substituted in one or more of the 2-, 3- or 6- positions.

4. A process according to Claim 1, wherein $R_5$ is a group $-YR_{10}$ and $-CHZXR_6$ is methyl.

5. A process according to Claim 1, wherein $R_5$ is alkyl C 1 to 6 and $R_6$ is $-CH_2CH_2NH_2$ or a group $R_{11}$.

6. A process according to Claim 1, wherein the group $-CHZXR_6$ is selected from (2-aminoethylthio)methyl; (2-aminoethylsulphonyl)methyl; (aminoiminomethyl) thiomethyl; 1H-pyrrol-1-ylmethyl; (4H-1,2,4-triazol-4-yl) methyl; (5-ethoxycarbonyl-3,4-dihydro-4-oxopyrimidin-2-ylthio)methyl; (3,4-dihydro-4-oxopyrimidin-2-ylthio)methyl; (pyridin-2-ylthio)methyl; (pyridin-3-yloxy)methyl; methyl; (2-aminoethoxy)methyl and (2-((5-amino-1,2,4-triazol-3-yl)amino)ethoxy)methyl.

7. A process according to Claim 1, wherein the compound of formula I is selected from,
3-Methyl 5-(1-methylethyl) 4-(3-chloro-6-fluoro-2-(trifluoromethyl)phenyl)-2-(fluoromethyl)-1,4-dihydro-6-(pyridin-3-yloxymethyl)-3,5-pyridinedicarboxylate,

3-Methyl 5-(methylethyl) 4-(3-chloro-6-fluoro-2-(trifluoromethyl)phenyl)-2-(fluoromethyl)-1,4-dihydro--6-((1H-1,2,4-triazol-1-yl)methyl)-3,5-pyridinedicarboxylate.
5-Methyl 3-(1-methylethyl) 2-((2-aminoethyl) aminomethyl)-4-(3-chloro-6-fluoro-2-(trifluoromethyl)phenyl)-6-(fluoromethyl)-1,4-dihydro-3,5-pyridinedicarboxylate,
5-Methyl 3(-1-methylethyl) 2-((2-aminoethyl)thiomethyl)-4-(3-chloro-6-fluoro-2-(trifluoromethyl)phenyl)-6-(fluoromethyl)-1,4-dihydro-3,5-pyridinedicarboxylate,
5-Methyl-3-(1-methylethyl)-2-((2-amino-4,6-dihydroxypyrimidin-5-yl)methyl)-4-(3-chloro-6-fluoro-2- (trifluoromethyl)phenyl-6-(fluoromethyl)-1,4-dihydro-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-pyrazol-1-yl)methyl)-3,5-pyridinedicarboxylate,
5-Methyl 3-(1-methylethyl) 2-(((aminocarbonyl)oxy)methyl)-4-(3-chloro-6-fluoro-2-(trifluoromethyl)phenyl)-6-(fluoromethyl)-1,4-dihydro-3,5-pyridinedicarboxylate,
3-Methyl 5-(1-methylethyl) 4-(3-chloro-6-fluoro-2--(trifluoromethyl)phenyl)-2-(fluoromethyl)-1,4-dihydro-6-(pyrimidin-2-yloxymethyl)-3,5-pyridinedicarboxylate,
5-Methyl 3-(1-methylethyl) 2-((aminoacetyloxy)methyl)-4-(3-chloro-6-fluoro-2-(trifluoromethyl)phenyl)-6-(fluoromethyl)-1,4-dihydro-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-tetrazol-5-yl)methyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-(1H-pyrrol-1-ylmethyl)-3,5-pyridinedicarboxylate,
5-Methyl 3-(1-methylethyl) 2-(((aminoiminomethyl)hydrazono)methyl)-4-(3-chloro-6-fluoro-2-(trifluoromethyl)phenyl)-6-(fluoromethyl)-1,4-dihydro-3,5-pyridinedicarboxylate,
3-Methyl 5-(1-methylethyl) 4-(3-chloro-6-fluoro-2- (trifluoromethyl)phenyl)-2-(fluoromethyl)-1,4-dihydro-6-(1H-1,2,3-triazol-1-yl)methyl-3,5-pyridinedicarboxylate,
5-Methyl 3-(1-methylethyl) 4-(3-chloro-6-fluoro-2-(trifluoromethyl)phenyl)-2-((5-ethoxycarbonyl-3,4-dihydro-4-oxopyrimidin-2-ylthio)methyl)-6-(fluoromethyl)-1,4-dihydro-3,5-pyridinedicarboxylate,
3-Ethyl 5-methyl 2-((1H-benzotriazol-1-yloxy)methyl)-4-(2,3-dichlorophenyl)-6-(fluoromethyl)-1,4-dihydro-3,5-pyridinedicarboxylate,
3-Ethyl 5-methyl 2-((aminoiminomethyl)thiomethyl)-4-(2,3-dichlorophenyl)-6-(fluoromethyl)-1,4-dihydro-3,5-pyridinedicarboxylate,
5-Ethyl 3-methyl 4-(2-chlorophenyl)-2-(fluoromethyl)-1,4-dihydro-6-((4H-1,2,4-triazol-4-yl)methyl)-3,5-pyridinedicarboxylate,
5-Methyl 3-(1-methylethyl)2-[2-amino-2-(hydroximino)ethyl]-4-(3-chloro-6-fluoro-2-(trifluoromethyl)phenyl)-6-(fluoromethyl)-1,4-dihydro-3,5-pyridinedicarboxylate,
3-Methyl 5-(6-(1-methyl-3-phenylpyrazol-5-yloxy)hexyl) 4-(3-chloro-6-fluoro-2-(trifluoromethyl)phenyl)-2-(fluoromethyl)-1,4-dihydro-6-methyl-3,5-pyridinedicarboxylate,
3-Methyl 5-(6-phenoxyhexyl) 4-(3-chloro-6-fluoro-2-(trifluoromethyl)phenyl)-2-(fluoromethyl)-1,4-dihydro-6-methyl-3,5-pyridinedicarboxylate,
3-Methyl 5-(6-(6-methyl-2-phenylpyrimidin-4-ylthio)hexyl) 4-(3-chloro-6-fluoro-2-(trifluoromethyl)phenyl)-2-(fluoromethyl)-1,4-dihydro-6-methyl-3,5-pyridinedicarboxylate,
3-Methyl 5-(6-(1-methyl-5-phenylpyrazol-3-yloxy)hexyl) 4-(3-chloro-6-fluoro-2-(trifluoromethyl)-phenyl)-2-(fluoromethyl)-1,4-dihydro-6-methyl-3,5-pyridinedicarboxylate,
5-(7-Carboxyheptyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(8-Methoxy-8-oxooctyl) 3-methyl 4-(3-chloro-6-fluoro--2-(trifluoromethyl)phenyl)-2-(fluoromethyl)-1,4-dihydro-6-methyl-3,5-pyridinedicarboxylate,
3-Methyl 5-(2-oxo-2-((3-(5-phenylpyrazol-3-yloxy)propyl)amino)ethyl) 4-(3-chloro-6-fluoro-2-(trifluoromethyl)phenyl)-2-(fluoromethyl)-1,4-dihydro-6-methyl-3,5-pyridinedicarboxylate,
3-Ethyl 5-methyl 2-(2-(2-aminoethoxy)ethoxymethyl)-4-(3-chloro-6-fluoro-2-(trifluoromethyl)phenyl)-6-(fluoromethyl)-1,4-dihydro-3,5-pyridinedicarboxylate,
3-Ethyl 5-methyl 2-(2-aminoethoxymethyl)-4-(2-chlorophenyl)-6-(fluoromethyl)-1,4-dihydro-3,5-pyridinedicarboxylate,
3-Ethyl 5-methyl 2-((2-aminoethoxy)methyl)-4-(3- chloro-2-(trifluoromethyl)phenyl)-6-(fluoromethyl)-1,4-dihydro-3,5-pyridinedicarboxylate,
3-Methyl 5-(1-methylethyl) 2-(fluoromethyl)-4-(2-fluoro-6-nitrophenyl)-1,4-dihydro-6-methyl-3,5-pyridinedicarboxylate,
3-Methyl 5-(1-methylethyl) 4-(2-chloro-6-methylphenyl)-2-(fluoromethyl)-1,4-dihydro-6-methyl-3,5-pyridinedicarboxylate,
3-Cyclobutyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,
5-Methyl 3-(3-thietanyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-((2-aminoethoxy)methyl)-6-(fluoromethyl)-4-(pentafluorophenyl)-1,4-dihydro-3,5-pyridinedicarboxylate,
3-Ethyl 5-methyl 2-((2-aminoethoxy)methyl)-6-(fluoromethyl)-4-(2,3,5,6-tetrafluorophenyl)-1,4-dihydro-3,5-pyridinedicarboxylate,
3-Ethyl 5-methyl 2-((2-aminoethoxy)methyl)-4-(2,3-dichlorophenyl)-6-(fluoromethyl)-1,4-dihydro-3,5-pyridinedicarboxylate,
3-Methyl 5-(1-methylethyl) 2-(fluoromethyl)-1,4-dihydro-6-methyl-4-(2,3,5,6-tetrafluorophenyl)-3,5- pyridinedicarboxylate,
3-Methyl 5-(1-methylethyl) 4-(2-fluoro-5-methoxyphenyl)-2-(fluoromethyl)-1,4-dihydro-6-methyl-3,5-pyridinedicarboxylate,
5-(6-(2,6-Dimethylpyrimidin-4-yloxy)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-Methyl 3-(2,2,2-trichloroethyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,
3-Ethyl 5-methyl 2-((2-((5-amino-1H-1,2,4-triazol-3-yl)amino)ethoxy)methyl)-6-(fluoromethyl)-1,4-dihydro-4-(2,3,5,6-tetrafluorophenyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-imidazol-1-yl)methyl)-3,5-pyridinedicarboxylate,
5-Methyl 3-(1-methylethyl) 2-((6-amino-1,2-dihydro-2-oxopyrid-1-yl)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-((2-aminopyrid-3-yloxy)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-(((1H-5-amino-1,2,4-triazol-3-yl)thio)methyl)-4-(3-chloro-6-fluoro-2- (trifluoromethyl)phenyl)-6-(fluoromethyl)-1,4-dihydro-3,5-pyridinedicarboxylate,
5-Methyl 3-(1-methylethyl) 2-((5-amino-1H-1,2,4-triazol-1-yl)ethyl)-4-(3-chloro-6-fluoro-2-(trifluoromethyl)phenyl)-6-(fluoromethyl)-1,4-dihydro-3,5-pyridinedicarboxylate,
5-methyl 3-(1-methylethyl) 2-((3-amino-1H-1,2,4-triazol-1-yl)methyl)-4-(3-chloro-6-fluoro-2-(trifluoromethyl)phenyl)-6-(fluoromethyl)-1,4-dihydro-3,5-pyridinedicarboxylate,
5-methyl 3-(1-methylethyl) 2-((3-amino-4H-1,2,4-triazol-4-yl)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 4-((3-chloro-6-fluoro-2-(trifluoromethyl)phenyl)-6-(fluoromethyl)-1,4-dihydro-2-((1-pyridinium)methyl)-3,5-pyridinedicarboxylate bromide,
5-Methyl 3-(1-methylethyl) 2-((aminoiminomethyl)thiomethyl)-4-(3-chloro-6-fluoro-2-(trifluoromethyl)phenyl)-6-fluoromethyl-1,4-dihydro-3,5-pyridinedicarboxylate,
5-Methyl 3-(1-methylethyl) 2-((2-aminoethylsulphonyl)methyl)-4-(3-chloro-6-fluoro-2-(trifluoromethyl)phenyl)-6-(fluoromethyl)-1,4-dihydro-3,5-pyridinedicarboxylate,
3-Ethyl 5-methyl 2-((2-aminoethylthio)methyl)-4-(3-chloro-6-fluoro-2-(trifluoromethyl)phenyl)-6-(fluoromethyl)- 1,4-dihydro-3,5-pyridinedicarboxylate,
3-Ethyl 5-methyl 2-((2-aminoethylthio)methyl)-4-(2-chlorophenyl)-6-(fluoromethyl)-1,4-dihydro-3,5-pyridinedicarboxylate,
5-Methyl 3-(methylethyl) 2-((aminooxy)methyl)-4-(3-chloro-6-fluoro-2-(trifluoromethyl)phenyl)-6-(fluoromethyl)-1,4-dihydro-3,5-pyridinedicarboxylate,
5-Methyl 3-(1-methylethyl) 2-((4-(2-aminoethyl)-1H-imidazol-1-yl)methyl)-4-(3-chloro-6-fluoro-2-(trifluoromethyl)phenyl)-6-(fluoromethyl)-1,4-dihydro-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((2-(1H-imidazol-4-yl)ethylamino)methyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-1,2,4-triazol-3-ylthio)methyl)-3,5-pyridinedicarboxylate,
5-Methyl 3-(1-methylethyl) 4-(-3-chloro-6-fluoro-2-(trifluoromethyl)phenyl)-2-((4,6-diaminopyrimidin-2-ylthio)methyl)-6-(fluoromethyl)-1,4-dihydro-3,5-pyridinedicarboxylate,
3-Ethyl 5-methyl 4-(2-chlorophenyl)-2-((5-ethoxycarbonyl-3,4-dihydro-4-oxopyrimidin-2-ylthio)methyl)-6-(fluoromethyl)-1,4-dihydro-3,5-pyridinedicarboxylate,
3-Ethyl 5-methyl 4-(2-chlorophenyl)-2-((3,4-dihydro-4-oxopyrimidin-2-ylthio)methyl)-6-(fluoromethyl)-1,4-dihydro-3,5-pyridinedicarboxylate,
5-Ethyl 3-methyl 4-(2-chlorophenyl)-2-(fluoromethyl)-1,4-dihydro-6-((pyrimidin-2-ylthio)methyl)-3,5-pyridinedicarboxylate,
5-Ethyl 3-methyl 4-(2-chlorophenyl)-2-(fluoromethyl)-1,4-dihydro-6-((pyridin-2-ylthio)methyl)-3,5-pyridinedicarboxylate,
3-Ethyl 5-methyl 2-((1H-benzimidazol-2-ylthio)methyl)-4-(2-chlorophenyl)-6-(fluoromethyl)-1,4-dihydro-3,5-pyridinedicarboxylate,
5-Ethyl 3-methyl 4-(2-chlorophenyl)-2-(fluoromethyl)-1,4-dihydro-6-((1H-imidazol-2-ylthio)methyl)-3,5-pyridinedicarboxylate,
3-Ethyl 5-methyl 4-(2,3-dichlorophenyl)-2-((3,4-dihydro-4-oxopyrimidin-2-ylthio)methyl)-6-(fluoromethyl)-1,4-dihydro-3,5-pyridinedicarboxylate.
3-Ethyl 5-methyl 4-(dichlorophenyl)-2-((5-(ethoxycarbonyl)-3,4-dihydro-4-oxopyrimidin--2-ylthio)methyl)-6-(fluoromethyl)-1,4-dihydro-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((5-methoxy-1H-benzimidazol-2-ylthio)methyl)-3.5- pyridinedicarboxylate,

3-Ethyl 5-methyl 2-((6-carboxy-3,4-dihydro-4-oxopyrimidin-2-ylthio)methyl)-4-(2-chlorophenyl)-6-(fluoromethyl)-1,4-dihydro-3,5-pyridinedicarboxylate,
3-Ethyl 5-methyl 4-(2,3-dichlorophenyl)-2-((3,4-dihydro-6-hydroxy-4-oxopyrimidin-2-ylthio)methyl)-6-(fluoromethyl)-1,4-dihydro-3,5-pyridinedicarboxylate,
3-Ethyl 5-methyl 4-(2,3-dichlorophenyl)-2-((3,4-dihydro-6-methyl-4-oxopyrimidin-2-ylthio)methyl)-6-(fluoromethyl)-1,4-dihydropyridine-3,5-dicarboxylate,
3-Ethyl 5-methyl 2-((5-ethoxycarbonyl)-3,4-dihydro-4-oxopyrimidin-2-ylthio)methyl)-6-(fluoromethyl)-1,4-dihydro-4-(2-trifluoromethyl)phenyl)-3,5-pyridinedicarboxylate,
3-Ethyl 5-methyl 2-((3,4-dihydro-4-oxopyrimidin-2-ylthio)methyl)-6-(fluoromethyl)-1,4-dihydro-4-(2-trifluoromethyl)phenyl)-3,5-pyridinedicarboxylate,
3-Ethyl 5-methyl 2-((4-amino-5-(ethoxycarbonyl)-pyrimidin-2-ylthio)methyl)-4-(2-chlorophenyl)-6-(fluoromethyl)-1,4-dihydro-3,5-pyridinedicarboxylate,
5-Ethyl-3-methyl-4-(2,3-dichlorophenyl)-2-(fluoromethyl)-1,4-dihydro-6-(pyrid-2-ylthiomethyl)-3,5-pyridinedicarboxylate.
3-Ethyl 5-methyl 4-(2,3-dichlorophenyl)-2-((((cyanoimino)methylamino)methylthio)methyl)-6-(fluoromethyl)-1,4- dihydro-3,5-pyridinedicarboxylate,
3-Ethyl 5-methyl 2-(((acetylimino)aminomethylthio)methyl)-4-(2,3-dichlorophenyl)-6-(fluoromethyl)-1,4-dihydro-3,5-pyridinedicarboxylate,
5-Methyl 3-(1-methylethyl) 2-((2-aminocarbonyloxy) methyl)-4-(2,3-dichlorophenyl)-6-(fluoromethyl)-1,4-dihydro-3,5-pyridinedicarboxylate,
5-Ethyl 3-methyl 4-(2-chlorophenyl)-2-(fluoromethyl)-1,4-dihydro-6-((1H-pyrrol-1-yl)methyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((4H-1,2,4-triazol-4-yl)methyl)-3,5-pyridinedicarboxylate,
3-Methyl 5-(6-(5-phenylpyrazol-3-ylamino)hexyl) 4-(3-chloro-6-fluoro-2-(trifluoromethyl)phenyl)-2-(fluoromethyl)-1,4-dihydro-6-methyl-3,5-pyridinedicarboxylate,
3-Methyl 5-(6-pyrid-3-yloxy)hexyl) 4-(3-chloro-6-fluoro-2-(trifluoromethyl)phenyl)-2-(fluoromethyl)-1,4-dihydro-6-methyl-3,5-pyridinedicarboxylate,
5-(6-(1H-Imidazol-1-yl)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(6-(4,6-Dimethylpyrimidin-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(6-(1H-Benzimidazol-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-((8-Ethoxy-8-oxo)octyl) 3-methyl 4-(3-chloro-6-fluoro-2-(trifluoromethyl)phenyl)-2-(fluoromethyl)-1,4-
5-((8-Amino-8-oxo)octyl) 3-methyl 4-(3-chloro-6-fluoro-2-(trifluoromethyl)phenyl)-2-(fluoromethyl)-1,4-dihydro-6-methyl-3,5-pyridinedicarboxylate,
3-Ethyl 5-methyl 2-((2-aminoethoxy)methyl)-6-(fluoromethyl)-1,4-dihydro-4-(5-nitrothien-2-yl)-3,5-pyridinedicarboxylate,
3-Ethyl 5-methyl 2-((2-aminoethoxy)methyl)-4-(5-cyanothien-2-yl)-6-(fluoromethyl)-1,4-dihydro-3,5-pyridinedicarboxylate,
3-Ethyl 5-methyl 2-((2-aminoethoxy)methyl)-4-(3-cyanothien-2-yl)-6-(fluoromethyl)-1,4-dihydro-3,5-pyridinedicarboxylate,
3-Ethyl 5-methyl 2-((2-aminoethoxy(methyl)-6-(fluoromethyl)-4-(3-methylthien-2-yl)-1,4-dihydro-3,5-pyridinedicarboxylate,
3-Ethyl 5-methyl 2-((2-(2-aminoethoxy)ethoxy)methyl)-4-(2-chlorophenyl)-6-(fluoromethyl)-1,4-dihydro-3,5- pyridinedicarboxylate,
Diethyl 2-((2-aminoethoxy)methyl)-4-(2-chlorophenyl)-1,4-dihydro-6-(trifluoromethyl)-3,5-pyridinedicarboxylate,
3-Ethyl 5-methyl 4-(3-amino-2,6-difluorophenyl)-2-((2-aminoethoxy)methyl)-6-(fluoromethyl)-1,4-dihydro-3,5-pyridinedicarboxylate,
5-Methyl 3-(6-((6-methyl-2-phenylpyrimidin-4-yl)oxy)hexyl) 2-((2-aminoethoxy)methyl)-4-(2-chlorophenyl)-6-(fluoromethyl)-1,4-dihydro-3,5-pyridinedicarboxylate,
5-Methyl 3-(6-((6-methyl-2-phenylpyrimidin-4-yl)thio)hexyl) 2-((2-aminoethoxy)methyl)-4-(2-chlorophenyl)-6-(fluoromethyl)-1,4-dihydro-3,5-pyridinedicarboxylate,
3-(6-((4,6-Dimethylpyrimidin-2-yl)thio)hexyl) 5-methyl 2-((2-aminoethoxy)methyl)-4-(2-chlorophenyl)-6-(fluoromethyl)-1.4-dihydro-3,5-pyridinedicarboxylate,
3-Ethyl 5-methyl 2-((2-aminoethoxy)methyl)-4-(2-chloro-3,5-dimethoxyphenyl)-6-(fluoromethyl)-1,4-dihydro-3,5-pyridinedicarboxylate,
3-Ethyl 5-methyl 2-((2-aminoethoxy)methyl)-4-(2-chloro-3-(trifluoromethyl)phenyl)-6-(fluoromethyl)-1,4-dihydro-3,5-pyridinedicarboxylate,
3-Ethyl 5-methyl 2-((2-aminoethoxy)methyl)-4-(3-cyanophenyl)-6-(fluoromethyl)-1,4-dihydro-3,5-pyridinedicarboxylate,
3-Ethyl 5-methyl 2-((2-aminoethoxy)methyl)-4-(2,5- difluoro-6-(trifluoromethyl)phenyl)-6-(fluoromethyl)-1,4-dihydro-3,5-pyridinedicarboxylate.

3-Ethyl 5-methyl 2-((2-aminoethoxy)methyl)-4-(2-fluoro-5-methoxyphenyl)-6-(fluoromethyl)-1,4-dihydro-3,5-pyridinedicarboxylate.
5-Methyl 3-((6-phenoxy)hexyl) 2-((2-aminoethoxy)methyl)-4-(2-chlorophenyl)-6-(fluoromethyl)-1,4-dihydro-3,5-pyridinedicarboxylate,
3-Ethyl 5-methyl 2-((2-aminoethoxy)methyl)-4-(6-chloro-2-fluoro-3-methoxyphenyl)-6-(fluoromethyl)-1,4-dihydro-3,5-pyridinedicarboxylate,
5-Methyl 3-(2,2,2-trichloroethyl) 2-((2-aminoethoxy)methyl)-4-(2-chlorophenyl)-6-(fluoromethyl)-1,4-dihydro-3,5-pyridinedicarboxylate,
5-Methyl 3-(2,2,2-trifluoroethyl) 2-((2-aminoethoxy)methyl)-4-(2-chlorophenyl)-6-(fluoromethyl)-1,4-dihydro-3,5-pyridinedicarboxylate,
3-Methyl 5-(1-methylethyl) 4-(3-chloro-2-fluorophenyl)-2-(fluoromethyl)-1,4-dihydro-6-methyl-3,5-pyridinedicarboxylate,
3-Methyl 5-(1-methylethyl) 4-(2,6-difluorophenyl)-2-(fluoromethyl)-1,4-dihydro-6-methyl-3,5-pyridinedicarboxylate,
3-Methyl 5-(1-methylethyl) 2-(fluoromethyl)-1,4-dihydro-6-methyl-4-(3-nitro-2-(trifluoromethyl)phenyl)-3,5- pyridinedicarboxylate,
3-Methyl 5-(1-methylethyl) 4-(2-fluoro-3-(trifluoromethyl)phenyl)-2-(fluoromethyl)-1,4-dihydro-6-methyl-3,5-pyridinedicarboxylate,
3-Methyl 5-(1-methylethyl) 4-(3-chloro-2-(trifluoromethyl)phenyl)-2-(fluoromethyl)-1,4-dihydro-6-methyl-3,5-pyridinedicarboxylate,
3-Methyl 5-(1-methylethyl) 4-(3-chloro-2-nitrophenyl)--2-(fluoromethyl)-1,4-dihydro-6-methyl-3,5-pyridinedicarboxylate,
3-Methyl 5-(1-methylethyl) 4-(2,3-difluorophenyl)-2-(fluoromethyl)-1,4-dihydro-6-methyl-3,5-pyridinedicarboxylate,
3-Methyl 5-(1-methylethyl) 2-(fluoromethyl)-1,4-dihydro-4-(5-methoxy-2-nitrophenyl)-6-methyl-3,5-pyridinedicarboxylate,
3-Methyl 5-(1-methylethyl) 2-fluoromethyl-4-(2-fluoro-6-(pentafluoroethyl)phenyl)-1,4-dihydro-6-methyl-3,5-pyridinedicarboxylate,
3-Methyl 5-(1-methylethyl) 2-(fluoromethyl)-4-(4-fluoro-2-(trifluoromethyl)phenyl)-1,4-dihydro-6-methyl-3,5-pyridinedicarboxylate,
3-Methyl 5-(1-methylethyl) 2-(fluoromethyl)-4-(8-fluoronaphthalen-1-yl)-1,4-dihydro-6-methyl-3,5-pyridinedicarboxylate,
5-Methyl 3-(2,2,2-trifluoroethyl) 2-((2-aminoethoxy)methyl)-6-(fluoromethyl)-1,4-dihydro-4-(2-(trifluoromethyl)phenyl)-3,5-pyridinedicarbxylate,
5-Methyl 3-(1-methylethyl) 2-((2-aminoethoxy)methyl)-6-(fluoromethyl)-1,4-dihydro-4-(2,3,5,6-tetrafluorophenyl)-3,5-pyridinedicarboxylate,
5-Methyl 3-(1-methylethyl) 2-((2-aminoethoxy)methyl)-6-(fluoromethyl)-1,4-dihydro-4-(2,4,6-trifluorophenyl)-3,5-pyridinedicarboxylate,
3-Methyl 5-(2,2,2-trifluoroethyl) 2-(fluoromethyl)-1,4-dihydro-6-methyl-4-(2-(trifluoromethyl)-phenyl)-3.5-pyridinedicarboxylate,
3-Methyl 5-(1-methylethyl) 2-(fluoromethyl)-1,4-dihydro-6-methyl-4-(2,4,6-trifluorophenyl)-3,5-pyridinedicarboxylate,
3-Methyl 5-(1-methylethyl) 2-(fluoromethyl)-1,4-dihydro-6-methyl-4-(pentafluorophenyl)-3,5-pyridinedicarboxylate,
3-Methyl 5-(1-methylethyl) 4-(5-fluoro-2-methoxyphenyl)-2-(fluoromethyl)-1,4-dihydro-6-methyl-3,5-pyridinedicarboxylate,
3-Methyl 5-(1-methylethyl) 4-(2-chloro-3,5-dimethoxyphenyl)-2-(fluoromethyl)-1,4-dihydro-6-methyl-3,5-dihydropyridine,
3-Methyl 5-(1-methylethyl) 4-(6-chloro-2-fluoro-3- methoxyphenyl)-2-(fluoromethyl)-1,4-dihydro-6-methyl-3,5-pyridinedicarboxylate,
Diethyl 2-((2-aminoethoxy)methyl)-4-(3-chloro-6-fluoro-2-(trifluoromethyl)phenyl)-1,4-dihydro-6-(trifluoromethyl)-3,5-pyridinedicarboxylate,
5-Methyl 3(-2,2,2-trichloroethyl) 2-((2-((5-amino-1H-1,2,4-triazol-3-yl)amino)ethoxy)methyl)-4-(2-chlorophenyl)-6-(fluoromethyl)-1,4-dihydro-3,5-pyridinedicarboxylate,
3-Ethyl 5-methyl 2-((2-((5-amino-1H-1,2,4-triazol-3-yl)amino)ethoxy)methyl)-6-(fluoromethyl)-1,4-dihydro-4-(pentafluorophenyl)-3,5-pyridinedicarboxylate,
3-Ethyl 5-methyl 2-((2-(2-(5-amino-1H-1,2,4-triazol-3-yl) aminoethoxy)ethoxy)methyl)-4-(2-chlorophenyl)-6-(fluoromethyl)-1,4-dihydro-3,5-pyridinedicarboxylate,
3-Ethyl 5-methyl 2-((2-((5-amino-1H-1,2,4-triazol-3-yl)amino)ethoxy)methyl)-4-(3-cyanothien-2-yl)-6-(fluoromethyl)-1.4-dihydro-3.5-pyridinedicarboxylate,
3-Methyl 5-(1-methylethyl) 4-(5-amino-2-chlorophenyl)-2-(fluoromethyl)-1,4-dihydro-6-methyl-3,5-pyridinedicarboxylate,
and pharmaceutically acceptable salts, esters, amides and protected derivatives thereof.

Claims for the following Contracting State: GR

1. A process for the production of a compound of formula I,

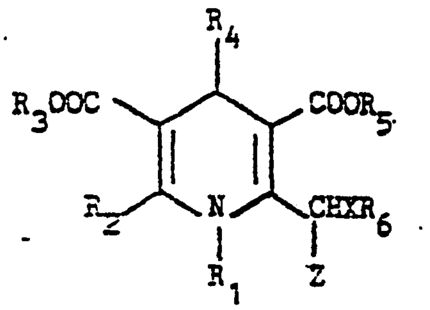

I

in which $R_2$ is alkyl C 1 to 6 optionally substituted by one or more fluorine atoms,

$R_3$ is alkyl C 1 to 6,

$R_4$ is phenyl, naphthyl or an unsaturated sulphur containing heterocyclic ring, each of which is optionally substituted by one or more groups selected from $-CF_3$, -CN, $-NO_2$, $NH_2$, halogen, alkyl C 1 to 6, alkoxy C 1 to 6 or $C_2F_5$,

$R_5$ is alkyl C 1 to 10 optionally interrupted by $-CONR_8-$ and optionally substituted by one or more halogen atoms or by a group $-YR_7$

Y is -O-, $-S(O)_m$, $-NR_9$ or a bond,

$R_7$ is -COOH or a group $R_{10}$,

X is -O-, -NR-, $-S(O)_{m_1}$, or a bond,

Z is hydrogen or together with R forms a bond,

R is hydrogen, alkyl C 1 to 6 or together with Z forms a bond,

$R_6$ is a group $R_{11}$,

$R_{10}$ and $R_{11}$, which may be the same or different, represent a 5 or 6 membered unsaturated nitrogen containing heterocyclic ring which is optionally fused to a phenyl ring, $R_{10}$ and $R_{11}$ optionally being substituted by one or more groups selected from oxo, hydroxy, alkyl C 1 to 6, $-(CH_2)_{n_1}NH_2$, alkoxy C 1 to 6, phenyl or -COOH,

and when X is -NR- or $-S(O)_{m_1}-$, $R_6$ may in addition be hydrogen or a group $-CH_2CH_2NH_2$, $-CH_2CH_2R_{11}$ or $NHC(=NH)NH_2$,

when X is -O-, $R_6$ may also be a group $-NH_2$, $-C(=O)(CH_2)_nNH_2$, $-CH_2CH_2NR_{14}R_{15}$ or $CH_2CH_2OCH_2NR_{14}R_{15}$,

when X is -NR-, $-S(O)_{m_1}-$, or a bond, $R_6$ may also be hydrogen or a group $-C(=NR_{12})NHR_{13}$,

when Y is -O-, $R_{10}$ may in addition be phenyl,

and when $-CHZXR_6$ is $-CH_2OCH_2CH_2NH_2$ then $R_5$ may be thietanyl,

$R_1$, $R_8$, $R_9$, $R_{13}$ and $R_{14}$, which may be the same or different, are each hydrogen or alkyl C 1 to 6,

$R_{12}$ is hydrogen, alkylcarbonyl C2 to 7, nitrile or hydroxy,

$R_{15}$ is hydrogen or a group $R_{11}$,

m and $m_1$, which may be the same or different, are each 0, 1 or 2,

n is 0 or 1,

$n_1$ is an integer from 0 to 6

and pharmaceutically acceptable salts, esters, amides and protected derivatives thereof,

with the provisos that:-

i) when $R_2$ is unsubstituted alkyl then

a) the group $-CHZXR_6$ is not methyl, $-CH_2OCONH_2$, $-CH_2OCH_2CH_2NH_2$, $-CH=NCH_2CH_2NH_2$ or the groups of formulae XXV and XXVI,

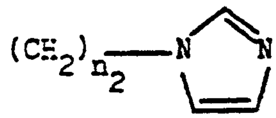

XXV

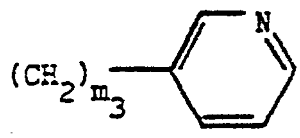

XXVI

in which $n_2$ is 1 or 2, and
$m_3$ is 0, 1 or 2, and
b) $R_4$ is not a group of formula XXVII,

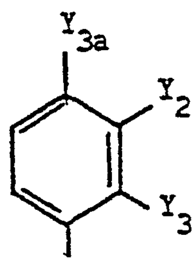

XXVII

in which $Y_2$ is hydrogen, -Cl or $-CF_3$, and $Y_3$ and $Y_{3a}$, which may be same or different, are each hydrogen or -Cl,
ii) when $-CHZXR_6$ is methyl and
A. $R_5$ is unsubstituted alkyl, then $R_4$ is not a monosubstituted phenyl group, 2-fluoro-6-(trifluoromethyl) phenyl, a group of formula XXVIII,

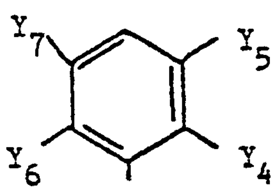

XXVIII

in which, either
a) $Y_4$ is -Cl, methyl or methoxy,
$Y_5$ is -Cl, $-NO_2$, $-CF_3$, methyl or methoxy, and
$Y_6$ and $Y_7$ are both hydrogen or
b) $Y_5$ is $-NO_2$, $Y_6$ is -Cl or methyl and $Y_4$ and $Y_7$ are both hydrogen;
or a 2,3,6-trisubstituted phenyl group other then 6-chloro-2-fluoro-3-methoxyphenyl; and
B. $R_5$ is methyl then $R_4$ is not a pentafluorophenyl group,
iii) when $R_2$ is $-CH_2F$ and
A. $R_5$ is unsubstituted alkyl, other than cycloalkyl, and the group $-CHZXR_6$ is $-CH_2OCH_2CH_2NR_{14}R_{15}$ then $R_4$ is not a group of formula XXIX,

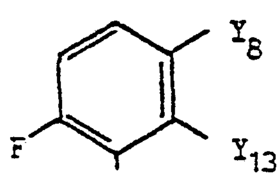

XXIX

in which,

a) $Y_{13}$ is $-CF_3$ and $Y_8$ is -Cl, or
b) $Y_{13}$ is -F and $Y_8$ is either -F or $-NO_2$, and
B. $R_5$ is alkyl substituted by halogen then $R_4$ is not 2,3,6-trisubstituted phenyl group, and
C. $R_5$ is 1-methylethyl then
a) $R_4$ is not a group of formula XXX,

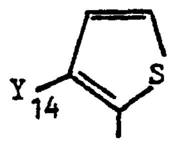

XXX

in which $Y_{14}$ is nitro, nitrile or methyl, and
b) $R_4$ is not a group of formula XXXI,

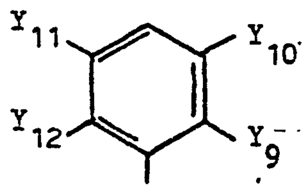

XXXI

in which $Y_9$ and $Y_{11}$, which may be the same or different, are each -Cl or -F,
$Y_{10}$ is -Cl or $-CF_3$, and
$Y_{12}$ is $-CF_3$ or hydrogen;
iv) when $R_5$ is an uninterrupted alkyl group and Y is -O-, then $R_7$ is not a monosubstituted pyrazol-3-yl group, a 2,5-disubstituted pyrimidin-4-yl group, or protected derivatives thereof,
which comprises

a) production of a compound of formula I in which X is -O-, -NR-, -S- or a bond other than a carbon carbon bond, by reaction of a compound of formula II,

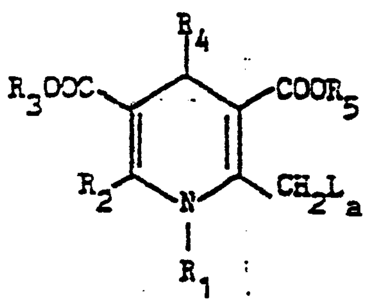

II

in which $R_1$, $R_2$, $R_3$, $R_4$ and $R_5$ are as defined above, and
$L_a$ is a good leaving group,
with a compound of formula III,
$R_{11}X_aH$ III
in which $R_{11}$ is as defined above, and
$X_a$ represents -O-, -NR-, -S- or a bond

b) production of a compound of formula I in which at least one of m or $m_1$ is 1 or 2 by selective oxidation of a corresponding compound of formula I in which at least one of m or $m_1$ is 0 or 1 respectively,

c) production of a compound of formula I in which at least one of $R_5$, $R_6$, $R_{10}$ and $R_{11}$ carries an $NH_2$ group by deprotection of a corresponding compound of formula I in which at least one of $R_5$, $R_6$, $R_{10}$ and $R_{11}$ carries a protected -NH- group,

d) production of a compound of formula I in which X is a bond and $R_{11}$ is a group of formula IV,

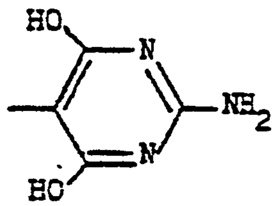

IV

by reacting guanidine with a compound of formula V,

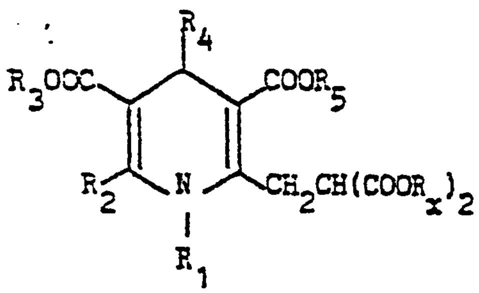

V

in which $R_1$, $R_2$, $R_3$, $R_4$ and $R_5$ are as defined above, and
$R_x$ is alkyl C 1 to 6,

e) production of a compound of formula I in which X is -O-, -S- or -NR- and $R_{11}$ is a pyrimidin-2-yl group by reaction of a corresponding compound of formula I in which X is -O-, -S- or -NR- and $R_{11}$ is hydrogen with a compound of formula VI,

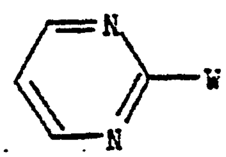

VI

in which W is a good leaving group,

f) production of a compound of formula I in which X is a bond and $R_{11}$ is a 1H-tetrazol-5-yl group by reacting a corresponding compound of formula I in which $R_{11}$ is nitrile with an azide,

g) production of a compound of formula I in which X is a bond and $R_{11}$ is 1H-pyrrol-1-yl group by reaction of a corresponding compound of formula I in which the group $XR_{11}$ is $-NH_2$ with 2,5-dimethoxytetrahydrofuran,

h) production of a compound of formula I in which X is -O- and $R_6$ is $-CONH_2$ by hydrolysis of the product of the reaction of a corresponding compound of formula I in which X is -O- and $R_6$ is hydrogen with a compound of formula VII

$L_h-N=C=O$ VII

in which $L_h$ is a good leaving group,

i) production of a compound of formula I in which R and Z together form a bond and $R_6$ is a group $-NHC(=NH)NH_2$ by reacting a compound of formula VIII,

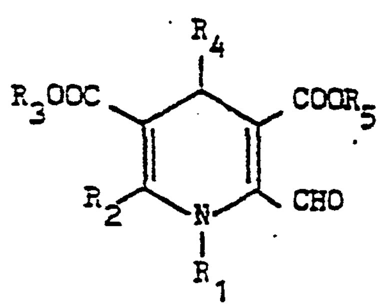

VIII

in which $R_1$, $R_2$, $R_3$, $R_4$ and $R_5$ are as defined above,
with aminoguanidine or a salt thereof.

j) production of a compound of formula I in which $R_{11}$ is a 1,2,3-triazol-1-yl group by reaction of a compound of formula I in which $R_{11}$ is an azide group, with a compound of formula IX,

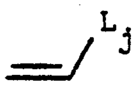

IX

in which $L_j$ is a good leaving group,

k) production of a compound of formula I in which $R_{11}$ is a 1,2,4-triazol-4-yl group by reaction of a compound of formula I in which $R_{11}$ is an amino group, with a compound of formula XI,

$L_kHC=N\text{-}N=CHL_k$, XI

in which $L_k$ and $L_{k'}$, which may be the same or different, are good leaving groups,

l) production of a compound of formula I in which $R_6$ is a group $-C(=NOH)NH_2$ by reaction of a compound of formula I in which $R_6$ is nitrile, with hydroxylamine, or a salt thereof,

m) production of a compound of formula I in which at least one of $R_5$, $R_{10}$ and $R_{11}$ carries a -COOH group by reductive cleavage or hydrolysis of a corresponding compound of formula I in which at least one of $R_5$, $R_{10}$ and $R_{11}$ carries a carboxylic acid ester group,

n) production of a compound of formula I in which at least one of $R_5$, $R_{10}$ and $R_{11}$ carries a carboxylic acid ester group by esterification or transesterification of a corresponding compound of formula I in which at least one of $R_5$, $R_{10}$ and $R_{11}$ carries a -COOH group,

o) production of a compound of formula I in which $R_1$ is alkyl C 1 to 6, by C 1 to 6 alkylation of a corresponding compound of formula I in which $R_1$ is hydrogen,

p) production of a compound of formula I in which $R_6$ contains a 3-amino-4H-1,2,4-triazol-5-yl group by reaction of a corresponding compound of formula I in which $R_6$ contains an amine group, with a compound of formula XII,

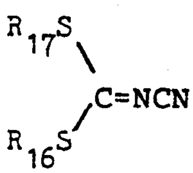

XII

in which $R_{17}$ and $R_{16}$, which may be the same or different are each alkyl C 1 to 6,
and
hydrazine or a derivative thereof,

q) production of a compound of formula I in which Z is hydrogen, X is -O- and $R_6$ is $-CH_2CH_2NH_2$ by reduction of a compound of formula XIII,

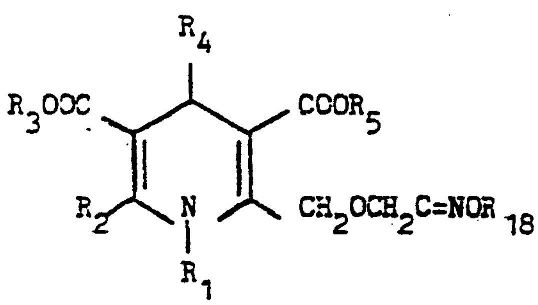

XIII

in which $R_1$, $R_2$, $R_3$, $R_4$ and $R_5$ are as defined above,
$R_{18}$ is hydrogen or alkyl C 1 to 10,

r) production of a compound of formula I in which $R_5$ contains an amide group by reaction of a corresponding compound of formula I in which $R_5$ is a group $-(CH_2)_{n_r}CO_2H$ with the corresponding amine,

in which $n_r$ is an integer from 1 to 8,

s) production of an optical isomer of a compound of formula I by resolution of a mixture of optical isomers of the compound,

t) production of a pharmaceutically acceptable salt of a compound of formula I, by treating a compound of formula I, or another salt, with a compound containing an available pharmaceutically

acceptable ion and capable of converting the compound of formula I or the other salt to a pharmaceutically acceptable salt of the compound of formula I,

u) reaction of the compounds of formulae XIV, XV, XVI and XVII,

$R_4CHO$ XIV

$R_5OOCCH_2COCH_2XR_6$ XV

$R_3OOCCH_2COR_2$ XVI

$R_1NH_2$ XVII

or their partial condensates, or protected derivatives thereof,

in which $R_1$, $R_2$, $R_3$, $R_4$, $R_5$, X and $R_6$ are as defined above,

and where desired or necessary converting the resulting compound of formula I to a pharmaceutically acceptable salt, ester, amide or protected derivative thereof or vice versa.

2. A process according to Claim 1, wherein $R_2$ is fluoromethyl.

3. A process according to Claim 1, wherein $R_4$ is a phenyl group substituted in one or more of the 2-, 3- or 6- positions.

4. A process according to Claim 1, wherein $R_5$ is a group $-YR_{10}$ and $-CHZXR_6$ is methyl.

5. A process according to Claim 1, wherein $R_5$ is alkyl C 1 to 6 and $R_6$ is $-CH_2CH_2NH_2$ or a group $R_{11}$.

6. A process according to Claim 1, wherein the group $-CHZXR_6$ is selected from (2-aminoethylthio)methyl; (2-aminoethylsulphonyl)methyl; (aminoiminomethyl)thiomethyl; 1H-pyrrol-1-ylmethyl; (4H-1,2,4-triazol-4-yl)methyl; (5-ethoxycarbonyl-3,4-dihydro-4-oxopyrimidin-2-ylthio)methyl; (3,4-dihydro-4-oxopyrimidin-2-ylthio)methyl; (pyridin-2-ylthio)methyl; (pyridin-3-yloxy)methyl; methyl; (2-aminoethoxy)methyl and (2-((5-amino-1,2,4-triazol-3-yl)amino)ethoxy)methyl.

7. A process according to Claim 1, wherein the compound of formula I is selected from,

3-Methyl 5-(1-methylethyl) 4-(3-chloro-6-fluoro-2-(trifluoromethyl)phenyl)-2-(fluoromethyl)-1,4-dihydro-6-(pyridin-3-yloxymethyl)-3,5-pyridinedicarboxylate,

3-Methyl 5-(methylethyl) 4-(3-chloro-6-fluoro-2-(trifluoromethyl)-phenyl)-2-(fluoromethyl)-1,4-dihydro--6-((1H-1,2,4-triazol-1-yl)methyl)-3,5-pyridinedicarboxylate,

5-Methyl 3-(1-methylethyl) 2-((2-aminoethyl)aminomethyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 2-((2-aminoethyl)thiomethyl)-4-(3-chloro-6-fluoro-2-(trifluoromethyl)phenyl)-6-(fluoromethyl)-1,4-dihydro-3,5-pyridinedicarboxylate,

5-Methyl-3-(1-methylethyl)-2-((2-amino-4,6-dihydroxypyrimidin-5-yl)methyl)-4-(3-chloro-6-fluoro-2- (trifluoromethyl)phenyl-6-(fluoromethyl)-1,4-dihydro-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-((1H-pyrazol-1-yl)methyl)-3,5-pyridinedicarboxylate,

5-Methyl 3-(1-methylethyl) 2-(((aminocarbonyl)oxy)methyl)-4-(3-chloro-6-fluoro-2-(trifluoromethyl)phenyl)-6-(fluoromethyl)-1,4-dihydro-3,5-pyridinedicarboxylate,

3-Methyl 5-(1-methylethyl) 4-(3-chloro-6-fluoro-2--(trifluoromethyl)phenyl)-2-(fluoromethyl)-1,4-dihydro-6-(pyrimidin-2-yloxymethyl)-3,5-pyridinedicarboxylate,

5-Methyl 3-(1-methylethyl) 2-((aminoacetyloxy)methyl)-4-(3-chloro-6-fluoro-2-(trifluoromethyl)phenyl)-6-(fluoromethyl)-1,4-dihydro-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-((1H-tetrazol-5-yl)methyl)-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-(1H-pyrrol-1-ylmethyl)-3,5-pyridinedicarboxylate,

5-Methyl 3-(1-methylethyl) 2-(((aminoiminomethyl)hydrazono)methyl)-4-(3-chloro-6-fluoro-2-(trifluoromethyl)phenyl)-6-(fluoromethyl)-1,4-dihydro-3,5-pyridinedicarboxylate,

3-Methyl 5-(1-methylethyl) 4-(3-chloro-6-fluoro-2- (trifluoromethyl)phenyl)-2-(fluoromethyl)-1,4-dihydro-6-(1H-1,2,3-triazol-1-yl)methyl-3,5-pyridinedicarboxylate,

5-Methyl 3-(1-methylethyl) 4-(3-chloro-6-fluoro-2-(trifluoromethyl)phenyl)-2-((5-ethoxycarbonyl-3,4-dihydro-4-oxopyrimidin-2-ylthio)methyl)-6-(fluoromethyl)-1,4-dihydro-3,5-pyridinedicarboxylate,

3-Ethyl 5-methyl 2-((1H-benzotriazol-1-yloxy)methyl)-4-(2,3-dichlorophenyl)-6-(fluoromethyl)-1,4-dihydro-3,5-pyridinedicarboxylate,

3-Ethyl 5-methyl 2-((aminoiminomethyl)thiomethyl)-4-(2,3-dichlorophenyl)-6-(fluoromethyl)-1,4-dihydro-3,5-pyridinedicarboxylate,

5-Ethyl 3-methyl 4-(2-chlorophenyl)-2-(fluoromethyl)-1,4-dihydro-6-((4H-1,2,4-triazol-4-yl)methyl)-3,5-pyridinedicarboxylate,

5-Methyl 3-(1-methylethyl)2-[2-amino-2-(hydroximino) ethyl]-4-(3-chloro-6-fluoro-2-(trifluoromethyl)phenyl)-6-(fluoromethyl)-1,4-dihydro-3,5-pyridinedicarboxylate,

3-Methyl 5-(6-(1-methyl-3-phenylpyrazol-5-yloxy)hexyl 4-(3-chloro-6-fluoro-2-(trifluoromethyl)phenyl)-2-(fluoromethyl)-1,4-dihydro-6-methyl-3,5-pyridinedicarboxylate,

3-Methyl 5-(6-phenoxyhexyl) 4-(3-chloro-6-fluoro-2-(trifluoromethyl)phenyl)-2-(fluoromethyl)-1,4-dihydro-6-methyl-3,5-pyridinedicarboxylate,

3-Methyl 5-(6-(6-methyl-2-phenylpyrimidin-4-ylthio)hexyl) 4-(3-chloro-6-fluoro-2-(trifluoromethyl)phenyl)-2-(fluoromethyl)-1,4-dihydro-6-methyl-3,5-pyridinedicarboxylate,

3-Methyl 5-(6-(1-methyl-5-phenylpyrazol-3-yloxy)hexyl) 4-(3-chloro-6-fluoro-2-(trifluoromethyl)phenyl)-2-(fluoromethyl)-1,4-dihydro-6-methyl-3,5-pyridinedicarboxylate,

5-(7-Carboxyheptyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-(8-Methoxy-8-oxooctyl) 3-methyl 4-(3-chloro-6-fluoro--2-(trifluoromethyl)phenyl)-2-(fluoromethyl)-1,4-dihydro-6-methyl-3,5-pyridinedicarboxylate,

3-Methyl 5-(2-oxo-2-((3-(5-phenylpyrazol-3-yloxy)propyl)amino)ethyl) 4-(3-chloro-6-fluoro-2-(trifluoromethyl)phenyl)-2-(fluoromethyl)-1,4-dihydro-6-methyl-3,5-pyridinedicarboxylate,

3-Ethyl 5-methyl 2-(2-(2-aminoethoxy)ethoxymethyl)-4-(3-chloro-6-fluoro-2-(trifluoromethyl)phenyl)-6-(fluoromethyl)-1,4-dihydro-3,5-pyridinedicarboxylate,

3-Ethyl 5-methyl 2-(2-aminoethoxymethyl)-4-(2-chlorophenyl)-6-(fluoromethyl)-1,4-dihydro-3,5-pyridinedicarboxylate,

3-Ethyl 5-methyl 2-((2-aminoethoxy)methyl)-4-(3- chloro-2-(trifluoromethyl)phenyl)-6-(fluoromethyl)-1,4-dihydro-3,5-pyridinedicarboxylate,

3-Methyl 5-(1-methylethyl) 2-(fluoromethyl)-4-(2-fluoro-6-nitrophenyl)-1,4-dihydro-6-methyl-3,5-pyridinedicarboxylate,

3-Methyl 5-(1-methylethyl) 4-(2-chloro-6-methylphenyl)-2-(fluoromethyl)-1,4-dihydro-6-methyl-3,5-pyridinedicarboxylate,

3-Cyclobutyl 5-methyl 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,

5-Methyl 3-(3-thietanyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 2-((2-aminoethoxy)methyl)-6-(fluoromethyl)-4-(pentafluorophenyl)-1,4-dihydro-3,5-pyridinedicarboxylate,

3-Ethyl 5-methyl 2-((2-aminoethoxy)methyl)-6-(fluoromethyl)-4-(2,3,5,6-tetrafluorophenyl)-1,4-dihydro-3,5-pyridinedicarboxylate,

3-Ethyl 5-methyl 2-((2-aminoethoxy)methyl)-4-(2,3-dichlorophenyl)-6-(fluoromethyl)-1,4-dihydro-3,5-pyridinedicarboxylate,

3-Methyl 5-(1-methylethyl) 2-(fluoromethyl)-1,4-dihydro-6-methyl-4-(2,3,5,6-tetrafluorophenyl)-3,5- pyridinedicarboxylate,

3-Methyl 5-(1-methylethyl) 4-(2-fluoro-5-methoxyphenyl)-2-(fluoromethyl)-1,4-dihydro-6-methyl-3,5-pyridinedicarboxylate,

5-(6-(2,6-Dimethylpyrimidin-4-yloxy)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,

5-Methyl 3-(2,2,2-trichloroethyl) 2-((2-aminoethoxy)methyl)-4-(3-chloro-6-fluoro-2-(trifluoromethyl)phenyl)-6-(fluoromethyl)-1,4-dihydro-3,5-pyridinedicarboxylate,

3-Ethyl 5-methyl 2-((2-((5-amino-1H-1,2,4-triazol-3-yl)amino)ethoxy)methyl)-6-(fluoromethyl)-1,4-dihydro-4-(2,3,5,6-tetrafluorophenyl)-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-((1H-imidazol-1-yl)methyl)-3,5-pyridinedicarboxylate,

5-Methyl 3-(1-methylethyl) 2-((6-amino-1,2-dihydro-2-oxopyrid-1-yl)methyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 2-((2-aminopyrid-3-yloxy)methyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 2-(((1H-5-amino-1,2,4-triazol-3-yl)thio)methyl)-4-(3-chloro-6-fluoro-2- (trifluoromethyl)phenyl)-6-(fluoromethyl)-1,4-dihydro-3,5-pyridinedicarboxylate,

5-Methyl 3-(1-methylethyl) 2-((5-amino-1H-1,2,4-triazol-1-yl)methyl)-4-(3-chloro-6-fluoro-2-(trifluoromethyl)phenyl)-6-(fluoromethyl)-1,4-dihydro-3,5-pyridinedicarboxylate,

5-methyl 3-(1-methylethyl) 2-((3-amino-1H-1,2,4-triazol-1-yl)methyl)-4-(3-chloro-6-fluoro-2-(trifluoromethyl)phenyl)-6-(fluoromethyl)-1,4-dihydro-3,5-pyridinedicarboxylate,

5-methyl 3-(1-methylethyl) 2-((3-amino-4H-1,2,4-triazol-4-yl)methyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 4-((3-chloro-6-fluoro-2-(trifluoromethyl)phenyl)-6-(fluoromethyl)-1,4-dihydro-2-((1-pyridinium)methyl)-3,5-pyridinedicarboxylate bromide,

5-Methyl 3-(1-methylethyl) 2-((aminoiminomethyl)-thiomethyl)-4-(3-chloro-6-fluoro-2-(trifluoromethyl)phenyl)-6-fluoromethyl-1,4-dihydro-3,5-pyridinedicarboxylate,

5-Methyl 3-(1-methylethyl) 2-((2-aminoethylsulphonyl) methyl)-4-(3-chloro-6-fluoro-2-(trifluoromethyl)phenyl)-6-(fluoromethyl)-1,4-dihydro-3,5-pyridinedicarboxylate,

3-Ethyl 5-methyl 2-((2-aminoethylthio)methyl)-4-(3-chloro-6-fluoro-2-(trifluoromethyl)phenyl)-6-(fluoromethyl)- 1,4-dihydro-3,5-pyridinedicarboxylate,

3-Ethyl 5-methyl 2-((2-aminoethylthio)methyl)-4-(2-chlorophenyl)-6-(fluoromethyl)-1,4-dihydro-3,5-pyridinedicarboxylate,

5-Methyl 3-(methylethyl) 2-((aminooxy)methyl)-4-(3-chloro-6-fluoro-2-(trifluoromethyl)phenyl)-6-(fluoromethyl)-1,4-dihydro-3,5-pyridinedicarboxylate,

5-Methyl 3-(1-methylethyl) 2-((4-(2-aminoethyl)-1H-imidazol-1-yl)methyl)-4-(3-chloro-6-fluoro-2-(trifluoromethyl)phenyl)-6-(fluoromethyl)-1,4-dihydro-3,5-pyridinedicarboxylate,

3-Methyl 5-(1-methylethyl) 4-(3-chloro-6-fluoro-2-(trifluoromethyl)phenyl)-2-(fluoromethyl)-1,4-dihydro-6-((2-(1H-imidazol-4-yl)ethylamino)methyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((1H-1,2,4-triazol-3-ylthio)methyl)-3,5-pyridinedicarboxylate,
5-Methyl 3-(1-methylethyl) 4(-3-chloro-6-fluoro-2-(trifluoromethyl)phenyl)-2-((4,6-diaminopyrimidin-2-ylthio)methyl)-6-(fluoromethyl)-1,4-dihydro-3.5-pyridinedicarboxylate,
3-Ethyl 5-methyl 4-(2-chlorophenyl)-2-((5-ethoxycarbonyl-3,4-dihydro-4-oxopyrimidin-2-ylthio)methyl)-6-(fluoromethyl)-1,4-dihydro-3,5-pyridinedicarboxylate,
3-Ethyl 5-methyl 4-(2-chlorophenyl)-2-((3,4-dihydro-4-oxopyrimidin-2-ylthio)methyl)-6-(fluoromethyl)-1,4-dihydro-3,5-pyridinedicarboxylate,
5-Ethyl 3-methyl 4-(2-chlorophenyl)-2-(fluoromethyl)-1,4-dihydro-6-((pyrimidin-2-ylthio)methyl)-3,5-pyridinedicarboxylate,
5-Ethyl 3-methyl 4-(2-chlorophenyl)-2-(fluoromethyl)-1,4-dihydro-6-((pyridin-2-ylthio)methyl)-3,5-pyridinedicarboxylate,
3-Ethyl 5-methyl 2-((1H-benzimidazol-2-ylthio)methyl)-4-(2-chlorophenyl)-6-(fluoromethyl)-1,4-dihydro-3,5-pyridinedicarboxylate.
5-Ethyl 3-methyl 4-(2-chlorophenyl)-2-(fluoromethyl)-1,4-dihydro-6-((1H-imidazol-2-ylthio)methyl)-3,5-pyridinedicarboxylate,
3-Ethyl 5-methyl 4-(2,3-dichlorophenyl)-2-((3,4-dihydro-4-oxopyrimidin-2-ylthio)methyl)-6-(fluoromethyl)-1,4-dihydro-3,5-pyridinedicarboxylate,
3-Ethyl 5-methyl 4-(2,3-dichlorophenyl)-2-((5-(ethoxycarbonyl)-3,4-dihydro-4-oxopyrimidin-2-ylthio)methyl)-6-(fluoromethyl)-1,4-dihydro-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((5-methoxy-1H-benzimidazol-2-ylthio)methyl)-3,5- pyridinedicarboxylate,
3-Ethyl 5-methyl 2-((6-carboxy-3,4-dihydro-4-oxopyrimidin-2-ylthio)methyl)-4-(2-chlorophenyl)-6-(fluoromethyl)-1,4-dihydro-3,5-pyridinedicarboxylate,
3-Ethyl 5-methyl 4-(2,3-dichlorophenyl)-2-((3,4-dihydro-6-hydroxy-4-oxopyrimidin-2-ylthio)methyl)-6-(fluoromethyl)-1,4-dihydro-3,5-pyridinedicarboxylate,
3-Ethyl 5-methyl 4-(2,3-dichlorophenyl)-2-((3,4-dihydro-6-methyl-4-oxopyrimidin-2-ylthio)methyl)-6-(fluoromethyl)-1,4-dihydropyridine-3,5-dicarboxylate,
3-Ethyl 5-methyl 2-((5-(ethoxycarbonyl)-3,4-dihydro-4-oxopyrimidin-2-ylthio)methyl)-6-(fluoromethyl)-1,4-dihydro-4-(2-trifluoromethyl)phenyl)-3,5-pyridinedicarboxylate.
3-Ethyl 5-methyl 2-((3,4-dihydro-4-oxopyrimidin-2-ylthio)methyl)-6-(fluoromethyl)-1,4-dihydro-4-(2-trifluoromethyl)phenyl)-3,5-pyridinedicarboxylate,
3-Ethyl 5-methyl 2-((4-amino-5-(ethoxycarbonyl)-pyrimidin-2-ylthio)methyl)-4-(2-chlorophenyl)-6-(fluoromethyl)-1,4-dihydro-3,5-pyridinedicarboxylate,
5-Ethyl-3-methyl-4-(2,3-dichlorophenyl)-2-(fluoromethyl)-1,4-dihydro-6-(pyrid-2-ylthiomethyl)-3,5-pyridinedicarboxylate,
3-Ethyl 5-methyl 4-(2,3-dichlorophenyl)-2-((((cyanoimino)methylamino)methylthio)methyl)-6-(fluoromethyl)-1,4- dihydro-3,5-pyridinedicarboxylate,
3-Ethyl 5-methyl 2-(((acetylimino)aminoethylthio) methyl)-4-(2,3-dichlorophenyl)-6-(fluoromethyl)-1,4-dihydro-3,5-pyridinedicarboxylate,
5-Methyl 3-(1-methylethyl) 2-((2-aminocarbonyloxy) methyl)-4-(2,3-dichlorophenyl)-6-(fluoromethyl)-1,4-dihydro-3,5-pyridinedicarboxylate,
5-Ethyl 3-methyl 4-(2-chlorophenyl)-2-(fluoromethyl)-1,4-dihydro-6-((1H-pyrrol-1-yl)methyl)-3,5-pyridinedicarboxylate,
3-Methyl 5-(1-methylethyl) 4-(3-chloro-6-fluoro-2-(trifluoromethyl)phenyl)-2-(fluoromethyl)-1,4-dihydro-6-((4H-1,2,4-triazol-4-yl)methyl)-3,5-pyridinedicarboxylate,
3-Methyl 5-(6-(5-phenylpyrazol-3-ylamino)hexyl) 4-(3-chloro-6-fluoro-2-(trifluoromethyl)phenyl)-2-(fluoromethyl)-1,4-dihydro-6-methyl-3,5-pyridinedicarboxylate,
3-Methyl 5-(6-(pyrid-3-yloxy)hexyl) 4-(3-chloro-6-fluoro-2-(trifluoromethyl)phenyl)-2-(fluoromethyl)-1,4-dihydro-6-methyl-3,5-pyridinedicarboxylate,
5-(6-(1H-Imidazol-1-yl)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(6-(4,6-Dimethylpyrimidin-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-(6-(1H-Benzimidazol-2-ylthio)hexyl) 3-methyl 4-(3-chloro-6-fluoro-2-(trifluoromethyl)phenyl)-2-(fluoromethyl)-1,4-dihydro-6-methyl-3,5-pyridinedicarboxylate,
5-((8-Ethoxy-8-oxo)octyl) 3-methyl 4-(3-chloro-6-fluoro-2-(trifluoromethyl)phenyl)-2-(fluoromethyl)-1,4-
5-((8-Amino-8-oxo)octyl) 3-methyl 4-(3-chloro-6-fluoro-2-(trifluoromethyl)phenyl)-2-(fluoromethyl)-1,4-dihydro-6-methyl-3,5-pyridinedicarboxylate,
3-Ethyl 5-methyl 2-((2-aminoethoxy)methyl)-6-(fluoromethyl)-1,4-dihydro-4-(5-nitrothien-2-yl)-3,5-pyridinedicarboxylate,
3-Ethyl 5-methyl 2-((2-aminoethoxy)methyl)-4-(5-cyanothien-2-yl)-6-(fluoromethyl)-1,4-dihydro-3,5-pyridinedicarboxylate.

3-Ethyl 5-methyl 2-((2-aminoethoxy)methyl)-4-(3-cyanothien-2-yl)-6-(fluoromethyl)-1,4-dihydro-3,5-pyridinedicarboxylate,
3-Ethyl 5-methyl 2-((2-aminoethoxy(methyl)-6-(fluoromethyl)-4-(3-methylthien-2-yl)-1,4-dihydro-3,5-pyridinedicarboxylate,
3-Ethyl 5-methyl 2-((2-(2-aminoethoxy)ethoxy)methyl)-4-(2-chlorophenyl)-6-(fluoromethyl)-1,4-dihydro-3,5- pyridinedicarboxylate,
Diethyl 2-((2-aminoethoxy)methyl)-4-(2-chlorophenyl)-1,4-dihydro-6-(trifluoromethyl)-3,5-pyridinedicarboxylate,
3-Ethyl 5-methyl 4-(3-amino-2,6-difluorophenyl)-2-((2-aminoethoxy)methyl)-6-(fluoromethyl)-1,4-dihydro-3,5-pyridinedicarboxylate,
5-Methyl 3-(6-((6-methyl-2-phenylpyrimidin-4-yl)oxy) hexyl) 2-((2-aminoethoxy)methyl)-4-(2-chlorophenyl)-6-(fluoromethyl)-1,4-dihydro-3,5-pyridinedicarboxylate,
5-Methyl 3-(6-((6-methyl-2-phenylpyrimidin-4-yl)thio)hexyl) 2-((2-aminoethoxy)methyl)-4-(2-chlorophenyl)-6-(fluoromethyl)-1,4-dihydro-3,5-pyridinedicarboxylate,
3-(6-((4,6-Dimethylpyrimidin-2-yl)thio)hexyl) 5-methyl 2-((2-aminoethoxy)methyl)-4-(2-chlorophenyl)-6-(fluoromethyl)-1,4-dihydro-3,5-pyridinedicarboxylate,
3-Ethyl 5-methyl 2-((2-aminoethoxy)methyl)-4-(2-chloro-3,5-dimethoxyphenyl)-6-(fluoromethyl)-1,4-dihydro-3,5-pyridinedicarboxylate,
3-Ethyl 5-methyl 2-((2-aminoethoxy)methyl)-4-(2-chloro-3-(trifluoromethyl)phenyl)-6-(fluoromethyl)-1,4-dihydro-3,5-pyridinedicarboxylate,
3-Ethyl 5-methyl 2-((2-aminoethoxy)methyl)-4-(3-cyanophenyl)-6-(fluoromethyl)-1,4-dihydro-3,5-pyridinedicarboxylate,
3-Ethyl 5-methyl 2-((2-aminoethoxy)methyl)-4-(2,5- difluoro-6-(trifluoromethyl)phenyl)-6-(fluoromethyl)-1,4-dihydro-3,5-pyridinedicarboxylate,
3-Ethyl 5-methyl 2-((2-aminoethoxy)methyl)-4-(2-fluoro-5-methoxyphenyl)-6-(fluoromethyl)-1,4-dihydro-3,5-pyridinedicarboxylate,
5-Methyl 3-((6-phenoxy)hexyl) 2-((2-aminoethoxy) methyl)-4-(2-chlorophenyl)-6-(fluoromethyl)-1,4-dihydro-3,5-pyridinedicarboxylate,
3-Ethyl 5-methyl 2-((2-aminoethoxy)methyl)-4-(6-chloro-2-fluoro-3-methoxyphenyl)-6-(fluoromethyl)-1,4-dihydro-3,5-pyridinedicarboxylate,
5-Methyl 3-(2,2,2-trichloroethyl) 2-((2,aminoethoxy)methyl)-4-(2-chlorophenyl)-6-(fluoromethyl)-1,4-dihydro-3,5-pyridinedicarboxylate,
5-Methyl 3-(2,2,2-trifluoroethyl) 2-((2-aminoethoxy)methyl)-4-(2-chlorophenyl)-6-(fluoromethyl)-1,4-dihydro-3,5-pyridinedicarboxylate,
3-Methyl 5-(1-methylethyl) 4-(3-chloro-2-fluorophenyl)-2-(fluoromethyl)-1,4-dihydro-6-methyl-3,5-pyridinedicarboxylate,
3-Methyl 5-(1-methylethyl) 4-(2,6-difluorophenyl)-2-(fluoromethyl)-1,4-dihydro-6-methyl-3,5-pyridinedicarboxylate,
3-Methyl 5-(1-methylethyl) 2-(fluoromethyl)-1,4-dihydro-6-methyl-4-(3-nitro-2-(trifluoromethyl)phenyl)-3,5- pyridinedicarboxylate,
3-Methyl 5-(1-methylethyl) 4-(2-fluoro-3-(trifluoromethyl)phenyl)-2-(fluoromethyl)-1,4-dihydro-6-methyl-3,5-pyridinedicarboxylate,
3-Methyl 5-(1-methylethyl) 4-(3-chloro-2-(trifluoromethyl)phenyl)-2-(fluoromethyl)-1,4-dihydro-6-methyl-3,5-pyridinedicarboxylate.
3-Methyl 5-(1-methylethyl) 4-(3-chloro-2-nitrophenyl)--2-(fluoromethyl)-1,4-dihydro-6-methyl-3,5-pyridinedicarboxylate,
3-Methyl 5-(1-methylethyl) 4-(2,3-difluorophenyl)-2-(fluoromethyl)-1,4-dihydro-6-methyl-3,5-pyridinedicarboxylate,
3-Methyl 5-(1-methylethyl) 2-(fluoromethyl)-1,4-dihydro-4-(5-methoxy-2-nitrophenyl)-6-methyl-3,5-pyridinedicarboxylate,
3-Methyl 5-(1-methylethyl) 2-fluoromethyl-4-(2-fluoro-6-(pentafluoroethyl)phenyl)-1,4-dihydro-6-methyl-3,5-pyridinedicarboxylate,
3-Methyl 5-(1-methylethyl) 2-(fluoromethyl)-4-(4-fluoro-2-(trifluoromethyl)phenyl)-1,4-dihydro-6-methyl-3,5-pyridinedicarboxylate,
3-Methyl 5-(1-methylethyl) 2-(fluoromethyl)-4-(8-fluoronaphthalen-1-yl)-1,4-dihydro-6-methyl-3,5-pyridinedicarboxylate.
5-Methyl 3-(2,2,2-trifluoroethyl) 2-((2-aminoethoxy)methyl)-6-(fluoromethyl)-1,4-dihydro-4-(2-(trifluoromethyl)phenyl)-3,5-pyridinedicarbxylate,
5-Methyl 3-(1-methylethyl) 2-((2-aminoethoxy)methyl)-6-(fluoromethyl)-1,4-dihydro-4-(2,3,5,6-tetrafluorophenyl)-3,5-pyridinedicarboxylate,
5-Methyl 3-(1-methylethyl) 2-((2-aminoethoxy)methyl)-6-(fluoromethyl)-1,4-dihydro-4-(2,4,6-trifluorophenyl)-3,5-pyridinedicarboxylate,
3-Methyl 5-(2,2,2-trifluoroethyl) 2-(fluoromethyl)-1,4-dihydro-6-methyl-4-(2-(trifluoromethyl)-phenyl)-3,5-pyridinedicarboxylate,
3-Methyl 5-(1-methylethyl) 2-(fluoromethyl)-1,4-dihydro-6-methyl-4-(2,4,6-trifluorophenyl)-3,5-pyridinedicarboxylate.
3-Methyl 5-(1-methylethyl) 2-(fluoromethyl)-1,4-dihydro-6-methyl-4-(pentafluorophenyl)-3,5-pyridinedicarboxylate.
3-Methyl 5-(1-methylethyl) 4-(5-fluoro-2-methoxyphenyl)-2-(fluoromethyl)-1,4-dihydro-6-methyl-3,5-pyridinedicarboxylate,
3-Methyl 5-(1-methylethyl) 4-(2-chloro-3,5-dimethoxyphenyl)-2-(fluoromethyl)-1,4-dihydro-6-methyl-3,5-dihydropyridine,
3-Methyl 5-(1-methylethyl) 4-(6-chloro-2-fluoro-3- methoxyphenyl)-2-(fluoromethyl)-1,4-dihydro-6-methyl-3,5-pyridinedicarboxylate,
Diethyl 2-((2-aminoethoxy)methyl)-4-(3-chloro-6-fluoro-2-(trifluoromethyl)phenyl)-1,4-dihydro-6-(trifluoromethyl)-3,5-pyridinedicarboxylate.
5-Methyl 3-(2,2,2-trichloroethyl) 2-((2-((5-amino-1H-1,2,4-triazol-3-yl)amino)ethoxy)methyl)-4-(2-chlorophenyl)-6-(fluoromethyl)-1,4-dihydro-3,5-pyridinedicarboxylate,
3-Ethyl 5-methyl 2-((2-((5-amino-1H-1,2,4-triazol-3-yl)amino)ethoxy)methyl)-6-(fluoromethyl)-1,4-dihydro-4-(pentafluorophenyl)-3,5-pyridinedicarboxylate,
3-Ethyl 5-methyl 2-((2-(2-(5-amino-1H-1,2,4-triazol-3-yl) aminoethoxy)ethoxy)methyl)-4-(2-chlorophenyl)-6-(fluoromethyl)-1,4-dihydro-3,5-pyridinedicarboxylate,
3-Ethyl 5-methyl 2-((2-((5-amino-1H-1,2,4-triazol-3-yl)amino)ethoxy)methyl)-4-(3-cyanothien-2-yl)-6-(fluoromethyl)-1,4-dihydro-3,5-pyridinedicarboxylate.
3-Methyl 5-(1-methylethyl) 4-(5-amino-2-chlorophenyl)-2-(fluoromethyl)-1,4-dihydro-6-methyl-3,5-pyridinedicarboxylate,
and pharmaceutically acceptable salts, esters, amides and protected derivatives thereof.

8. A compound of formula I in which $R_1$ is hydrogen for use as an intermediate.
9. A compound of formula I in which $R_6$ is $-CH_2CH_2NH_2$ for use as an intermediate.
10. A compound of formula I in which $-CHZXR_6$ is a group $-CH_2Br$ for use as an intermediate.